# EUROPEAN PATENT APPLICATION

(11) **EP 2 891 664 A1**
(43) Date of publication of application: **08.07.2015**
(21) Application number: 14150377.1
(22) Date of filing: 07.01.2014
(51) Int. Cl.: C07K 16/00

(54) **Novel methods for the stabilisation of immunoglobulin constant domains**

(71) Applicant: Technische Universität München, 80333 München (DE); Helmholtz Zentrum München Deutsches Forschungszentrum für Gesundheit und Umwelt GmbH, 85764 Neuherberg (DE)
(72) Inventor: Buchner, Johannes, 93346 Ihrlerstein (DE); Feige, Matthias, Memphis, TN Tennessee 38103 (US); Marcinowski, Moritz, 80539 München (DE); Hennig, Janosch, 85778 Haimhausen (DE)
(74) Representative: Vossius & Partner Patentanwälte Rechtsanwälte mbB

(57) **Abstract**

The present invention relates to a method of producing a modified protein having an increased stability and/or improved folding efficiency as compared to the unmodified protein, the method comprising (i) modifying a nucleic acid molecule encoding a protein comprising at least one immunoglobulin constant domain-like region by (a-i) replacing the nucleotides encoding at least one amino acid, preferably an uncharged amino acid, in the loop separating the C strand from the D strand with nucleotides encoding a charged amino acid selected from the group consisting of Arg, Lys, His, Glu and Asp and/or replacing the nucleotides encoding at least one amino acid, preferably an uncharged amino acid, in the helix connecting the E strand with the F strand with nucleotides encoding a charged amino acid selected from the group consisting of Arg, Lys, His, Glu and Asp; (a-ii) replacing the nucleotides encoding at least one amino acid not having a side chain that can form a hydrogen bond in the loop separating the C strand from the D strand with nucleotides encoding an amino acid having a side chain capable of forming a hydrogen bond selected from the group consisting of Gln, Asn, Tyr, Ser and Thr and/or replacing the nucleotides encoding at least one amino acid not having a side chain that can form a hydrogen bond in the helix connecting the E strand with the F strand with nucleotides encoding an amino acid having a side chain capable of forming a hydrogen bond selected from the group consisting of Gln, Asn, Tyr, Ser and Thr; and/or (a-iii) replacing the nucleotides encoding at least one amino acid in the loop separating the C strand from the D strand with nucleotides encoding a cysteine and/or replacing the nucleotides encoding at least one amino acid in the helix connecting the E strand with the F strand with nucleotides encoding a cysteine; and/or (b) replacing the nucleotides encoding at least one non-hydrophobic amino acid at a position suitable to participate in the formation of the hydrophobic core with nucleotides encoding a hydrophobic amino acid selected from the group consisting of Val, Ile, Leu, Met, Phe, Trp and Pro; and (ii) expressing the nucleic acid molecule to produce the stabilised protein. The present invention further relates to a method of producing a modified protein having an improved secretion from cells as compared to the unmodified protein, as well as to a protein comprising at least one immunoglobulin constant domain-like region having an additional salt bridge, an additional hydrogen bond, an additional disulfide bridge and/or an extended hydrophobic core. The present invention further relates to a nucleic acid molecule encoding the modified protein of the invention, as well as a vector comprising said nucleic acid molecule and a host cell comprising the vector. Further, the present invention relates also to a composition as well as to a kit.

SEQUENCE LISTING

<110> Technische Universität München; Helmholtz Zentrum München

<120> Novel methods for the stabilisation of proteins

<130> W2002 EP

<160> 155

<170> BiSSAP 1.2

<210> 1
<211> 103
<212> PRT
<213> Ginglymostoma cirratum


<400> 1
Arg Gln Thr Asp Ile Ser Val Ser Leu Leu Lys Pro Pro Phe Glu Glu
1               5                   10                  15
Ile Trp Thr Gln Gln Thr Ala Thr Ile Val Cys Glu Ile Val Tyr Ser
            20                  25                  30
Asp Leu Glu Asn Ile Lys Val Phe Trp Gln Val Asn Gly Val Glu Arg
        35                  40                  45
Lys Lys Gly Val Glu Thr Gln Asn Pro Glu Trp Ser Gly Ser Lys Ser
    50                  55                  60
Thr Ile Val Ser Lys Leu Lys Val Met Ala Ser Glu Trp Asp Ser Gly
65                  70                  75                  80
Thr Glu Tyr Val Cys Leu Val Glu Asp Ser Glu Leu Pro Thr Pro Val
                85                  90                  95
Lys Ala Ser Ile Arg Lys Ala
            100

<210> 2
<211> 101
<212> PRT
<213> Ginglymostoma cirratum


<400> 2
Glu Ile Ala Val Leu Leu Arg Asp Pro Thr Val Glu Glu Ile Trp Ile
1               5                   10                  15
Asp Lys Ser Ala Thr Leu Val Cys Glu Val Leu Ser Thr Val Ser Ala
            20                  25                  30
Gly Val Val Val Ser Trp Met Val Asn Gly Lys Val Arg Asn Glu Gly
        35                  40                  45
Val Gln Met Glu Pro Thr Lys Met Ser Gly Asn Gln Tyr Leu Thr Ile
    50                  55                  60
Ser Arg Leu Thr Ser Ser Val Glu Glu Trp Gln Ser Gly Val Glu Tyr
65                  70                  75                  80
Thr Cys Ser Ala Lys Gln Asp Gln Ser Ser Thr Pro Val Val Lys Arg
                85                  90                  95
Thr Arg Lys Ala Arg
            100

<210> 3
<211> 106
<212> PRT
<213> Homo sapiens


<400> 3
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105

<210> 4
<211> 106
<212> PRT
<213> Homo sapiens


<400> 4
Gly Gln Pro Lys Ala Asn Pro Thr Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Gly Ser Pro
        35                  40                  45
Val Lys Ala Gly Val Glu Thr Thr Lys Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210> 5
<211> 106
<212> PRT
<213> Homo sapiens


<400> 5
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210> 6
<211> 106
<212> PRT
<213> Homo sapiens


<400> 6
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Ala Lys Ala Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys

65                  70                  75                  80
Ser His Lys Ser Tyr Ser Cys Gln Val Thr His Glu Gly Ser Thr Val
                85                  90                  95
Glu Lys Thr Val Ala Pro Thr Glu Cys Ser
            100                 105

<210> 7
<211> 106
<212> PRT
<213> Mus musculus

<400> 7
Ala Asp Ala Ala Pro Thr Val Ser Ile Phe Pro Pro Ser Ser Glu Gln
1               5                   10                  15
Leu Thr Ser Gly Gly Ala Ser Val Val Cys Phe Leu Asn Asn Phe Tyr
            20                  25                  30
Pro Lys Asp Ile Asn Val Lys Trp Lys Ile Asp Gly Ser Glu Arg Gln
        35                  40                  45
Asn Gly Val Leu Asn Ser Trp Thr Asp Gln Asp Ser Lys Asp Ser Thr
    50                  55                  60
Tyr Ser Met Ser Ser Thr Leu Thr Leu Thr Lys Asp Glu Tyr Glu Arg
65                  70                  75                  80
His Asn Ser Tyr Thr Cys Glu Ala Thr His Lys Thr Ser Thr Ser Pro
                85                  90                  95
Ile Val Lys Ser Phe Asn Arg Asn Glu Cys
            100                 105

<210> 8
<211> 105
<212> PRT
<213> Mus musculus

<400> 8
Gln Pro Lys Ser Ser Pro Ser Val Thr Leu Phe Pro Pro Ser Ser Glu
1               5                   10                  15
Glu Leu Glu Thr Asn Lys Ala Thr Leu Val Cys Thr Ile Thr Asp Phe
            20                  25                  30
Tyr Pro Gly Val Val Thr Val Asp Trp Lys Val Asp Gly Thr Pro Val
        35                  40                  45
Thr Gln Gly Met Glu Thr Thr Gln Pro Ser Lys Gln Ser Asn Asn Lys
    50                  55                  60
Tyr Met Ala Ser Ser Tyr Leu Thr Leu Thr Ala Arg Ala Trp Glu Arg
65                  70                  75                  80
His Ser Ser Tyr Ser Cys Gln Val Thr His Glu Gly His Thr Val Glu
                85                  90                  95
Lys Ser Leu Ser Arg Ala Asp Cys Ser
            100                 105

<210> 9
<211> 104
<212> PRT
<213> Mus musculus

<400> 9
Gln Pro Lys Ser Thr Pro Thr Leu Thr Val Phe Pro Pro Ser Ser Glu
1               5                   10                  15
Glu Leu Lys Glu Asn Lys Ala Thr Leu Val Cys Leu Ile Ser Asn Phe
            20                  25                  30
Ser Pro Ser Gly Val Thr Val Ala Trp Lys Ala Asn Gly Thr Pro Ile
        35                  40                  45
Thr Gln Gly Val Asp Thr Ser Asn Pro Thr Lys Glu Gly Asn Lys Phe
    50                  55                  60
Met Ala Ser Ser Phe Leu His Leu Thr Ser Asp Gln Trp Arg Ser His
65                  70                  75                  80
Asn Ser Phe Thr Cys Gln Val Thr His Glu Gly Asp Thr Val Glu Lys
                85                  90                  95

Ser Leu Ser Pro Ala Glu Cys Leu
                100

<210> 10
<211> 104
<212> PRT
<213> Mus musculus


<400> 10
Gln Pro Lys Ser Thr Pro Thr Leu Thr Met Phe Pro Pro Ser Pro Glu
1               5                   10                  15
Glu Leu Gln Glu Asn Lys Ala Thr Leu Val Cys Leu Ile Ser Asn Phe
                20                  25                  30
Ser Pro Ser Gly Val Thr Val Ala Trp Lys Ala Asn Gly Thr Pro Ile
        35                  40                  45
Thr Gln Gly Val Asp Thr Ser Asn Pro Thr Lys Glu Asp Asn Lys Tyr
    50                  55                  60
Met Ala Ser Ser Phe Leu His Leu Thr Ser Asp Gln Trp Arg Ser His
65                  70                  75                  80
Asn Ser Phe Thr Cys Gln Val Thr His Glu Gly Asp Thr Val Glu Lys
                85                  90                  95
Ser Leu Ser Pro Ala Glu Cys Leu
                100

<210> 11
<211> 103
<212> PRT
<213> Oryctolagus cuniculus


<400> 11
Asp Pro Val Ala Pro Thr Val Leu Ile Phe Pro Pro Ala Ala Asp Gln
1               5                   10                  15
Val Ala Thr Gly Thr Val Thr Ile Val Cys Val Ala Asn Lys Tyr Phe
                20                  25                  30
Pro Asp Val Thr Val Thr Trp Glu Val Asp Gly Thr Thr Gln Thr Thr
        35                  40                  45
Gly Ile Glu Asn Ser Lys Thr Pro Gln Asn Ser Ala Asp Cys Thr Tyr
    50                  55                  60
Asn Leu Ser Ser Thr Leu Thr Leu Thr Ser Thr Gln Tyr Asn Ser His
65                  70                  75                  80
Lys Glu Tyr Thr Cys Lys Val Thr Gln Gly Thr Thr Ser Val Val Gln
                85                  90                  95
Ser Phe Asn Arg Gly Asp Cys
                100

<210> 12
<211> 103
<212> PRT
<213> Oryctolagus cuniculus


<400> 12
Ala Thr Leu Ala Pro Thr Val Leu Ile Phe Pro Pro Ala Pro Ala Gln
1               5                   10                  15
Leu Ala Thr Gly Ala Val Thr Ile Val Cys Val Ala Asn Lys Tyr Phe
                20                  25                  30
Pro Asp Gly Thr Val Thr Trp Glu Val Asp Gly Lys Pro Leu Thr Thr
        35                  40                  45
Gly Ile Glu Thr Ser Lys Thr Pro Gln Asn Ser Asp Asp Cys Thr Tyr
    50                  55                  60
Asn Leu Ser Ser Thr Leu Thr Leu Gln Lys Ser Asn Tyr Asn Ser His
65                  70                  75                  80
Asn Glu Tyr Thr Cys Gln Val Ala Gln Gly Ala Gly Ser Val Val Gln
                85                  90                  95
Ser Phe Ser Arg Lys Asn Cys
                100

<210> 13
<211> 104
<212> PRT
<213> Oryctolagus cuniculus


<400> 13
Ala Thr Leu Ala Pro Thr Val Leu Ile Phe Pro Pro Ser Pro Ala Glu
1               5                   10                  15
Leu Ala Thr Gly Thr Ala Thr Ile Val Cys Val Ala Asn Lys Tyr Phe
            20                  25                  30
Pro Asp Gly Thr Val Thr Trp Gln Val Asp Gly Lys Pro Leu Thr Thr
        35                  40                  45
Gly Ile Glu Thr Ser Lys Thr Pro Gln Asn Ser Asp Asp Cys Thr Tyr
    50                  55                  60
Asn Leu Ser Ser Thr Leu Thr Leu Lys Ser Asp Glu Tyr Asn Ser His
65                  70                  75                  80
Asp Glu Tyr Thr Cys Gln Val Ala Gln Gly Ser Gly Ser Pro Val Val
                85                  90                  95
Gln Ser Phe Ser Arg Lys Asn Cys
            100

<210> 14
<211> 104
<212> PRT
<213> Oryctolagus cuniculus


<220>
<221> VARIANT
<222> 67
<223> /replace=Ala
      /replace=Val
      /replace=Thr
      /replace=Ser
      /replace=Arg
      /replace=Gln
      /replace=Pro
      /replace=Asn
      /replace=Met
      /replace=Phe
      /replace=Lys
      /replace=Ile
      /replace=His
      /replace=Gly
      /replace=Trp
      /replace=Glu
      /replace=Asp
      /replace=Cys
      /replace=Leu
      /replace=Tyr

<400> 14
Asp Pro Pro Ile Ala Pro Thr Val Leu Leu Phe Pro Pro Ser Ala Asp
1               5                   10                  15
Gln Leu Thr Thr Glx Thr Val Thr Ile Val Cys Val Ala Asn Lys Phe
            20                  25                  30
Arg Pro Asp Asp Ile Thr Val Thr Trp Lys Val Asp Asp Glu Ile Gln
        35                  40                  45
Gln Ser Gly Ile Glu Asn Ser Thr Thr Pro Gln Ser Pro Glu Asp Cys
    50                  55                  60
Thr Tyr Xaa Leu Ser Ser Thr Leu Ser Leu Thr Lys Ala Gln Tyr Asn
65                  70                  75                  80
Ser His Ser Tyr Thr Cys Gln Val His Asn Ser Ala Gly Ser Ile Val
                85                  90                  95
Glx Ser Phe Asn Arg Gly Asp Cys
            100

<210> 15

<211> 106
<212> PRT
<213> Oryctolagus cuniculus


<400> 15
Gly Asp Pro Val Ala Pro Ser Val Leu Leu Phe Pro Pro Ser Lys Glu
1               5                   10                  15
Glu Leu Thr Thr Gly Thr Ala Thr Ile Val Cys Val Ala Asn Lys Phe
            20                  25                  30
Tyr Pro Ser Asp Ile Thr Val Thr Trp Lys Val Asp Gly Thr Thr Gln
        35                  40                  45
Gln Ser Gly Ile Glu Asn Ser Lys Thr Pro Gln Ser Pro Glu Asp Asn
    50                  55                  60
Thr Tyr Ser Leu Ser Ser Thr Leu Ser Leu Thr Ser Ala Gln Tyr Asn
65                  70                  75                  80
Ser His Ser Val Tyr Thr Cys Glu Val Val Gln Gly Ser Ala Ser Pro
                85                  90                  95
Ile Val Gln Ser Phe Asn Arg Gly Asp Cys
            100                 105

<210> 16
<211> 105
<212> PRT
<213> Oryctolagus cuniculus


<400> 16
Gln Pro Ala Val Thr Pro Ser Val Ile Leu Phe Pro Pro Ser Ser Glu
1               5                   10                  15
Glu Leu Lys Asp Asn Lys Ala Thr Leu Val Cys Leu Ile Ser Asp Phe
            20                  25                  30
Tyr Pro Arg Thr Val Lys Val Asn Trp Lys Ala Asp Gly Asn Ser Val
        35                  40                  45
Thr Gln Gly Val Asp Thr Thr Gln Pro Ser Lys Gln Ser Asn Asn Lys
    50                  55                  60
Tyr Ala Ala Ser Ser Phe Leu His Leu Thr Ala Asn Gln Trp Lys Ser
65                  70                  75                  80
Tyr Gln Ser Val Thr Cys Gln Val Thr His Glu Gly His Thr Val Glu
                85                  90                  95
Lys Ser Leu Ala Pro Ala Glu Cys Ser
            100                 105

<210> 17
<211> 100
<212> PRT
<213> Homo sapiens


<400> 17
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro
            100

<210> 18
<211> 113
<212> PRT
<213> Homo sapiens

<400> 18
Pro Cys Pro Ala Pro Glu Leu Leu Gly Gly Pro Ser Val Phe Leu Phe
1               5                   10                  15
Pro Pro Lys Pro Lys Asp Thr Leu Met Ile Ser Arg Thr Pro Glu Val
            20                  25                  30
Thr Cys Val Val Val Asp Val Ser His Glu Asp Pro Glu Val Lys Phe
        35                  40                  45
Asn Trp Tyr Val Asp Gly Val Glu Val His Asn Ala Lys Thr Lys Pro
    50                  55                  60
Arg Glu Glu Gln Tyr Asn Ser Thr Tyr Arg Val Val Ser Val Leu Thr
65                  70                  75                  80
Val Leu His Gln Asp Trp Leu Asn Gly Lys Glu Tyr Lys Cys Lys Val
                85                  90                  95
Ser Asn Lys Ala Leu Pro Ala Pro Ile Glu Lys Thr Ile Ser Lys Ala
            100                 105                 110
Lys

<210> 19
<211> 107
<212> PRT
<213> Homo sapiens

<400> 19
Gly Gln Pro Arg Glu Pro Gln Val Tyr Thr Leu Pro Pro Ser Arg Asp
1               5                   10                  15
Glu Leu Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu
        35                  40                  45
Asn Asn Tyr Lys Thr Thr Pro Pro Val Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Pro Gly Lys
            100                 105

<210> 20
<211> 100
<212> PRT
<213> Homo sapiens

<400> 20
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val Glu Arg
            100

<210> 21
<211> 109
<212> PRT
<213> Homo sapiens

<400> 21
Ala Pro Pro Val Ala Gly Pro Ser Val Phe Leu Phe Pro Pro Lys Pro
1               5                   10                  15
Lys Asp Thr Leu Met Ile Ser Arg Thr Pro Glu Val Thr Cys Val Val
            20                  25                  30
Val Asp Val Ser His Glu Asp Pro Glu Val Gln Phe Asn Trp Tyr Val
        35                  40                  45
Asp Gly Val Glu Val His Asn Ala Lys Thr Lys Pro Arg Glu Glu Gln
    50                  55                  60
Phe Asn Ser Thr Phe Arg Val Val Ser Val Leu Thr Val Val His Gln
65                  70                  75                  80
Asp Trp Leu Asn Gly Lys Glu Tyr Lys Cys Lys Val Ser Asn Lys Gly
                85                  90                  95
Leu Pro Ala Pro Ile Glu Lys Thr Ile Ser Lys Thr Lys
            100                 105

<210> 22
<211> 107
<212> PRT
<213> Homo sapiens


<400> 22
Gly Gln Pro Arg Glu Pro Gln Val Tyr Thr Leu Pro Pro Ser Arg Glu
1               5                   10                  15
Glu Met Thr Lys Asn Gln Val Ser Leu Thr Cys Leu Val Lys Gly Phe
            20                  25                  30
Tyr Pro Ser Asp Ile Ser Val Glu Trp Glu Ser Asn Gly Gln Pro Glu
        35                  40                  45
Asn Asn Tyr Lys Thr Thr Pro Pro Met Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Pro Gly Lys
            100                 105

<210> 23
<211> 100
<212> PRT
<213> Homo sapiens


<400> 23
Ala Ser Thr Lys Gly Pro Ser Val Phe Pro Leu Ala Pro Cys Ser Arg
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Thr Cys Asn Val Asn His Lys Pro Ser Asn Thr Lys Val Asp Lys
                85                  90                  95
Arg Val Glu Leu
            100

<210> 24
<211> 110
<212> PRT
<213> Homo sapiens


<400> 24
Ala Pro Glu Leu Leu Gly Gly Pro Ser Val Phe Leu Phe Pro Pro Lys

1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Gln Phe Lys Trp Tyr
        35                  40                  45
Val Asp Gly Val Glu Val His Asn Ala Lys Thr Lys Pro Arg Glu Glu
    50                  55                  60
Gln Tyr Asn Ser Thr Phe Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Thr Lys
            100                 105                 110

<210> 25
<211> 107
<212> PRT
<213> Homo sapiens


<400> 25
Gly Gln Pro Arg Glu Pro Gln Val Tyr Thr Leu Pro Pro Ser Arg Glu
1               5                   10                  15
Glu Met Thr Lys Asn Gln Val Ser Leu Thr Cys Leu Val Lys Gly Phe
            20                  25                  30
Tyr Pro Ser Asp Ile Ala Val Glu Trp Glu Ser Ser Gly Gln Pro Glu
        35                  40                  45
Asn Asn Tyr Asn Thr Thr Pro Pro Met Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Ile Phe Ser Cys Ser Val Met His Glu Ala Leu His Asn Arg Phe
                85                  90                  95
Thr Gln Lys Ser Leu Ser Leu Ser Pro Gly Lys
            100                 105

<210> 26
<211> 100
<212> PRT
<213> Homo sapiens


<400> 26
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser
            100

<210> 27
<211> 110
<212> PRT
<213> Homo sapiens


<400> 27
Ala Pro Glu Phe Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
            35                  40                  45
Val Asp Gly Val Glu Val His Asn Ala Lys Thr Lys Pro Arg Glu Glu
    50                  55                  60
Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
            100                 105                 110

<210> 28
<211> 107
<212> PRT
<213> Homo sapiens


<400> 28
Gly Gln Pro Arg Glu Pro Gln Val Tyr Thr Leu Pro Pro Ser Gln Glu
1               5                   10                  15
Glu Met Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu
        35                  40                  45
Asn Asn Tyr Lys Thr Thr Pro Pro Val Leu Asp Ser Asp Gly Ser Phe
    50                  55                  60
Phe Leu Tyr Ser Arg Leu Thr Val Asp Lys Ser Arg Trp Gln Glu Gly
65                  70                  75                  80
Asn Val Phe Ser Cys Ser Val Met His Glu Ala Leu His Asn His Tyr
                85                  90                  95
Thr Gln Lys Ser Leu Ser Leu Ser Leu Gly Lys
            100                 105

<210> 29
<211> 100
<212> PRT
<213> Mus musculus


<400> 29
Ala Lys Thr Thr Pro Pro Ser Val Tyr Pro Leu Ala Pro Gly Ser Ala
1               5                   10                  15
Ala Gln Thr Asn Ser Met Val Thr Leu Gly Cys Leu Val Lys Gly Tyr
            20                  25                  30
Phe Pro Glu Pro Val Thr Val Thr Trp Asn Ser Gly Ser Leu Ser Ser
        35                  40                  45
Gly Val His Thr Phe Pro Ala Val Leu Gln Ser Asp Leu Tyr Thr Leu
    50                  55                  60
Ser Ser Ser Val Thr Val Pro Ser Ser Pro Arg Pro Ser Glu Thr Val
65                  70                  75                  80
Thr Cys Asn Val Ala His Pro Ala Ser Ser Thr Lys Val Asp Lys Lys
                85                  90                  95
Ile Val Pro Arg
            100

<210> 30
<211> 107
<212> PRT
<213> Mus musculus


<400> 30
Val Pro Glu Val Ser Ser Val Phe Ile Phe Pro Pro Lys Pro Lys Asp
1               5                   10                  15
Val Leu Thr Ile Thr Leu Thr Pro Lys Val Thr Cys Val Val Val Asp
            20                  25                  30
Ile Ser Lys Asp Asp Pro Glu Val Gln Phe Ser Trp Phe Val Asp Asp
        35                  40                  45
Val Glu Val His Thr Ala Gln Thr Gln Pro Arg Glu Glu Gln Phe Asn

50                  55                  60
Ser Thr Phe Arg Ser Val Ser Glu Leu Pro Ile Met His Gln Asp Trp
65                  70                  75                  80
Leu Asn Gly Lys Glu Phe Lys Cys Arg Val Asn Ser Ala Ala Phe Pro
                85                  90                  95
Ala Pro Ile Glu Lys Thr Ile Ser Lys Thr Lys
            100                 105

<210> 31
<211> 107
<212> PRT
<213> Mus musculus

<400> 31
Gly Arg Pro Lys Ala Pro Gln Val Tyr Thr Ile Pro Pro Pro Lys Glu
1               5                   10                  15
Gln Met Ala Lys Asp Lys Val Ser Leu Thr Cys Met Ile Thr Asp Phe
            20                  25                  30
Phe Pro Glu Asp Ile Thr Val Glu Trp Gln Trp Asn Gly Gln Pro Ala
        35                  40                  45
Glu Asn Tyr Lys Asn Thr Gln Pro Ile Met Asn Thr Asn Gly Ser Tyr
    50                  55                  60
Phe Val Tyr Ser Lys Leu Asn Val Gln Lys Ser Asn Trp Glu Ala Gly
65                  70                  75                  80
Asn Thr Phe Thr Cys Ser Val Leu His Glu Gly Leu His Asn His His
                85                  90                  95
Thr Glu Lys Ser Leu Ser His Ser Pro Gly Lys
            100                 105

<210> 32
<211> 100
<212> PRT
<213> Mus musculus

<400> 32
Ala Lys Thr Thr Ala Pro Ser Val Tyr Pro Leu Ala Pro Val Cys Gly
1               5                   10                  15
Asp Thr Thr Gly Ser Ser Val Thr Leu Gly Cys Leu Val Lys Gly Tyr
            20                  25                  30
Phe Pro Glu Pro Val Thr Leu Thr Trp Asn Ser Gly Ser Leu Ser Ser
        35                  40                  45
Gly Val His Thr Phe Pro Ala Val Leu Gln Ser Asp Leu Tyr Thr Leu
    50                  55                  60
Ser Ser Ser Val Thr Val Thr Ser Ser Thr Trp Pro Ser Gln Ser Ile
65                  70                  75                  80
Thr Cys Asn Val Ala His Pro Ala Ser Ser Thr Lys Val Asp Lys Lys
                85                  90                  95
Ile Glu Pro Arg
            100

<210> 33
<211> 100
<212> PRT
<213> Mus musculus

<400> 33
Pro Ser Val Phe Ile Phe Pro Pro Lys Ile Lys Asp Val Leu Met Ile
1               5                   10                  15
Ser Leu Ser Pro Ile Val Thr Cys Val Val Val Asp Val Ser Glu Asp
            20                  25                  30
Asp Pro Asp Val Gln Ile Ser Trp Phe Val Asn Asn Val Glu Val His
        35                  40                  45
Thr Ala Gln Thr Gln Thr His Arg Glu Asp Tyr Asn Ser Thr Leu Arg
    50                  55                  60
Val Val Ser Ala Leu Pro Ile Gln His Gln Asp Trp Met Ser Gly Lys
65                  70                  75                  80

Glu Phe Lys Cys Lys Val Asn Asn Lys Asp Leu Pro Ala Pro Ile Glu
                85                  90                  95
Arg Thr Ile Ser
            100

<210> 34
<211> 97
<212> PRT
<213> Mus musculus


<400> 34
Pro Gln Val Tyr Val Leu Pro Pro Pro Glu Glu Glu Met Thr Lys Lys
1               5                   10                  15
Gln Val Thr Leu Thr Cys Met Val Thr Asp Phe Met Pro Glu Asp Ile
            20                  25                  30
Tyr Val Glu Trp Thr Asn Asn Gly Lys Thr Glu Leu Asn Tyr Lys Asn
        35                  40                  45
Thr Glu Pro Val Leu Asp Ser Asp Gly Ser Tyr Phe Met Tyr Ser Lys
    50                  55                  60
Leu Arg Val Glu Lys Lys Asn Trp Val Glu Arg Asn Ser Tyr Ser Cys
65                  70                  75                  80
Ser Val Val His Glu Gly Leu His Asn His His Thr Thr Lys Ser Phe
                85                  90                  95
Ser


<210> 35
<211> 100
<212> PRT
<213> Mus musculus


<400> 35
Lys Thr Thr Pro Pro Ser Val Tyr Pro Leu Ala Pro Gly Cys Gly Asp
1               5                   10                  15
Thr Thr Gly Ser Ser Val Thr Leu Gly Cys Leu Val Lys Gly Tyr Phe
            20                  25                  30
Pro Glu Ser Val Thr Val Thr Trp Asn Ser Gly Ser Leu Ser Ser Ser
        35                  40                  45
Val His Thr Phe Pro Ala Leu Leu Gln Ser Gly Leu Tyr Thr Met Ser
    50                  55                  60
Ser Ser Val Thr Val Pro Ser Ser Thr Trp Pro Ser Gln Thr Val Thr
65                  70                  75                  80
Cys Ser Val Ala His Pro Ala Ser Ser Thr Thr Val Asp Lys Lys Leu
                85                  90                  95
Glu Pro Ser Gly
            100

<210> 36
<211> 101
<212> PRT
<213> Mus musculus


<400> 36
Pro Ser Val Phe Ile Phe Pro Pro Asn Ile Lys Asp Val Leu Met Ile
1               5                   10                  15
Ser Leu Thr Pro Lys Val Thr Cys Val Val Val Asp Val Ser Glu Asp
            20                  25                  30
Asp Pro Asp Val Gln Ile Ser Trp Phe Val Asn Asn Val Glu Val His
        35                  40                  45
Thr Ala Gln Thr Gln Thr His Arg Glu Asp Tyr Asn Ser Thr Ile Arg
    50                  55                  60
Val Val Ser Thr Leu Pro Ile Gln His Gln Asp Trp Met Ser Gly Lys
65                  70                  75                  80
Glu Phe Lys Cys Lys Val Asn Asn Lys Asp Leu Pro Ser Pro Ile Glu
                85                  90                  95
Arg Thr Ile Ser Lys

            100

<210> 37
<211> 97
<212> PRT
<213> Mus musculus


<400> 37
Pro Gln Val Tyr Ile Leu Pro Pro Pro Ala Glu Gln Leu Ser Arg Lys
1               5                   10                  15
Asp Val Ser Leu Thr Cys Leu Val Val Gly Phe Asn Pro Gly Asp Ile
            20                  25                  30
Ser Val Glu Trp Thr Ser Asn Gly His Thr Glu Glu Asn Tyr Lys Asp
        35                  40                  45
Thr Ala Pro Val Leu Asp Ser Asp Gly Ser Tyr Phe Ile Tyr Ser Lys
    50                  55                  60
Leu Asn Met Lys Thr Ser Lys Trp Glu Lys Thr Asp Ser Phe Ser Cys
65                  70                  75                  80
Asn Val Arg His Glu Gly Leu Lys Asn Tyr Tyr Leu Lys Lys Thr Ile
                85                  90                  95
Ser


<210> 38
<211> 100
<212> PRT
<213> Mus musculus


<400> 38
Thr Thr Thr Ala Pro Ser Val Tyr Pro Leu Val Pro Gly Cys Ser Asp
1               5                   10                  15
Thr Ser Gly Ser Ser Val Thr Leu Gly Cys Leu Val Lys Gly Tyr Phe
            20                  25                  30
Pro Glu Pro Val Thr Val Lys Trp Asn Tyr Gly Ala Leu Ser Ser Gly
        35                  40                  45
Val Arg Thr Val Ser Ser Val Leu Gln Ser Gly Phe Tyr Ser Leu Ser
    50                  55                  60
Ser Leu Val Thr Val Pro Ser Ser Thr Trp Pro Ser Gln Thr Val Ile
65                  70                  75                  80
Cys Asn Val Ala His Pro Ala Ser Lys Thr Glu Leu Ile Lys Arg Ile
                85                  90                  95
Glu Pro Arg Ile
            100

<210> 39
<211> 110
<212> PRT
<213> Mus musculus


<400> 39
Gly Asn Ile Leu Gly Gly Pro Ser Val Phe Ile Phe Pro Pro Lys Pro
1               5                   10                  15
Lys Asp Ala Leu Met Ile Ser Leu Thr Pro Lys Val Thr Cys Val Val
            20                  25                  30
Val Asp Val Ser Glu Asp Asp Pro Asp Val His Val Ser Trp Phe Val
        35                  40                  45
Asp Asn Lys Glu Val His Thr Ala Trp Thr Gln Pro Arg Glu Ala Gln
    50                  55                  60
Tyr Asn Ser Thr Phe Arg Val Val Ser Ala Leu Pro Ile Gln His Gln
65                  70                  75                  80
Asp Trp Met Arg Gly Lys Glu Phe Lys Cys Lys Val Asn Asn Lys Ala
                85                  90                  95
Leu Pro Ala Pro Ile Glu Arg Thr Ile Ser Lys Pro Lys Gly
            100                 105                 110

<210> 40

<211> 104
<212> PRT
<213> Mus musculus

<400> 40
Arg Ala Gln Thr Pro Gln Val Tyr Thr Ile Pro Pro Pro Arg Glu Gln
1               5                   10                  15
Met Ser Lys Lys Lys Val Ser Leu Thr Cys Leu Val Thr Asn Phe Phe
            20                  25                  30
Ser Glu Ala Ile Ser Val Glu Trp Glu Arg Asn Gly Glu Leu Glu Gln
        35                  40                  45
Asp Tyr Lys Asn Thr Pro Pro Ile Leu Asp Ser Asp Gly Thr Tyr Phe
    50                  55                  60
Leu Tyr Ser Lys Leu Thr Val Asp Thr Asp Ser Trp Leu Gln Gly Glu
65                  70                  75                  80
Ile Phe Thr Cys Ser Val Val His Glu Ala Leu His Asn His His Thr
                85                  90                  95
Gln Lys Asn Leu Ser Arg Ser Pro
            100

<210> 41
<211> 100
<212> PRT
<213> Oryctolagus cuniculus

<400> 41
Gly Gln Pro Lys Ala Pro Ser Val Phe Pro Leu Ala Pro Cys Cys Gly
1               5                   10                  15
Asp Thr Pro Ser Ser Thr Val Thr Leu Gly Cys Leu Val Lys Gly Tyr
            20                  25                  30
Leu Pro Glu Pro Val Thr Val Thr Trp Asn Ser Gly Thr Leu Thr Asn
        35                  40                  45
Gly Val Arg Thr Phe Pro Ser Val Arg Gln Ser Ser Gly Leu Tyr Ser
    50                  55                  60
Leu Ser Ser Val Val Ser Val Thr Ser Ser Ser Gln Pro Val Thr Cys
65                  70                  75                  80
Asn Val Ala His Pro Ala Thr Asn Thr Lys Val Asp Lys Thr Val Ala
                85                  90                  95
Pro Ser Thr Cys
            100

<210> 42
<211> 100
<212> PRT
<213> Oryctolagus cuniculus

<400> 42
Pro Ser Val Phe Ile Phe Pro Pro Lys Pro Lys Asp Thr Leu Met Ile
1               5                   10                  15
Ser Arg Thr Pro Glu Val Thr Cys Val Val Val Asp Val Ser Gln Asp
            20                  25                  30
Asp Pro Glu Val Gln Phe Thr Trp Tyr Ile Asn Asn Glu Gln Val Arg
        35                  40                  45
Thr Ala Arg Pro Pro Leu Arg Glu Gln Gln Phe Asn Ser Thr Ile Arg
    50                  55                  60
Val Val Ser Thr Leu Pro Ile Thr His Gln Asp Trp Leu Arg Gly Lys
65                  70                  75                  80
Glu Phe Lys Cys Lys Val His Asn Lys Ala Leu Pro Ala Pro Ile Glu
                85                  90                  95
Lys Thr Ile Ser
            100

<210> 43
<211> 97
<212> PRT
<213> Oryctolagus cuniculus

<400> 43
Pro Lys Val Tyr Thr Met Gly Pro Pro Arg Glu Glu Leu Ser Ser Arg
1               5                   10                  15
Ser Val Ser Leu Thr Cys Met Ile Asn Gly Phe Tyr Pro Ser Asp Ile
            20                  25                  30
Ser Val Glu Trp Glu Lys Asn Gly Lys Ala Glu Asp Asn Tyr Lys Thr
        35                  40                  45
Thr Pro Ala Val Leu Asp Ser Asp Gly Ser Tyr Phe Leu Tyr Asn Lys
    50                  55                  60
Leu Ser Val Pro Thr Ser Glu Trp Gln Arg Gly Asp Val Phe Thr Cys
65                  70                  75                  80
Ser Val Met His Glu Ala Leu His Asn His Tyr Thr Gln Lys Ser Ile
                85                  90                  95
Ser

<210> 44
<211> 103
<212> PRT
<213> Homo sapiens

<400> 44
Ala Ser Thr Gln Ser Pro Ser Val Phe Pro Leu Thr Arg Cys Cys Lys
1               5                   10                  15
Asn Ile Pro Ser Asn Ala Thr Ser Val Thr Leu Gly Cys Leu Ala Thr
            20                  25                  30
Gly Tyr Phe Pro Glu Pro Val Met Val Thr Trp Asp Thr Gly Ser Leu
        35                  40                  45
Asn Gly Thr Thr Met Thr Leu Pro Ala Thr Thr Leu Thr Leu Ser Gly
    50                  55                  60
His Tyr Ala Thr Ile Ser Leu Leu Thr Val Ser Gly Ala Trp Ala Lys
65                  70                  75                  80
Gln Met Phe Thr Cys Arg Val Ala His Thr Pro Ser Ser Thr Asp Trp
                85                  90                  95
Val Asp Asn Lys Thr Phe Ser
            100

<210> 45
<211> 99
<212> PRT
<213> Homo sapiens

<400> 45
Pro Thr Val Lys Ile Leu Gln Ser Ser Cys Asp Gly Gly Gly His Phe
1               5                   10                  15
Pro Pro Thr Ile Gln Leu Leu Cys Leu Val Ser Gly Tyr Thr Pro Gly
            20                  25                  30
Thr Ile Asn Ile Thr Trp Leu Glu Asp Gly Gln Val Met Asp Val Asp
        35                  40                  45
Leu Ser Thr Ala Ser Thr Thr Gln Glu Gly Glu Leu Ala Ser Thr Gln
    50                  55                  60
Ser Glu Leu Thr Leu Ser Gln Lys His Trp Leu Ser Asp Arg Thr Tyr
65                  70                  75                  80
Thr Cys Gln Val Thr Tyr Gln Gly His Thr Phe Glu Asp Ser Thr Lys
                85                  90                  95
Lys Cys Ala

<210> 46
<211> 105
<212> PRT
<213> Homo sapiens

<400> 46

Pro Arg Gly Val Ser Ala Tyr Leu Ser Arg Pro Ser Pro Phe Asp Leu
1               5                   10                  15
Phe Ile Arg Lys Ser Pro Thr Ile Thr Cys Leu Val Val Asp Leu Ala
            20                  25                  30
Pro Ser Lys Gly Thr Val Asn Leu Thr Trp Ser Arg Ala Ser Gly Lys
        35                  40                  45
Pro Val Asn His Ser Thr Arg Lys Glu Glu Lys Gln Arg Asn Gly Thr
    50                  55                  60
Leu Thr Val Thr Ser Thr Leu Pro Val Gly Thr Arg Asp Trp Ile Glu
65                  70                  75                  80
Gly Glu Thr Tyr Gln Cys Arg Val Thr His Pro His Leu Pro Arg Ala
                85                  90                  95
Leu Met Arg Ser Thr Thr Lys Thr Ser
            100                 105

<210> 47
<211> 100
<212> PRT
<213> Homo sapiens


<400> 47
Pro Glu Val Tyr Ala Phe Ala Thr Pro Glu Trp Pro Gly Ser Arg Asp
1               5                   10                  15
Lys Arg Thr Leu Ala Cys Leu Ile Gln Asn Phe Met Pro Glu Asp Ile
            20                  25                  30
Ser Val Gln Trp Leu His Asn Glu Val Gln Leu Pro Asp Ala Arg His
        35                  40                  45
Ser Thr Thr Gln Pro Arg Lys Thr Lys Gly Ser Gly Phe Phe Val Phe
    50                  55                  60
Ser Arg Leu Glu Val Thr Arg Ala Glu Trp Glu Gln Lys Asp Glu Phe
65                  70                  75                  80
Ile Cys Arg Ala Val His Glu Ala Ala Ser Pro Ser Gln Thr Val Gln
                85                  90                  95
Arg Ala Val Ser
            100

<210> 48
<211> 100
<212> PRT
<213> Homo sapiens


<400> 48
Ala Pro Thr Lys Ala Pro Asp Val Phe Pro Ile Ile Ser Gly Cys Arg
1               5                   10                  15
His Pro Lys Asp Asn Ser Pro Val Val Leu Ala Cys Leu Ile Thr Gly
            20                  25                  30
Tyr His Pro Thr Ser Val Thr Val Thr Trp Tyr Met Gly Thr Gln Ser
        35                  40                  45
Gln Pro Gln Arg Thr Phe Pro Glu Ile Gln Arg Arg Asp Ser Tyr Tyr
    50                  55                  60
Met Thr Ser Ser Gln Leu Ser Thr Pro Leu Gln Gln Trp Arg Gln Gly
65                  70                  75                  80
Glu Tyr Lys Cys Val Val Gln His Thr Ala Ser Lys Ser Lys Lys Glu
                85                  90                  95
Ile Phe Arg Trp
            100

<210> 49
<211> 88
<212> PRT
<213> Homo sapiens


<400> 49
Ala Val Gln Asp Leu Trp Leu Arg Asp Lys Ala Thr Phe Thr Cys Phe
1               5                   10                  15
Val Val Gly Ser Asp Leu Lys Asp Ala His Leu Thr Trp Glu Val Ala

                20                  25                  30
Gly Lys Val Pro Thr Gly Gly Val Glu Glu Gly Leu Leu Glu Arg His
            35                  40                  45
Ser Asn Gly Ser Gln Ser Gln His Ser Arg Leu Thr Leu Pro Arg Ser
    50                  55                  60
Leu Trp Asn Ala Gly Thr Ser Val Thr Cys Thr Leu Asn His Pro Ser
65                  70                  75                  80
Leu Pro Pro Gln Arg Leu Met Ala
                85

<210> 50
<211> 107
<212> PRT
<213> Homo sapiens


<400> 50
Pro Ala Ala Gln Ala Pro Val Lys Leu Ser Leu Asn Leu Leu Ala Ser
1               5                   10                  15
Ser Asp Pro Pro Glu Ala Ala Ser Trp Leu Leu Cys Glu Val Ser Gly
                20                  25                  30
Phe Ser Pro Pro Asn Ile Leu Leu Met Trp Leu Glu Asp Gln Arg Glu
            35                  40                  45
Val Asn Thr Ser Gly Phe Ala Pro Ala Arg Pro Pro Pro Gln Pro Gly
    50                  55                  60
Ser Thr Thr Phe Trp Ala Trp Ser Val Leu Arg Val Pro Ala Pro Pro
65                  70                  75                  80
Ser Pro Gln Pro Ala Thr Tyr Thr Cys Val Val Ser His Glu Asp Ser
                85                  90                  95
Arg Thr Leu Leu Asn Ala Ser Arg Ser Leu Glu
            100                 105

<210> 51
<211> 105
<212> PRT
<213> Homo sapiens


<400> 51
Gly Ser Ala Ser Ala Pro Thr Leu Phe Pro Leu Val Ser Cys Glu Asn
1               5                   10                  15
Ser Pro Ser Asp Thr Ser Ser Val Ala Val Gly Cys Leu Ala Gln Asp
                20                  25                  30
Phe Leu Pro Asp Ser Ile Thr Leu Ser Trp Lys Tyr Lys Asn Asn Ser
            35                  40                  45
Asp Ile Ser Ser Thr Arg Gly Phe Pro Ser Val Leu Arg Gly Gly Lys
    50                  55                  60
Tyr Ala Ala Thr Ser Gln Val Leu Leu Pro Ser Lys Asp Val Met Gln
65                  70                  75                  80
Gly Thr Asp Glu His Val Val Cys Lys Val Gln His Pro Asn Gly Asn
                85                  90                  95
Lys Glu Lys Asn Val Pro Leu Pro Val
            100                 105

<210> 52
<211> 112
<212> PRT
<213> Homo sapiens


<400> 52
Ile Ala Glu Leu Pro Pro Lys Val Ser Val Phe Val Pro Pro Arg Asp
1               5                   10                  15
Gly Phe Phe Gly Asn Pro Arg Lys Ser Lys Leu Ile Cys Gln Ala Thr
                20                  25                  30
Gly Phe Ser Pro Arg Gln Ile Gln Val Ser Trp Leu Arg Glu Gly Lys
            35                  40                  45
Gln Val Gly Ser Gly Val Thr Thr Asp Gln Val Gln Ala Glu Ala Lys
    50                  55                  60

Glu Ser Gly Pro Thr Thr Tyr Lys Val Thr Ser Thr Leu Thr Ile Lys
65                  70                  75                  80
Glu Ser Asp Trp Leu Gly Gln Ser Met Phe Thr Cys Arg Val Asp His
                85                  90                  95
Arg Gly Leu Thr Phe Gln Gln Asn Ala Ser Ser Met Cys Val Pro Asp
            100                 105                 110

<210> 53
<211> 106
<212> PRT
<213> Homo sapiens

<400> 53
Gln Asp Thr Ala Ile Arg Val Phe Ala Ile Pro Pro Ser Phe Ala Ser
1               5                   10                  15
Ile Phe Leu Thr Lys Ser Thr Lys Leu Thr Cys Leu Val Thr Asp Leu
            20                  25                  30
Thr Thr Tyr Asp Ser Val Thr Ile Ser Trp Thr Arg Gln Asn Gly Glu
        35                  40                  45
Ala Val Lys Thr His Thr Asn Ile Ser Glu Ser His Pro Asn Ala Thr
    50                  55                  60
Phe Ser Ala Val Gly Glu Ala Ser Ile Cys Glu Asp Asp Trp Asn Ser
65                  70                  75                  80
Gly Glu Arg Phe Thr Cys Thr Val Thr His Thr Asp Leu Pro Ser Pro
                85                  90                  95
Leu Lys Gln Thr Ile Ser Arg Pro Lys Gly
            100                 105

<210> 54
<211> 129
<212> PRT
<213> Homo sapiens

<400> 54
Val Ala Leu His Arg Pro Asp Val Tyr Leu Leu Pro Pro Ala Arg Glu
1               5                   10                  15
Gln Leu Asn Leu Arg Glu Ser Ala Thr Ile Thr Cys Leu Val Thr Gly
            20                  25                  30
Phe Ser Pro Ala Asp Val Phe Val Gln Trp Met Gln Arg Gly Gln Pro
        35                  40                  45
Leu Ser Pro Glu Lys Tyr Val Thr Ser Ala Pro Met Pro Glu Pro Gln
    50                  55                  60
Ala Pro Gly Arg Tyr Phe Ala His Ser Ile Leu Thr Val Ser Glu Glu
65                  70                  75                  80
Glu Trp Asn Thr Gly Glu Thr Tyr Thr Cys Val Ala His Glu Ala Leu
                85                  90                  95
Pro Asn Arg Val Thr Glu Arg Thr Val Asp Lys Ser Thr Gly Lys Pro
            100                 105                 110
Thr Leu Tyr Asn Val Ser Leu Val Met Ser Asp Thr Ala Gly Thr Cys
        115                 120                 125
Tyr

<210> 55
<211> 100
<212> PRT
<213> Homo sapiens

<400> 55
Ala Ser Pro Thr Ser Pro Lys Val Phe Pro Leu Ser Leu Cys Ser Thr
1               5                   10                  15
Gln Pro Asp Gly Asn Val Val Ile Ala Cys Leu Val Gln Gly Phe Phe
            20                  25                  30
Pro Gln Glu Pro Leu Ser Val Thr Trp Ser Glu Ser Gly Gln Gly Val

35                  40                  45
Thr Ala Arg Asn Phe Pro Pro Ser Gln Asp Ala Ser Gly Asp Leu Tyr
    50                  55                  60
Thr Thr Ser Ser Gln Leu Thr Leu Pro Ala Thr Gln Cys Leu Ala Gly
65                  70                  75                  80
Lys Ser Val Thr Cys His Val Lys His Tyr Thr Asn Pro Ser Gln Asp
                85                  90                  95
Val Thr Val Pro
            100

<210> 56
<211> 96
<212> PRT
<213> Homo sapiens


<400> 56
Pro Arg Leu Ser Leu His Arg Pro Ala Leu Glu Asp Leu Leu Leu Gly
1               5                   10                  15
Ser Glu Ala Asn Leu Thr Cys Thr Leu Thr Gly Leu Arg Asp Ala Ser
            20                  25                  30
Gly Val Thr Phe Thr Trp Thr Pro Ser Ser Gly Lys Ser Ala Val Gln
        35                  40                  45
Gly Pro Pro Glu Arg Asp Leu Cys Gly Cys Tyr Ser Val Ser Ser Val
    50                  55                  60
Leu Pro Gly Cys Ala Glu Pro Trp Asn His Gly Lys Thr Phe Thr Cys
65                  70                  75                  80
Thr Ala Ala Tyr Pro Glu Ser Lys Thr Pro Leu Thr Ala Thr Leu Ser
                85                  90                  95



<210> 57
<211> 103
<212> PRT
<213> Homo sapiens


<400> 57
Pro Glu Val His Leu Leu Pro Pro Pro Ser Glu Glu Leu Ala Leu Asn
1               5                   10                  15
Glu Leu Val Thr Leu Thr Cys Leu Ala Arg Gly Phe Ser Pro Lys Asp
            20                  25                  30
Val Leu Val Arg Trp Leu Gln Gly Ser Gln Glu Leu Pro Arg Glu Lys
        35                  40                  45
Tyr Leu Thr Trp Ala Ser Arg Gln Glu Pro Ser Gln Gly Thr Thr Thr
    50                  55                  60
Phe Ala Val Thr Ser Ile Leu Arg Val Ala Ala Glu Asp Trp Lys Lys
65                  70                  75                  80
Gly Asp Thr Phe Ser Cys Met Val Gly His Glu Ala Leu Pro Leu Ala
                85                  90                  95
Phe Thr Gln Lys Thr Ile Asp
            100

<210> 58
<211> 100
<212> PRT
<213> Homo sapiens


<400> 58
Ala Ser Pro Thr Ser Pro Lys Val Phe Pro Leu Ser Leu Asp Ser Thr
1               5                   10                  15
Pro Gln Asp Gly Asn Val Val Val Ala Cys Leu Val Gln Gly Phe Phe
            20                  25                  30
Pro Gln Glu Pro Leu Ser Val Thr Trp Ser Glu Ser Gly Gln Asn Val
        35                  40                  45
Thr Ala Arg Asn Phe Pro Pro Ser Gln Asp Ala Ser Gly Asp Leu Tyr
    50                  55                  60

Thr Thr Ser Ser Gln Leu Thr Leu Pro Ala Thr Gln Cys Pro Asp Gly
65                  70                  75                  80
Lys Ser Val Thr Cys His Val Lys His Tyr Thr Asn Pro Ser Gln Asp
                85                  90                  95
Val Thr Val Pro
            100

<210> 59
<211> 96
<212> PRT
<213> Homo sapiens


<400> 59
Pro Arg Leu Ser Leu His Arg Pro Ala Leu Glu Asp Leu Leu Leu Gly
1               5                   10                  15
Ser Glu Ala Asn Leu Thr Cys Thr Leu Thr Gly Leu Arg Asp Ala Ser
            20                  25                  30
Gly Ala Thr Phe Thr Trp Thr Pro Ser Ser Gly Lys Ser Ala Val Gln
        35                  40                  45
Gly Pro Pro Glu Arg Asp Leu Cys Gly Cys Tyr Ser Val Ser Ser Val
    50                  55                  60
Leu Pro Gly Cys Ala Gln Pro Trp Asn His Gly Glu Thr Phe Thr Cys
65                  70                  75                  80
Thr Ala Ala His Pro Glu Leu Lys Thr Pro Leu Thr Ala Asn Ile Thr
                85                  90                  95



<210> 60
<211> 103
<212> PRT
<213> Homo sapiens


<400> 60
Pro Glu Val His Leu Leu Pro Pro Pro Ser Glu Glu Leu Ala Leu Asn
1               5                   10                  15
Glu Leu Val Thr Leu Thr Cys Leu Ala Arg Gly Phe Ser Pro Lys Asp
            20                  25                  30
Val Leu Val Arg Trp Leu Gln Gly Ser Gln Glu Leu Pro Arg Glu Lys
        35                  40                  45
Tyr Leu Thr Trp Ala Ser Arg Gln Glu Pro Ser Gln Gly Thr Thr Thr
    50                  55                  60
Phe Ala Val Thr Ser Ile Leu Arg Val Ala Ala Glu Asp Trp Lys Lys
65                  70                  75                  80
Gly Asp Thr Phe Ser Cys Met Val Gly His Glu Ala Leu Pro Leu Ala
                85                  90                  95
Phe Thr Gln Lys Thr Ile Asp
            100

<210> 61
<211> 106
<212> PRT
<213> Rattus norvegicus


<400> 61
Ala Asp Ala Ala Pro Thr Val Ser Ile Phe Pro Pro Ser Met Glu Gln
1               5                   10                  15
Leu Thr Ser Gly Gly Ala Thr Val Val Cys Phe Val Asn Asn Phe Tyr
            20                  25                  30
Pro Arg Asp Ile Ser Val Lys Trp Lys Ile Asp Gly Ser Glu Gln Arg
        35                  40                  45
Asp Gly Val Leu Asp Ser Val Thr Asp Gln Asp Ser Lys Asp Ser Thr
    50                  55                  60
Tyr Ser Met Ser Ser Thr Leu Ser Leu Thr Lys Val Glu Tyr Glu Arg
65                  70                  75                  80
His Asn Leu Tyr Thr Cys Glu Val Val His Lys Thr Ser Ser Ser Pro

                 85                          90                          95
Val Val Lys Ser Phe Asn Arg Asn Glu Cys
            100                 105

<210> 62
<211> 103
<212> PRT
<213> Gallus gallus


<400> 62
Gln Pro Lys Val Ala Pro Thr Ile Thr Leu Phe Pro Pro Ser Lys Glu
1               5                   10                  15
Glu Leu Asn Glu Ala Thr Lys Ala Thr Leu Val Cys Leu Ile Asn Asp
            20                  25                  30
Phe Tyr Pro Ser Pro Val Thr Val Asp Trp Val Ile Asp Gly Ser Thr
        35                  40                  45
Arg Ser Gly Glu Thr Thr Ala Pro Gln Arg Gln Ser Asn Ser Gln Tyr
    50                  55                  60
Met Ala Ser Ser Tyr Leu Ser Leu Ser Ala Ser Asp Trp Ser Ser His
65                  70                  75                  80
Glu Thr Tyr Thr Cys Arg Val Thr His Asn Gly Thr Ser Ile Thr Lys
                85                  90                  95
Thr Leu Lys Arg Ser Glu Cys
            100

<210> 63
<211> 100
<212> PRT
<213> Equus caballus


<400> 63
Ser Val Phe Ile Phe Pro Pro Asn Pro Lys Asp Thr Leu Met Ile Thr
1               5                   10                  15
Arg Thr Pro Glu Val Thr Cys Val Val Val Asp Val Ser Gln Glu Asn
            20                  25                  30
Pro Asp Val Lys Phe Asn Trp Tyr Met Asp Gly Val Glu Val Arg Thr
        35                  40                  45
Ala Thr Thr Arg Pro Lys Glu Glu Gln Phe Asn Ser Thr Tyr Arg Val
    50                  55                  60
Val Ser Val Leu Arg Ile Gln His Gln Asp Trp Leu Ser Gly Lys Glu
65                  70                  75                  80
Phe Lys Cys Lys Val Asn Asn Gln Ala Leu Pro Gln Pro Ile Glu Arg
                85                  90                  95
Thr Ile Thr Lys
            100

<210> 64
<211> 100
<212> PRT
<213> Ailuropoda melanoleuca


<400> 64
Ser Thr Thr Ala Pro Ser Val Phe Pro Leu Ala Pro Ser Cys Gly Val
1               5                   10                  15
Thr Ser Gly Ser Thr Val Ala Leu Ser Cys Leu Val Ser Gly Tyr Phe
            20                  25                  30
Pro Glu Pro Val Thr Val Ser Trp Asn Ser Gly Ser Val Thr Thr Phe
        35                  40                  45
Pro Ser Val Leu Gln Ser Ser Gly Leu Tyr Ser Leu Ser Ser Met Val
    50                  55                  60
Thr Val Pro Ser Ser Arg Trp Pro Ser Asp Thr Phe Thr Cys Thr Val
65                  70                  75                  80
Ala His Pro Ala Ser Asn Thr Lys Val Glu Lys Thr Val Pro Lys Lys
                85                  90                  95
Gly Pro Cys Lys
            100

<210> 65
<211> 110
<212> PRT
<213> Ailuropoda melanoleuca

<400> 65
Pro Ala Pro Glu Met Leu Gly Gly Pro Ser Val Phe Ile Phe Pro Pro
1               5                   10                  15
Lys Ala Lys Asp Ile Leu Lys Ile Ala Arg Thr Pro Glu Val Thr Cys
            20                  25                  30
Val Val Val Asp Leu Glu Asp Pro Glu Val Gln Ile Ser Trp Phe Val
        35                  40                  45
Asp Asp Gln Glu Val Arg Thr Ala Lys Thr Asn Pro Arg Glu Glu Gln
    50                  55                  60
Phe Asn Ser Thr Tyr Arg Val Val Ser Val Leu Pro Ile Gln His Gln
65                  70                  75                  80
Asp Trp Leu Ala Gly Lys Glu Phe Lys Cys Lys Val Asn Asn Lys Ala
                85                  90                  95
Leu Pro Ser Pro Ile Val Arg Thr Ile Ser Lys Ala Arg Gly
            100                 105                 110

<210> 66
<211> 106
<212> PRT
<213> Ailuropoda melanoleuca

<400> 66
Gln Ala Gln Gln Pro Asn Val Tyr Val Leu Pro Pro Pro Arg Asp Glu
1               5                   10                  15
Met Asn Lys Asn Thr Val Ser Val Thr Cys Leu Val Lys Asp Phe Tyr
            20                  25                  30
Pro Pro Asp Val Asp Val Glu Trp Gln Ser Asn Gly Gln Gln Val Pro
        35                  40                  45
Glu Thr Asn Ile Arg Thr Thr Pro Pro Gln Leu Asp Ala Asp Gly Thr
    50                  55                  60
Tyr Phe Leu Tyr Ser Lys Leu Ser Val Asp Lys Asn Arg Trp His Arg
65                  70                  75                  80
Gly Glu Val Phe Thr Cys Ala Val Met His Glu Ala Leu His Asn His
                85                  90                  95
His Thr Gln Lys Ser Ile Ser Gln Ser Pro
            100                 105

<210> 67
<211> 100
<212> PRT
<213> Xenopus laevis

<400> 67
Leu Gln Val Tyr Pro Leu Thr Pro Thr Phe His Asp Leu Tyr Phe Ser
1               5                   10                  15
Arg Asn Ala Lys Ile Thr Cys Leu Val Ser Ser Met Lys Thr Ile Glu
            20                  25                  30
Asn Phe Asp Ile Ser Trp Glu Arg Glu Lys Ala Gly Asn Leu Glu Phe
        35                  40                  45
Val Thr Glu Asp Pro Val Leu His Asp Asn Gly Thr Tyr Ser Val Ala
    50                  55                  60
Ser Ile Leu Ser Val Cys Ala Glu Asp Trp Glu Ser Gly Asp Lys Phe
65                  70                  75                  80
Ser Cys Thr Val Arg Ser Gln Asp Leu Pro Ser Pro Val Lys Lys Thr
                85                  90                  95
Ile Phe Lys Gln
            100

<210> 68
<211> 4

<212> PRT
<213> Artificial Sequence

<220>
<223> loop sequence

<400> 68
Arg Lys Lys Gly
1

<210> 69
<211> 4
<212> PRT
<213> Artificial Sequence

<220>
<223> loop sequence

<400> 69
Arg Asn Glu Gly
1

<210> 70
<211> 4
<212> PRT
<213> Artificial Sequence

<220>
<223> loop sequence

<400> 70
Gln Ser Gly Asn
1

<210> 71
<211> 4
<212> PRT
<213> Artificial Sequence

<220>
<223> loop sequence

<400> 71
Val Lys Ala Gly
1

<210> 72
<211> 4
<212> PRT
<213> Artificial Sequence

<220>
<223> loop sequence

<400> 72
Ala Lys Ala Gly
1

<210> 73
<211> 4
<212> PRT
<213> Artificial Sequence

<220>
<223> loop sequence

<400> 73
Arg Gln Asn Gly
1

<210> 74
<211> 4
<212> PRT
<213> Artificial Sequence

<220>
<223> loop sequence

<400> 74
Val Thr Gln Gly
1

<210> 75
<211> 4
<212> PRT
<213> Artificial Sequence

<220>
<223> loop sequence

<400> 75
Ile Thr Gln Gly
1

<210> 76
<211> 4
<212> PRT
<213> Artificial Sequence

<220>
<223> loop sequence

<400> 76
Gln Thr Thr Gly
1

<210> 77
<211> 4
<212> PRT
<213> Artificial Sequence

<220>
<223> loop sequence

<400> 77
Leu Thr Thr Gly
1

<210> 78
<211> 4
<212> PRT
<213> Artificial Sequence

<220>
<223> loop sequence

<400> 78
Gln Gln Ser Gly
1

<210> 79
<211> 4
<212> PRT
<213> Artificial Sequence

<220>
<223> loop sequence

<400> 79
Ser Gly Val His
1

<210> 80
<211> 4
<212> PRT
<213> Artificial Sequence

<220>
<223> loop sequence

<400> 80
Val His Asn Ala
1

<210> 81
<211> 4
<212> PRT
<213> Artificial Sequence

<220>
<223> loop sequence

<400> 81
Pro Glu Asn Asn
1

<210> 82
<211> 4
<212> PRT
<213> Artificial Sequence

<220>
<223> loop sequence

<400> 82
Val His Thr Ala
1

<210> 83
<211> 4
<212> PRT
<213> Artificial Sequence

<220>
<223> loop sequence

<400> 83
Pro Ala Glu Asn
1

<210> 84
<211> 4
<212> PRT
<213> Artificial Sequence

<220>
<223> loop sequence

<400> 84
Thr Glu Leu Asn
1

<210> 85
<211> 4
<212> PRT
<213> Artificial Sequence

<220>
<223> loop sequence

<400> 85
Ser Ser Val His
1

<210> 86
<211> 4
<212> PRT
<213> Artificial Sequence

<220>
<223> loop sequence

<400> 86
Thr Glu Glu Asn
1

<210> 87
<211> 4
<212> PRT
<213> Artificial Sequence

<220>
<223> loop sequence

<400> 87
Ser Gly Val Arg
1

<210> 88
<211> 4
<212> PRT
<213> Artificial Sequence

<220>
<223> loop sequence

<400> 88
Leu Glu Gln Asp
1

<210> 89
<211> 4
<212> PRT
<213> Artificial Sequence

<220>
<223> loop sequence

<400> 89
Asn Gly Val Arg
1

<210> 90
<211> 4
<212> PRT
<213> Artificial Sequence

<220>
<223> loop sequence

<400> 90
Val Arg Thr Ala
1

<210> 91
<211> 4

<212> PRT
<213> Artificial Sequence

<220>
<223> loop sequence

<400> 91
Ala Glu Asp Asn
1

<210> 92
<211> 4
<212> PRT
<213> Artificial Sequence

<220>
<223> loop sequence

<400> 92
Gly Thr Thr Met
1

<210> 93
<211> 4
<212> PRT
<213> Artificial Sequence

<220>
<223> loop sequence

<400> 93
Pro Val Asn His
1

<210> 94
<211> 4
<212> PRT
<213> Artificial Sequence

<220>
<223> loop sequence

<400> 94
Leu Pro Asp Ala
1

<210> 95
<211> 4
<212> PRT
<213> Artificial Sequence

<220>
<223> loop sequence

<400> 95
Gln Pro Gln Arg
1

<210> 96
<211> 4
<212> PRT
<213> Artificial Sequence

<220>
<223> loop sequence

<400> 96
Pro Thr Gly Gly
1

<210> 97
<211> 4
<212> PRT
<213> Artificial Sequence

<220>
<223> loop sequence

<400> 97
Val Asn Thr Ser
1

<210> 98
<211> 4
<212> PRT
<213> Artificial Sequence

<220>
<223> loop sequence

<400> 98
Asp Ile Ser Ser
1

<210> 99
<211> 4
<212> PRT
<213> Artificial Sequence

<220>
<223> loop sequence

<400> 99
Val Gly Ser Gly
1

<210> 100
<211> 4
<212> PRT
<213> Artificial Sequence

<220>
<223> loop sequence

<400> 100
Ala Val Lys Thr
1

<210> 101
<211> 4
<212> PRT
<213> Artificial Sequence

<220>
<223> loop sequence

<400> 101
Leu Ser Pro Glu
1

<210> 102
<211> 4
<212> PRT
<213> Artificial Sequence

<220>
<223> loop sequence

<400> 102
Val Thr Ala Arg
1

<210> 103
<211> 4
<212> PRT
<213> Artificial Sequence

<220>
<223> loop sequence

<400> 103
Ser Ala Val Gln
1

<210> 104
<211> 4
<212> PRT
<213> Artificial Sequence

<220>
<223> loop sequence

<400> 104
Leu Pro Arg Glu
1

<210> 105
<211> 4
<212> PRT
<213> Artificial Sequence

<220>
<223> loop sequence

<400> 105
Gln Arg Asp Gly
1

<210> 106
<211> 4
<212> PRT
<213> Artificial Sequence

<220>
<223> loop sequence

<400> 106
Arg Ser Gly Glu
1

<210> 107
<211> 4
<212> PRT
<213> Artificial Sequence

<220>
<223> loop sequence

<400> 107
Gln Ser Ser Gly
1

<210> 108
<211> 4
<212> PRT
<213> Artificial Sequence

<220>
<223> loop sequence

<400> 108
Val Pro Glu Thr
1

<210> 109
<211> 4
<212> PRT
<213> Artificial Sequence

<220>
<223> loop sequence

<400> 109
Asn Leu Glu Phe
1

<210> 110
<211> 5
<212> PRT
<213> Artificial Sequence

<220>
<223> helix sequence

<400> 110
Ala Ser Glu Trp Asp
1               5

<210> 111
<211> 5
<212> PRT
<213> Artificial Sequence

<220>
<223> helix sequence

<400> 111
Val Glu Glu Trp Gln
1               5

<210> 112
<211> 5
<212> PRT
<213> Artificial Sequence

<220>
<223> helix sequence

<400> 112
Lys Ala Asp Tyr Glu
1               5

<210> 113
<211> 5
<212> PRT
<213> Artificial Sequence

<220>
<223> helix sequence

<400> 113
Pro Glu Gln Trp Lys
1               5

<210> 114
<211> 5

<212> PRT
<213> Artificial Sequence

<220>
<223> helix sequence

<400> 114
Lys Asp Glu Tyr Glu
1               5

<210> 115
<211> 5
<212> PRT
<213> Artificial Sequence

<220>
<223> helix sequence

<400> 115
Ala Arg Ala Trp Glu
1               5

<210> 116
<211> 5
<212> PRT
<213> Artificial Sequence

<220>
<223> helix sequence

<400> 116
Ser Asp Gln Trp Arg
1               5

<210> 117
<211> 5
<212> PRT
<213> Artificial Sequence

<220>
<223> helix sequence

<400> 117
Ser Thr Gln Tyr Asn
1               5

<210> 118
<211> 5
<212> PRT
<213> Artificial Sequence

<220>
<223> helix sequence

<400> 118
Lys Ser Asn Tyr Asn
1               5

<210> 119
<211> 5
<212> PRT
<213> Artificial Sequence

<220>
<223> helix sequence

<400> 119
Ser Asp Glu Tyr Asn
1               5

<210> 120
<211> 5
<212> PRT
<213> Artificial Sequence

<220>
<223> helix sequence

<400> 120
Lys Ala Gln Tyr Asn
1               5

<210> 121
<211> 5
<212> PRT
<213> Artificial Sequence

<220>
<223> helix sequence

<400> 121
Ser Ala Gln Tyr Asn
1               5

<210> 122
<211> 5
<212> PRT
<213> Artificial Sequence

<220>
<223> helix sequence

<400> 122
Ala Asn Gln Trp Lys
1               5

<210> 123
<211> 5
<212> PRT
<213> Artificial Sequence

<220>
<223> helix sequence

<400> 123
Ser Ser Ser Leu Gly
1               5

<210> 124
<211> 5
<212> PRT
<213> Artificial Sequence

<220>
<223> helix sequence

<400> 124
His Gln Asp Trp Leu
1               5

<210> 125
<211> 5
<212> PRT
<213> Artificial Sequence

<220>
<223> helix sequence

<400> 125
Lys Ser Arg Trp Gln
1               5

<210> 126
<211> 5
<212> PRT
<213> Artificial Sequence

<220>
<223> helix sequence

<400> 126
Ser Ser Asn Phe Gly
1               5

<210> 127
<211> 5
<212> PRT
<213> Artificial Sequence

<220>
<223> helix sequence

<400> 127
Ser Ser Pro Arg Pro
1               5

<210> 128
<211> 5
<212> PRT
<213> Artificial Sequence

<220>
<223> helix sequence

<400> 128
Lys Ser Asn Trp Glu
1               5

<210> 129
<211> 5
<212> PRT
<213> Artificial Sequence

<220>
<223> helix sequence

<400> 129
Ser Ser Thr Trp Pro
1               5

<210> 130
<211> 5
<212> PRT
<213> Artificial Sequence

<220>
<223> helix sequence

<400> 130
His Gln Asp Trp Met
1               5

<210> 131
<211> 5
<212> PRT
<213> Artificial Sequence

<220>
<223> helix sequence

<400> 131
Lys Lys Asn Trp Val
1               5

<210> 132
<211> 5
<212> PRT
<213> Artificial Sequence

<220>
<223> helix sequence

<400> 132
Thr Ser Lys Trp Glu
1               5

<210> 133
<211> 5
<212> PRT
<213> Artificial Sequence

<220>
<223> helix sequence

<400> 133
Thr Ser Ser Ser Gln
1               5

<210> 134
<211> 5
<212> PRT
<213> Artificial Sequence

<220>
<223> helix sequence

<400> 134
Thr Ser Glu Trp Gln
1               5

<210> 135
<211> 5
<212> PRT
<213> Artificial Sequence

<220>
<223> helix sequence

<400> 135
Ser Gly Ala Trp Ala
1               5

<210> 136
<211> 5
<212> PRT
<213> Artificial Sequence

<220>
<223> helix sequence

<400> 136
Gln Lys His Trp Leu
1               5

<210> 137
<211> 5

<212> PRT
<213> Artificial Sequence

<220>
<223> helix sequence

<400> 137
Thr Arg Asp Trp Ile
1               5

<210> 138
<211> 5
<212> PRT
<213> Artificial Sequence

<220>
<223> helix sequence

<400> 138
Arg Ala Glu Trp Glu
1               5

<210> 139
<211> 5
<212> PRT
<213> Artificial Sequence

<220>
<223> helix sequence

<400> 139
Leu Gln Gln Trp Arg
1               5

<210> 140
<211> 5
<212> PRT
<213> Artificial Sequence

<220>
<223> helix sequence

<400> 140
Arg Ser Leu Trp Asn
1               5

<210> 141
<211> 5
<212> PRT
<213> Artificial Sequence

<220>
<223> helix sequence

<400> 141
Ala Pro Pro Ser Pro
1               5

<210> 142
<211> 5
<212> PRT
<213> Artificial Sequence

<220>
<223> helix sequence

<400> 142
Ser Lys Asp Val Met
1               5

<210> 143
<211> 5
<212> PRT
<213> Artificial Sequence

<220>
<223> helix sequence

<400> 143
Glu Ser Asp Trp Leu
1               5

<210> 144
<211> 5
<212> PRT
<213> Artificial Sequence

<220>
<223> helix sequence

<400> 144
Glu Asp Asp Trp Asn
1               5

<210> 145
<211> 5
<212> PRT
<213> Artificial Sequence

<220>
<223> helix sequence

<400> 145
Glu Glu Glu Trp Asn
1               5

<210> 146
<211> 5
<212> PRT
<213> Artificial Sequence

<220>
<223> helix sequence

<400> 146
Thr Gln Cys Leu Ala
1               5

<210> 147
<211> 5
<212> PRT
<213> Artificial Sequence

<220>
<223> helix sequence

<400> 147
Ala Glu Pro Trp Asn
1               5

<210> 148
<211> 5
<212> PRT
<213> Artificial Sequence

<220>
<223> helix sequence

<400> 148
Ala Glu Asp Trp Lys
1               5

<210> 149
<211> 5
<212> PRT
<213> Artificial Sequence

<220>
<223> helix sequence

<400> 149
Ala Thr Gln Cys Pro
1               5

<210> 150
<211> 5
<212> PRT
<213> Artificial Sequence

<220>
<223> helix sequence

<400> 150
Ala Gln Pro Trp Asn
1               5

<210> 151
<211> 5
<212> PRT
<213> Artificial Sequence

<220>
<223> helix sequence

<400> 151
Lys Val Glu Tyr Glu
1               5

<210> 152
<211> 5
<212> PRT
<213> Artificial Sequence

<220>
<223> helix sequence

<400> 152
Ala Ser Asp Trp Ser
1               5

<210> 153
<211> 5
<212> PRT
<213> Artificial Sequence

<220>
<223> helix sequence

<400> 153
Ser Ser Arg Trp Pro
1               5

<210> 154
<211> 5
<212> PRT
<213> Artificial Sequence

<220>
<223> helix sequence

<400> 154
Lys Asn Arg Trp His
1               5

<210> 155
<211> 5
<212> PRT
<213> Artificial Sequence

<220>
<223> helix sequence

<400> 155
Ala Glu Asp Trp Glu
1               5

## Description

The present invention relates to a method of producing a modified protein having an increased stability and/or improved folding efficiency as compared to the unmodified protein, the method comprising (i) modifying a nucleic acid molecule encoding a protein comprising at least one immunoglobulin constant domain-like region by (a-i) replacing the nucleotides encoding at least one amino acid, preferably an uncharged amino acid, in the loop separating the C strand from the D strand with nucleotides encoding a charged amino acid selected from the group consisting of Arg, Lys, His, Glu and Asp and/or replacing the nucleotides encoding at least one amino acid, preferably an uncharged amino acid, in the helix connecting the E strand with the F strand with nucleotides encoding a charged amino acid selected from the group consisting of Arg, Lys, His, Glu and Asp; (a-ii) replacing the nucleotides encoding at least one amino acid not having a side chain that can form a hydrogen bond in the loop separating the C strand from the D strand with nucleotides encoding an amino acid having a side chain capable of forming a hydrogen bond selected from the group consisting of Gln, Asn, Tyr, Ser and Thr and/or replacing the nucleotides encoding at least one amino acid not having a side chain that can form a hydrogen bond in the helix connecting the E strand with the F strand with nucleotides encoding an amino acid having a side chain capable of forming a hydrogen bond selected from the group consisting of Gln, Asn, Tyr, Ser and Thr; and/or (a-iii) replacing the nucleotides encoding at least one amino acid in the loop separating the C strand from the D strand with nucleotides encoding a cysteine and/or replacing the nucleotides encoding at least one amino acid in the helix connecting the E strand with the F strand with nucleotides encoding a cysteine; and/or (b) replacing the nucleotides encoding at least one non-hydrophobic amino acid at a position suitable to participate in the formation of the hydrophobic core with nucleotides encoding a hydrophobic amino acid selected from the group consisting of Val, Ile, Leu, Met, Phe, Trp and Pro; and (ii) expressing the nucleic acid molecule to produce the stabilised protein. The present invention further relates to a method of producing a modified protein having an improved secretion from cells as compared to the unmodified protein, as well as to a protein comprising at least one immunoglobulin constant domain-like region having an additional salt bridge, an additional hydrogen bond, an additional disulfide bridge and/or an extended hydrophobic core. The present invention further relates to a nucleic acid molecule encoding the modified protein of the invention, as well as a vector comprising said nucleic acid molecule and a host cell comprising the vector. Further, the present invention relates also to a composition as well as to a kit.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Proteins are large molecules made up of a primary sequence defined by their amino acid sequence, which is dictated by the nucleotide sequence of their genes, and which usually results in folding of the protein into a specific three-dimensional structure that determines its activity. The native three-dimensional structure of a protein is the result of an intricate balance among various interactions including hydrophobic interactions, covalent linkages, electrostatic interactions (charge repulsion and ion pairing = salt bridge), hydrogen bonding and van der Waals forces. Since the folded state is only marginally more stable than the unfolded state, any change in the protein environment may trigger protein degradation or inactivation.

Protein stability is a result of balancing between destabilizing and stabilizing forces. The destabilizing forces are mainly due to the large increase in entropy upon unfolding, and the stabilizing forces are provided by mostly non-covalent interactions. Disruption of any of these interactions will shift the balance and destabilize a protein and many factors are known that disrupt this delicate balance and affect protein stability. These include for example temperature, pH, ionic strength, metal ions, surface adsorption, shearing, shaking, additives, solvents, protein concentration, purity, morphism, pressure, and freeze/thawing-drying. Chemical transformations that can lead to protein instability include e.g. deamidation, oxidation, hydrolysis, isomerization, succinimidation, disulfide bond formation or breakage, non-disulfide crosslinking, and deglycosylation. One of the most challenging tasks in the development of protein pharmaceuticals and diagnostics therefore is to deal with their physical and chemical instabilities and to provide more stable proteins.

A commonly applied method of protein stabilization is the addition of excipients. Their stabilizing effects are concentration-and protein-dependent, although high concentrations of excipients may not be necessarily more effective, and in some cases, can have negative effects. Frequently used protein stabilizers include sugars and polyols, amino acids, amines, salts, polymers and surfactants, each of which may exert different stabilizing effects.

A different, although typically more complex, approach is based on structural modifications of the proteins of interest. For antibodies and for other proteins comprising Ig-like domains, such methods typically focus on the variable regions (see e.g. (Holliger and Hudson, 2005)). A common approach used to improve the stability of a protein of interest is a combination of random mutagenesis with a subsequent selection assay. This approach allows a high throughput assessment of different mutants and does not rely on previous knowledge of the structure or stabilizing forces of the specific protein. It is generally limited, however, to single point mutations. Structure-based optimizations could overcome this limitation and could provide a complementary approach of particular relevance for proteins. To this end, proteins in general, and antibodies in particular, have been stabilized by the addition of additional covalent linkages in the form of disulfide bridges, as described e.g. in (Ewert et al., 2004).

Protein stability plays a major role in protein secretion from protein-producing cells, together with other factors such as e.g. the protein's folding kinetics and its capability to pass the cellular quality control checkpoints. Generally, improving these characteristics can lead to improved secretion of a protein of interest and thus its yield. If protein assembly is a prerequisite for secretion, improved assembly kinetics or thermodynamics might be additional factors. Typically, improved protein secretion can be obtained by either improving the biophysical characteristics of the protein of interest or by altering the cellular folding environment, e.g. by overexpression of molecular chaperones.

However, despite the fact that a lot of effort has been invested into improving the properties of proteins, there is still a need to identify satisfying methods for enhancing their stability and/or secretion.

This need is addressed by the provision of the embodiments characterized in the claims.

Accordingly, the present invention relates to a method of producing a modified protein having an increased stability and/or folding efficiency as compared to the unmodified protein, the method comprising (i) modifying a nucleic acid molecule encoding a protein comprising at least one immunoglobulin constant domain-like region by
(a-i) replacing the nucleotides encoding at least one amino acid, preferably an uncharged amino acid, in the loop separating the C strand from the D strand with nucleotides encoding a charged amino acid selected from the group consisting of Arg, Lys, His, Glu and Asp and/or replacing the nucleotides encoding at least one amino acid, preferably an uncharged amino acid, in the helix connecting the E strand with the F strand with nucleotides encoding a charged amino acid selected from the group consisting of Arg, Lys, His, Glu and Asp;
(a-ii) replacing the nucleotides encoding at least one amino acid not having a side chain that can form a hydrogen bond in the loop separating the C strand from the D strand with nucleotides encoding an amino acid having a side chain capable of forming a hydrogen bond selected from the group consisting of Gin, Asn, Tyr, Ser and Thr and/or replacing the nucleotides encoding at least one amino acid not having a side chain that can form a hydrogen bond in the helix connecting the E strand with the F strand with nucleotides encoding an amino acid having a side chain capable of forming a hydrogen bond selected from the group consisting of Gln, Asn, Tyr, Ser and Thr; and/or
(a-iii) replacing the nucleotides encoding at least one amino acid in the loop separating the C strand from the D strand with nucleotides encoding a cysteine and/or replacing the nucleotides encoding at least one amino acid in the helix connecting the E strand with the F strand with nucleotides encoding a cysteine; and/or
(b) replacing the nucleotides encoding at least one non-hydrophobic amino acid at a position suitable to participate in the formation of the hydrophobic core with nucleotides encoding a hydrophobic amino acid selected from the group consisting of Val, Ile, Leu, Met, Phe, Trp and Pro; and
(ii) expressing the nucleic acid molecule to produce the stabilised protein.

Unless defined otherwise, all terms regarding the abbreviations and the nature of amino acids are used herein in accordance with the pertinent art. The abbreviations are based on established nomenclature employed in the art, which is shown in the following table 1:

**Table 1: Amino acids and their abbreviations**

| **Amino Acid** | **three-letter code** | **one-letter code** |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The term "protein" as used herein refers to any high molecular mass compound consisting of one or more linear chains of the 20 amino acids of the genetic code joined by peptide bonds, occurring in living systems. The amino acids in the chains may be naturally modified by e.g. glycosylation, acetylation, phosphorylation and similar modifications, which are well known in the art.

The term "protein" as used herein includes proteins soluble in aqueous solvents, like soluble proteins comprising immunoglobulin domains such as soluble antibodies, as well as membrane proteins such as surface receptors bearing one or more immunoglobulin domains. The term "protein" refers to single-domain proteins as well as to multi-domain proteins. Proteins may also form dimers, trimers and higher oligomers, i.e. they can consist of more than one protein chain. Protein molecules forming such dimers, trimers etc. may be identical or non-identical. The corresponding higher order structures are, consequently, termed homo- or heterodimers, homo- or heterotrimers etc. Such homo- or heteromers also fall under the definition of the term "protein".

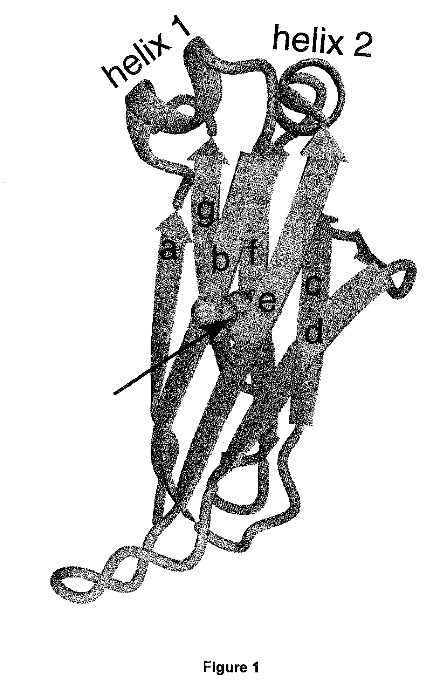

The protein to be modified, i.e. the starting protein subjected to the claimed method, is a protein comprising at least one immunoglobulin constant domain-like region. In accordance with the present invention, an "immunoglobulin constant domain-like region" is a region resembling an antibody constant domain in that it shows the typical β-sheet sandwich immunoglobulin structure described in the literature (Bork et al., 1994) and that has an immunoglobulin folding pattern of constant antibody domains which typically comprises a conserved buried disulfide bridge perpendicular to the layers of the β-sandwich and a conserved tryptophan residue adjacent to this disulfide bridge in the hydrophobic core. Generally, constant antibody domains show a greek key topology and are made up of seven β-strands (A-G), however, the details of the fold may vary (Bork et al., 1994). These strands are connected by either loops or helical elements in varying occurrence. A constant domain immunoglobulin structure is shown in Figure 1.

Such a protein comprising at least one immunoglobulin constant domain-like region can be an antibody or a non-antibody protein.

The term "antibody", as used in accordance with the present invention, relates to a specific type of proteins, the immunoglobulins, which are capable of recognizing a unique part of a target, the antigen. The term "antibody" includes polyclonal or monoclonal antibodies as well as derivatives thereof which retain their binding specificity. The term also includes synthetic, chimeric, single chain and humanized antibodies or derivatives or fragments thereof, which still retain their binding specificity. Fragments of antibodies comprise, amongst others, Fab or Fab' fragments, Fc, Fd, F(ab')₂, as well as multimeric formats such as minibodies or chemically conjugated Fab'-multimers (see, for example, (Altshuler et al., 2010) and (Holliger and Hudson, 2005)). The term "antibody" also includes embodiments such as chimeric (human constant domain, non-human variable domain), single chain and humanized (human antibody with the exception of non-human CDRs) antibodies, as well as heavy chain antibodies such as found in camelids or sharks, which consist of only heavy chains. Most preferably, the antibody is a monoclonal antibody, such as a human or humanized antibody.

Various techniques for the production of antibodies and fragments thereof are well known in the art and described, e.g. in (Altshuler et a[., 2010). Thus, polyclonal antibodies can be obtained from the blood of an animal following immunisation with an antigen in mixture with additives and adjuvans and monoclonal antibodies can be produced by any technique, which provides antibodies produced by continuous cell line cultures. Examples for such techniques are described, e.g. (Harlow and Lane, 1988, 1999) and include the hybridoma technique originally described by Köhler and Milstein (Kohler and Milstein, 1975) the trioma technique, the human B-cell hybridoma technique (see e.g. (Kozbor et al., 1983)) and the EBV-hybridoma technique to produce human monoclonal antibodies ((Cole et al., 1985)). Furthermore, recombinant antibodies may be obtained from monoclonal antibodies or can be prepared *de novo* using various display methods such as phage, ribosomal, mRNA, or cell display. A suitable system for the expression of the recombinant (humanized) antibodies or fragments thereof may be selected from, for example, bacteria, yeast, insects, mammalian cell lines or transgenic animals or plants (see, e.g., US patent 6,080,560; (Holliger and Hudson, 2005)). Further, techniques described for the production of single chain antibodies (see, inter alia, US Patent 4,946,778) can be adapted to produce single chain antibodies specific for the desired target. Surface plasmon resonance as employed in the BIAcore system can be used to identify variants with increased affinity, for example in the context of screening antibodies displayed by phages.

The antibody may be any class of antibody. Preferably, the antibody is monoclonal. More preferably, the antibody is of the IgG, IgM, IgA, IgD, IgE or IgY class or is an antibody consisting of heavy-chains only. Most preferably, the antibody is of the IgG class.

In an alternative embodiment of the method of the invention, the protein comprising at least one immunoglobulin constant domain-like region can also be a protein that is not an antibody. Non-limiting examples of such non-antibody proteins comprising at least one immunoglobulin constant domain-like region include interleukins and interleukin receptors.

In an even more preferred embodiment of the method of the invention, the protein comprising at least one immunoglobulin constant domain-like region is a eukaryotic protein. Eukaryotic proteins are well known in the art and include, e.g. animals, plants and fungi. Proteins having at least one immunoglobulin constant domain-like region have been described for many of these eukaryotes, for example in (Muller et al., 2001), where a receptor protein tyrosine phosphatase was cloned from the marine demosponge Geodia cydonium, the phylogenetically oldest metazoan taxon or in amphibians, reptiles and fishes ((Cooper and Alder, 2006; Flajnik and Kasahara, 2010)).

More preferably, the eukaryotic protein is a mammalian protein, even more preferably a protein selected from the group consisting of rodent proteins, camelide proteins and primate proteins and most preferably selected from the group consisting of mouse proteins, rabbit proteins and human proteins.

The term comprising, as used herein, denotes that further steps and/or components can be included in addition to the specifically recited steps and/or components. However, this term also encompasses that the claimed subject-matter consists of exactly the recited steps and/or components.

The term "at least" as used herein, such as e.g. the term "at least one" refers to the specifically recited amount or number but also to more than the specifically recited amount or number. For example, the term "at least one immunoglobulin constant domain-like region" encompasses also at least two, at least three, at least four, at least five immunoglobulin constant domain-like regions and so on. Furthermore, this term also encompasses exactly one, exactly two, exactly three, exactly four, exactly five immunoglobulin constant domain-like regions and so on.

In accordance with the present invention, the starting protein is modified by the claimed method, thereby resulting in the "modified protein". The modification can be one or more of the specifically recited modifications.

The amino acid to be replaced in (a-i) is preferably an uncharged amino acid. Alternatively, the amino acid to be replaced in (a-i) can be a charged amino acid, in which case the charged amino acid is replaced by a different, charged amino acid. The term "charged amino acid", in accordance with the present invention, relates to an amino acid selected from the group consisting of Arg, Lys, His, Glu and Asp. Thus, uncharged amino acids are defined herein as any other amino acids apart from Arg, Lys, His, Glu and Asp, such as e.g. Ala, Asn, Cys, Gin, Gly, Ile, Leu, Met, Phe, Pro, Ser, Thr, Trp, Tyr and Val.

By replacing an uncharged amino acid with a charged amino acid in accordance with option (a-i), an additional interaction partner is introduced into the protein, thereby enabling the formation of an additional salt bridge. When replacing a charged amino acid with a different, charged amino acid, it is preferred that
(i) the replacement results in the generation of two opposing charges (i.e. negative and positive) in cases where to identical charges were previously present, wherein one charged amino acid is in the loop separating the C strand from the D strand and the second, differently charged amino acid is in the helix connecting the E strand with the F strand, or
(ii) the replacement results in an improved sterical environment, e.g. by replacing a small charged amino acid with a larger one or *vice versa,* thereby providing more favourable conditions for the formation of an additional salt bridge.

In option (i), when e.g. two anionic groups are present, for example in form of aspartic acid and/or glutamic acid, one of these amino acids may be replaced by an amino acid selected from the group consisting of lysine, arginine and histidine. The same applies when two positively charged amino acids are present, such as two amino acids selected from the group consisting of lysine, arginine and histidine, in which case one of these amino acids may be replaced by either aspartic acid or glutamic acid.

In option (ii), for example, the "short" amino acid aspartic acid may be replaced by the "longer" amino acid glutamic acid, where this is sterically advantageous. Analogously, histidine might be replaced by arginine; or lysine might be replaced by arginine.

In accordance with the present invention, the modifications characterised in (a-i) lead to the introduction of an additional salt bridge in said at least one immunoglobulin constant domain-like region. More specifically, by modifying the recited amino acid residues, a salt bridge is formed between said loop separating the C strand from the D strand and said helix connecting the E strand with the F strand. A salt bridge is a non-covalent interaction that results from the combination opposing electrostatic interactions. A salt bridge typically arises between charged amino acids, e.g. between the anionic carboxylate (RCOO-) of either aspartic acid or glutamic acid and the cationic ammonium (RNH3+) from lysine or the guanidinium (RNHC(NH₂)₂+) of arginine, but other residues with ionisable side chains such as histidine can also participate. The term "additional salt bridge", as used herein, refers to the fact that after the modification has been introduced, the modified protein comprises at least one more salt bridge between the recited sequences than the starting protein before carrying out the modification.

The amino acid to be replaced in (a-ii) is an amino acid not having a side chain that can form a hydrogen bond. Such amino acids not having a side chain that can form a hydrogen bond are defined herein as Ala, Arg, Asp, Cys, Glu, Gly, His, Ile, Leu, Lys, Met, Phe, Pro, Trp and Val. By replacing such an amino acid with an amino acid selected from the group of Gln, Asn, Tyr, Ser and Thr, an additional interaction partner is introduced into the protein that can participate in the formation of an additional hydrogen bond.

The amino acid to be replaced in (a-iii) is any amino acid other than a cysteine. By replacing such an amino acid with a cysteine, an additional interaction partner is introduced into the protein that can participate in the formation of a disulfide bond.

The modification in (a-i), (a-ii) and (a-iii) are introduced into the loop separating the C strand from the D strand and/or the helix connecting the E strand with the F strand.

The term "the loop separating the C strand from the D strand" (also referred to herein as the "C-D-connecting-loop"), as used herein, is based on the well-known terminology employed for the structural elements of immunoglobulin constant domains, as discussed herein above. Typically, immunoglobulin constant domains contain seven β strands, i.e. structural domains capable of forking β sheets, which are termed strand A to strand G. The individual β strands are separated by loops or helices, depending on the nature of the protein. Accordingly, the "loop separating the C strand from the D strand" is the loop that separates the third from the fourth β strand, based on the three-dimensional structure or similarity/prediction of the secondary elements from the primary amino acid sequence of an immunoglobulin constant domain or an immunoglobulin constant domain-like region.

In the shark IgNAR C4 sequence represented in SEQ ID NO:1 this loop corresponds to amino acids at positions 48 to 51, i.e. the sequence RKKG (SEQ ID NO: 68). Similarly, in the shark IgNAR C2 sequence represented in SEQ ID NO:2 this loop corresponds to amino acids at positions 45 to 48, i.e. the sequence RNEG (SEQ ID NO: 69). A corresponding loop is comprised in all immunoglobulin constant domains or immunoglobulin constant domain-like regions and can be identified by the skilled person without further ado. The following table 2 provides an overview of selected immunoglobulin constant domains, their SEQ ID number as well as the position of the loop separating the C strand from the D strand and its sequence.

**Table 2: The position of the loop separating the C strand from the D strand in selected immunoglobulin constant domains and its amino acid sequence.**

| **Ig constant domain (UniProtKB accession number)** | **SEQ ID NO:** | **Position of the loop in the indicated SEQ ID NO** | **Position of the loop in the indicated UniProtKB accession number** | **Sequence of the loop** |
|---|---|---|---|---|
| Nurse Shark C4 (Q90544) | 1 | 48-51 | 501-504 | RKKG (SEQ ID NO: 68) |
| Nurse Shark C2 (Q90544) | 2 | 45-48 | 288-291 | RNEG (SEQ ID NO: 69) |
| Human Ig Kappa LC (P01834) | 3 | 47-50 | 47-50 | QSGN (SEQ ID NO: 70) |
| Human Ig lambda-1 (P0CG04) | 4 | 49-52 | 49-52 | VKAG (SEQ ID NO: 71) |
| Human Ig lambda-2 (P0CG05) | 5 | 49-52 | 49-52 | VKAG (SEQ ID NO: 71) |
| Human Ig lambda-3 (P0CG06) | 6 | 49-52 | 49-52 | AKAG (SEQ ID NO: 72) |
| Mouse Ig kappa (P01837) | 7 | 47-50 | 47-51 | RQNG (SEQ ID NO: 73) |
| Mouse Ig lambda-1 (P01843) | 8 | 48-51 | 48-51 | VTQG (SEQ ID NO: 74) |
| Mouse Ig lambda-2 (P01844) | 9 | 48-51 | 48-51 | ITQG (SEQ ID NO: 75) |
| Mouse Ig lambda-3 (P01845) | 10 | 48-51 | 48-51 | ITQG (SEQ ID NO: 75) |
| Rabbit Ig kappa b4 (P01840) | 11 | 46-49 | 46-49 | QTTG (SEQ ID NO: 76) |
| Rabbit Ig kappa b5 (P01841) | 12 | 46-49 | 46-49 | LTTG (SEQ ID NO: 77) |
| Rabbit Ig kappa b5 var (P03984) | 13 | 46-49 | 46-49 | LTTG (SEQ ID NO: 77) |
| Rabbit Ig kappa b9 (P01838) | 14 | 48-51 | 48-51 | QQSG (SEQ ID NO: 78) |
| Rabbit Ig kappa b4 (P01839) | 15 | 48-51 | 48-51 | QQSG (SEQ ID NO: 78) |
| Rabbit Ig lambda (P01847) | 16 | 48-51 | 48-51 | VTQG (SEQ ID NO: 74) |
| Human Ig gamma 1 CH1 (P01857) | 17 | 48-51 | 48-51 | SGVH (SEQ ID NO: 79) |
| Human Ig gamma 1 CH2 (P01857) | 18 | 57-60 | 167-170 | VHNA (SEQ ID NO: 80) |
| Human Ig gamma 1 CH3 (P01857) | 19 | 47-50 | 270-273 | PENN (SEQ ID NO: 81) |
| Human Ig gamma 2 CH1 (P01859) | 20 | 48-51 | 48-51 | SGVH (SEQ ID NO: 79) |
| Human Ig gamma 2 CH2 (P01859) | 21 | 53-56 | 163-166 | VHNA (SEQ ID NO: 80) |
| Human Ig gamma 2 CH3 (P01859) | 22 | 47-50 | 266-269 | PENN (SEQ ID NO: 81) |
| Human Ig gamma 3 CH1 (P01860) | 23 | 48-51 | 48-51 | SGVH (SEQ ID NO: 79) |
| Human Ig gamma 3 CH2 (P01860) | 24 | 54-57 | 214-217 | VHNA (SEQ ID NO: 80) |
| Human Ig gamma 3 CH3 (P01860) | 25 | 47-50 | 317-320 | PENN (SEQ ID NO: 81) |
| Human Ig gamma 4 CH1 (P01861) | 26 | 48-51 | 48-51 | SGVH (SEQ ID NO: 79) |
| Human Ig gamma 4 CH2 (P01861) | 27 | 54-57 | 164-167 | VHNA (SEQ ID NO: 80) |
| Human Ig gamma 4 CH3 (P01861) | 28 | 47-50 | 267-270 | PENN (SEQ ID NO: 81) |
| Mouse Ig gamma 1 CH1 (P01868) | 29 | 48-51 | 48-51 | SGVH (SEQ ID NO: 79) |
| Mouse Ig gamma 1 CH2 (P01868) | 30 | 51-54 | 161-164 | VHTA (SEQ ID NO: 82) |
| Mouse Ig gamma 1 CH3 (P01868) | 31 | 47-50 | 264-267 | PAEN (SEQ ID NO: 83) |
| Mouse Ig gamma 2A CH1 (P01863) | 32 | 48-51 | 48-51 | SGVH (SEQ ID NO: 79) |
| Mouse Ig gamma 2A CH2 (P01863) | 33 | 47-50 | 167-170 | VHTA (SEQ ID NO: 82) |
| Mouse Ig gamma 2A CH3 (P01863) | 34 | 42-45 | 270-273 | TELN (SEQ ID NO: 84) |
| Mouse Ig gamma 2B CH1 (P01867) | 35 | 48-51 | 48-51 | SSVH (SEQ ID NO: 85) |
| Mouse Ig gamma 2B CH2 (P01867) | 36 | 47-50 | 172-175 | VHTA (SEQ ID NO: 82) |
| Mouse Ig gamma 2B CH3 (P01867) | 37 | 42-45 | 275-278 | TEEN (SEQ ID NO: 86) |
| Mouse Ig gamma 3 CH1 (P03987) | 38 | 47-50 | 47-50 | SGVR (SEQ ID NO: 87) |
| Mouse Ig gamma 3 CH2 (P03987) | 39 | 53-56 | 166-169 | VHTA (SEQ ID NO: 82) |
| Mouse Ig gamma 3 CH3 (P03987) | 40 | 46-49 | 269-272 | LEQD (SEQ ID NO: 88) |
| Rabbit Ig gamma CH1 (P01870) | 41 | 48-51 | 48-51 | NGVR (SEQ ID NO: 89) |
| Rabbit Ig gamma CH2 (P01870) | 42 | 47-50 | 160-163 | VRTA (SEQ ID NO: 90) |
| Rabbit Ig gamma CH3 (P01870) | 43 | 42-45 | 263-266 | AEDN (SEQ ID NO: 91) |
| Human Ig epsilon CH1 (P01854) | 44 | 50-53 | 50-53 | GTTM (SEQ ID NO: 92) |
| Human Ig epsilon CH2 (P01854) | 45 | 45-48 | 156-159 | MDVD (SEQ ID NO: 92) |
| Human Ig epsilon CH3 (P01854) | 46 | 49-52 | 262-265 | PVNH (SEQ ID NO: 93) |
| Human Ig epsilon CH4 (P01854) | 47 | 43-46 | 366-369 | LPDA (SEQ ID NO: 94) |
| Human Ig delta CH1 (P01880) | 48 | 49-52 | 49-52 | QPQR (SEQ ID NO: 95) |
| Human Ig delta CH2 (P01880) | 49 | 36-39 | 210-213 | PTGG (SEQ ID NO: 96) |
| Human Ig delta CH3 (P01880) | 50 | 49-52 | 315-318 | VNTS (SEQ ID NO: 97) |
| Human Ig mu CH1 (P01871) | 51 | 49-52 | 49-52 | DISS (SEQ ID NO: 98) |
| Human Ig mu CH2 (P01871) | 52 | 50-53 | 155-158 | VGSG (SEQ ID NO: 99) |
| Human Ig mu CH3 (P01871) | 53 | 49-52 | 266-269 | AVKT (SEQ ID NO: 100) |
| Human Ig mu CH4 (P01871) | 54 | 49-52 | 372-375 | LSPE (SEQ ID NO: 101) |
| Human Ig alpha CH1 (P01876) | 55 | 48-51 | 48-51 | VTAR (SEQ ID NO: 102) |
| Human Ig alpha CH2 (P01876) | 56 | 45-48 | 169-172 | SAVQ (SEQ ID NO: 103) |
| Human Ig alpha CH3 (P01876) | 57 | 44-47 | 271-274 | LPRE (SEQ ID NO: 104) |
| Human Ig alpha 2 CH1 (P01877) | 58 | 48-51 | 48-51 | VTAR (SEQ ID NO: 102) |
| Human Ig alpha 2 CH2 (P01877) | 59 | 45-48 | 156-159 | SAVQ (SEQ ID NO: 103) |
| Human Ig alpha 2 CH3 (P01877) | 60 | 44-47 | 258-261 | LPRE (SEQ ID NO: 104) |
| Rat Ig Kappa chain C (P01836) | 61 | 47-50 | 47-50 | QRDG (SEQ ID NO: 105) |
| Chicken Ig lambda C (P20763) | 62 | 49-52 | 49-52 | RSGE (SEQ ID NO: 106) |
| Horse Ig gamma (Q95M34) | 63 | 46-49 | 172-175 | VRTA (SEQ ID NO: 90) |
| Giant Panda CH1 (D2I829) | 64 | 53-56 | 53-56 | QSSG (SEQ ID NO: 107) |
| Giant Panda CH2 (D2I829) | 65 | 53-56 | 158-161 | VRTA (SEQ ID NO: 90) |
| Giant Panda CH3 (D2I829) | 66 | 47-50 | 262-265 | VPET (SEQ ID NO: 108) |
| African clawed frog (Q6INK3) | 67 | 45-48 | 405-408 | NLEF (SEQ ID NO: 109) |

Accordingly, also the term "the helix connecting the E strand with the F strand" (also referred to herein as the "E-F-connecting helix"), as used herein, is based on said well-known terminology employed for the structural elements of immunoglobulin constant domains. Thus, this helix corresponds to the α helix that separates the fifth from the sixth β strand, based on the primary amino acid sequence of an immunoglobulin constant domain or an immunoglobulin constant domain-like region.

In the shark IgNAR C4 sequence represented in SEQ ID NO:1 this helix corresponds to amino acids at position 74 to 78, i.e. the sequence ASEWD (SEQ ID NO:110). Similarly, in the shark IgNAR C2 sequence represented in SEQ ID NO:2 this helix corresponds to amino acids at position 71 to 75, i.e. the sequence VEEWQ (SEQ ID NO:111). The same helix is comprised in all immunoglobulin constant domains or immunoglobulin constant domain-like regions and can be identified by the skilled without further ado. The following table 3 provides an overview of selected immunoglobulin constant domains, their SEQ ID number as well as the position of the helix connecting the E strand with the F strand and its sequence.

**Table 3: The position of the helix connecting the E strand with the F strand in selected immunoglobulin constant domains and its amino acid sequence.**

| **Ig constant domain (UniProtKB accession number)** | **SEQ ID NO:** | **Position of the helix in the indicated SEQ ID NO** | **Position of the helix in the indicated UniProtKB accession number** | **Sequence of the helix** |
|---|---|---|---|---|
| Nurse Shark C4 (Q90544) | 1 | 74-78 | 527-531 | ASEWD (SEQ ID NO: 110) |
| Nurse Shark C2 (Q90544) | 2 | 71-75 | 314-318 | VEEWQ (SEQ ID NO: 111) |
| Human Ig Kappa LC (P01834) | 3 | 75-79 | 75-79 | KADYE (SEQ ID NO: 112) |
| Human Ig lambda-1 (P0CG04) | 4 | 76-80 | 76-80 | PEQWK (SEQ ID NO: 113) |
| Human Ig lambda-2 (P0CG05) | 5 | 76-80 | 76-80 | PEQWK (SEQ ID NO: 113) |
| Human Ig lambda-3 (P0CG06) | 6 | 76-80 | 76-80 | PEQWK (SEQ ID NO: 113) |
| Mouse Ig kappa (P01837) | 7 | 75-79 | 75-79 | KDEYE (SEQ ID NO: 114) |
| Mouse Ig lambda-1 (P01843) | 8 | 75-79 | 75-79 | ARAWE (SEQ ID NO: 115) |
| Mouse Ig lambda-2 (P01844) | 9 | 74-78 | 74-78 | SDQWR (SEQ ID NO: 116) |
| Mouse Ig lambda-3 (P01845) | 10 | 74-78 | 74-78 | SDQWR (SEQ ID NO: 116) |
| Rabbit Ig kappa b4 (P01840) | 11 | 74-78 | 74-78 | STQYN (SEQ ID NO: 117) |
| Rabbit Ig kappa b5 (P01841) | 12 | 74-78 | 74-78 | KSNYN (SEQ ID NO: 118) |
| Rabbit Ig kappa b5 var (P03984) | 13 | 74-78 | 74-78 | SDEYN (SEQ ID NO: 119) |
| Rabbit Ig kappa b9 (P01838) | 14 | 76-80 | 76-80 | KAQYN (SEQ ID NO: 120) |
| Rabbit Ig kappa b4 (P01839) | 15 | 76-80 | 76-80 | SAQYN (SEQ ID NO: 121) |
| Rabbit Ig lambda (P01847) | 16 | 75-79 | 75-79 | ANQWK (SEQ ID NO: 122) |
| Human Ig gamma 1 CH1 (P01857) | 17 | 73-77 | 73-77 | SSSLG (SEQ ID NO: 123) |
| Human Ig gamma 1 CH2 (P01857) | 18 | 83-87 | 193-196 | HQDWL (SEQ ID NO: 124) |
| Human Ig gamma 1 CH3 (P01857) | 19 | 74-78 | 297-301 | KSRWQ (SEQ ID NO: 125) |
| Human Ig gamma 2 CH1 (P01859) | 20 | 73-77 | 73-77 | SSNFG (SEQ ID NO: 126) |
| Human Ig gamma 2 CH2 (P01859) | 21 | 79-83 | 189-193 | HQDWL (SEQ ID NO: 124) |
| Human Ig gamma 2 CH3 (P01859) | 22 | 74-78 | 293-297 | KSRWQ (SEQ ID NO: 125) |
| Human Ig gamma 3 CH1 (P01860) | 23 | 73-77 | 73-77 | SSSLG (SEQ ID NO: 123) |
| Human Ig gamma 3 CH2 (P01860) | 24 | 80-84 | 240-244 | HQDWL (SEQ ID NO: 124) |
| Human Ig gamma 3 CH3 (P01860) | 25 | 74-78 | 344-348 | KSRWQ (SEQ ID NO: 125) |
| Human Ig gamma 4 CH1 (P01861) | 26 | 73-77 | 73-77 | SSSLG (SEQ ID NO: 123) |
| Human Ig gamma 4 CH2 (P01861) | 27 | 80-84 | 190-194 | HQDWL (SEQ ID NO: 124) |
| Human Ig gamma 4 CH3 (P01861) | 28 | 74-78 | 294-298 | KSRWQ (SEQ ID NO: 125) |
| Mouse Ig gamma 1 CH1 (P01868) | 29 | 72-76 | 72-76 | SSPRP (SEQ ID NO: 127) |
| Mouse Ig gamma 1 CH2 (P01868) | 30 | 77-81 | 187-191 | HQDWL (SEQ ID NO: 124) |
| Mouse Ig gamma 1 CH3 (P01868) | 31 | 74-78 | 291-295 | KSNWE (SEQ ID NO: 128) |
| Mouse Ig gamma 2A CH1 (P01863) | 32 | 72-76 | 72-76 | SSTWP (SEQ ID NO: 129) |
| Mouse Ig gamma 2A CH2 (P01863) | 33 | 73-77 | 193-197 | HQDWM (SEQ ID NO: 130) |
| Mouse Ig gamma 2A CH3 (P01863) | 34 | 69-73 | 297-301 | KKNWV (SEQ ID NO: 131) |
| Mouse Ig gamma 2B CH1 (P01867) | 35 | 72-76 | 72-76 | SSTWP (SEQ ID NO: 129) |
| Mouse Ig gamma 2B CH2 (P01867) | 36 | 73-77 | 198-202 | HQDWM (SEQ ID NO: 130) |
| Mouse Ig gamma 2B CH3 (P01867) | 37 | 69-73 | 302-306 | TSKWE (SEQ ID NO: 132) |
| Mouse Ig gamma 3 CH1 (P03987) | 38 | 71-75 | 71-75 | SSTWP (SEQ ID NO: 129) |
| Mouse Ig gamma 3 CH2 (P03987) | 39 | 79-83 | 192-196 | HQDWM (SEQ ID NO: 130) |
| Mouse Ig gamma 3 CH3 (P03987) | 40 | 73-77 | 296-300 | TDSWL (SEQ ID NO: 132) |
| Rabbit Ig gamma CH1 (P01870) | 41 | 72-76 | 72-76 | TSSSQ (SEQ ID NO: 133) |
| Rabbit Ig gamma CH2 (P01870) | 42 | 73-77 | 186-190 | HQDWL (SEQ ID NO: 124) |
| Rabbit Ig gamma CH3 (P01870) | 43 | 69-73 | 290-294 | TSEWQ (SEQ ID NO: 134) |
| Human Ig epsilon CH1 (P01854) | 44 | 75-79 | 75-79 | SGAWA (SEQ ID NO: 135) |
| Human Ig epsilon CH2 (P01854) | 45 | 71-75 | 182-186 | QKHWL (SEQ ID NO: 136) |
| Human Ig epsilon CH3 (P01854) | 46 | 75-79 | 288-292 | TRDWI (SEQ ID NO: 137) |
| Human Ig epsilon CH4 (P01854) | 47 | 71-75 | 394-398 | RAEWE (SEQ ID NO: 138) |
| Human Ig delta CH1 (P01880) | 48 | 74-78 | 74-78 | LQQWR (SEQ ID NO: 139) |
| Human Ig delta CH2 (P01880) | 49 | 63-67 | 237-241 | RSLWN (SEQ ID NO: 140) |
| Human Ig delta CH3 (P01880) | 50 | 78-82 | 344-348 | APPSP (SEQ ID NO: 141) |
| Human Ig mu CH1 (P01871) | 51 | 75-79 | 75-79 | SKDVM (SEQ ID NO: 142) |
| Human Ig mu CH2 (P01871) | 52 | 81-85 | 186-190 | ESDWL (SEQ ID NO: 143) |
| Human Ig mu CH3 (P01871) | 53 | 75-79 | 292-296 | EDDWN (SEQ ID NO: 144) |
| Human Ig mu CH4 (P01871) | 54 | 79-83 | 402-406 | EEEWN (SEQ ID NO: 145) |
| Human Ig alpha CH1 (P01876) | 55 | 75-79 | 75-79 | TQCLA (SEQ ID NO: 146) |
| Human Ig alpha CH2 (P01876) | 56 | 69-73 | 193-197 | AEPWN (SEQ ID NO: 147) |
| Human Ig alpha CH3 (P01876) | 57 | 75-79 | 302-306 | AEDWK (SEQ ID NO: 148) |
| Human Ig alpha 2 CH1 (P01877) | 58 | 74-78 | 74-78 | ATQCP (SEQ ID NO: 149) |
| Human Ig alpha 2 CH2 (P01877) | 59 | 69-73 | 180-184 | AQPWN (SEQ ID NO: 150) |
| Human Ig alpha 2 CH3 (P01877) | 60 | 75-79 | 289-293 | AEDWK (SEQ ID NO: 148) |
| Rat Ig Kappa chain C (P01836) | 61 | 75-79 | 75-79 | KVEYE (SEQ ID NO: 151) |
| Chicken Ig lambda C (P20763) | 62 | 74-78 | 74-78 | ASDWS (SEQ ID NO: 152) |
| Horse Ig gamma (Q95M34) | 63 | 72-76 | 198-202 | HQDWL (SEQ ID NO: 124) |
| Giant Panda CH1 (D21829) | 64 | 68-72 | 68-72 | SSRWP (SEQ ID NO: 153) |
| Giant Panda CH2 (D2I829) | 65 | 79-83 | 184-188 | HQDWL (SEQ ID NO: 124) |
| Giant Panda CH3 (D2I829) | 66 | 75-79 | 290-294 | KNRWH (SEQ ID NO: 154) |
| African clawed frog (Q6INK3) | 67 | 71-75 | 431-435 | AEDWE (SEQ ID NO: 155) |

In accordance with option (a) of the method of the present invention, either an amino acid in the loop separating the C strand from the D strand is replaced, or an amino acid in the helix connecting the E strand with the F strand is replaced, or both replacements are carried out.

Whether amino acids in only one or both positions have to be replaced can be determined by the skilled person based on the amino acid sequence of the starting protein.

For example, in those cases where either the C-D-connecting-loop or the E-F-connecting-helix already contains a charged amino acid not forming a salt bridge with another charged amino acid, the replacement of only one amino acid in the second sequence will be sufficient to result in the formation of an additional salt bridge, either by introducing a charged amino acid or by replacing an existing charged amino acid with a charged amino acid having the opposite charge or that is sterically more suitable. On the other hand, where neither the C-D-connecting-loop nor the E-F-connecting-helix contains a charged amino acid, the replacement of an amino acid with a charged amino acid has to be carried out in both.

As another example, in those cases where one of the C-D-connecting-loop or the E-F-connecting-helix already contains an amino acid having a side chain capable of forming a hydrogen bond but wherein said amino acid does not form a hydrogen bond, the replacement of only one amino acid in the second sequence for an amino acid having a side chain capable of forming a hydrogen bond will be sufficient to result in the formation of an additional hydrogen bond. On the other hand, where neither of the two sequences, i.e. neither the C-D-connecting-loop nor the E-F-connecting-helix, contains a free (i.e. not involved in the formation of an existing hydrogen bond) amino acid having a side chain capable of forming a hydrogen bond, the replacement for an amino acid having a side chain capable of forming a hydrogen bond has to be carried out in both sequences, i.e. in the C-D-connecting-loop and in the E-F-connecting-helix. The same considerations apply *mutatis mutandis* to the replacement of amino acids with cysteine(s).

As used throughout the present application, the term "free charged amino acid" relates to a charged amino acid that, although it has a side chain that is available for the formation of a salt bridge, nonetheless is not involved in the formation of an existing salt bridge, i.e. this amino acid is free to form a salt bridge.

As used throughout the present application, the term "free amino acid having a side chain capable of forming a hydrogen bond" relates to an amino acid that, although it has a side chain that is available for forming a hydrogen bond, nonetheless is not involved in the formation of an existing hydrogen bond, i.e. this amino acid is free to form a hydrogen bond.

As used throughout the present application, the term "free Cys" or "free cystein" relates to a cysteine that is not involved in the formation of an existing disulfide bridge, i.e. this cysteine is free to form a disulfide bridge.

Accordingly, the modifications in accordance with option (a-i) of the method of the present invention comprise at least the following options:
(1) In the loop separating the C strand from the D strand, no free charged amino acid is present. In this case, said loop is modified by replacing an amino acid, preferably an uncharged amino acid, with a charged amino acid.
(2) In the loop separating the C strand from the D strand, a free charged amino acid is present. In this case, several options are available:
   (2-1) Said loop does not need to be modified and the charged amino acid already present in the loop may be used as a basis for introducing an additional salt bridge by replacing an amino acid in the helix connecting the E strand with the F strand.
   (2-2) Said amino acid is replaced by a differently charged amino acid having the opposite charge. This option applies in particular in those cases where in the helix connecting the E strand with the F strand a free amino acid of the same charge is present.
   (2-3) Said amino acid is replaced by a charged amino acid having a different size, i.e. a larger or smaller amino acid of the same charge. This option applies where such an amendment is advantageous under sterical view points and is chosen based on the nature of the free charged amino acid in the helix connecting the E strand with the F strand, which may be naturally present or introduced.
(3) In the helix connecting the E strand with the F strand, no free charged amino acid is present. In this case, said helix is modified by replacing an amino acid, preferably an uncharged amino acid, with a charged amino acid.
(4) In the helix connecting the E strand with the F strand, a free charged amino acid is present. In this case, several options are again available:
   (4-1) Said helix does not need to be modified and the charged amino acid already present in the helix may be used as a basis for introducing an additional salt bridge by replacing an amino acid in the loop separating the C strand from the D strand.
   (4-2) Said amino acid is replaced by a differently charged amino acid having the opposite charge. This option applies in particular in those cases where in the loop separating the C strand from the D strand a free amino acid of the same charge is present.
   (4-3) Said amino acid is replaced by a charged amino acid having a different size, i.e. a larger or smaller amino acid of the same charge. This option applies where such an amendment is advantageous under sterical view points and is chosen based on the nature of the free charged amino acid in the loop separating the C strand from the D strand, which may be naturally present or introduced.

As detailed above, it is understood that the nature of the charged amino acid(s) to be introduced is to be selected such that the two amino acids that are to form the salt bridge are charged oppositely, i.e. the amino acid pair that is to form the salt bridge has to comprise one amino acid with an anionic nature and one amino acid with a cationic nature. Thus, where the amino acid in the loop separating the C strand from the D strand that is to participate in the formation of the additional salt bridge (i.e. being either already present or being introduced) has an anionic carboxylate (RCOO-), then the amino acid in the helix connecting the E strand with the F strand that is to participate in the formation of the additional salt bridge (i.e. being either already present or being introduced) has an anionic nature, such as e.g. the cationic ammonium (RNH3+) from lysine or the guanidinium (RNHC(NH₂)₂+) of arginine.

Furthermore, depending on the sterical requirements in the molecule to be modified, an existing charged amino acid can be replaced by a more suitable charged amino acid, such as a smaller or larger amino acid.

Accordingly, option (a-i) of the method of the present invention includes the replacement of at least one amino acid such that an amino acid-pair is formed that consists of (1) an anionic amino acid in one position as referred to in (a-i), and a cationic amino acid in the second position. It is particularly preferred that this amino acid-pair is selected from the group consisting of Glu and Arg; Glu and Lys; Glu and His; Asp and Arg; Asp and Lys; and Asp and His, in order to allow for the formation of a salt bridge. Most preferably, the amino acid-pair is Glu and Arg.

Similar considerations apply to option (a-ii) of the method of the invention, which comprises at least the following options:
(1) In the loop separating the C strand from the D strand, no free amino acid having a side chain capable of forming a hydrogen bond is present. In this case, said loop is modified by replacing one of the amino acid not having a side chain capable of forming a hydrogen bond with a Gin, Asn, Tyr, Ser or Thr.
(2) In the loop separating the C strand from the D strand, a free amino acid having a side chain capable of forming a hydrogen bond is present. In this case, several options are available:
   (2-1) Said loop does not need to be modified and the free amino acid already present in the loop may be used as a basis for introducing an additional hydrogen bond by replacing an amino acid in the helix connecting the E strand with the F strand.
   (2-2) Said amino acid is replaced by an amino acid having a different size, i.e. a larger or smaller amino acid. This option applies where such an amendment is advantageous under sterical view points and is chosen based on the nature of the free amino acid in the helix connecting the E strand with the F strand, which may be naturally present or introduced.
(3) In the helix connecting the E strand with the F strand, no free amino acid having a side chain capable of forming a hydrogen bond is present. In this case, said helix is modified by replacing one of the amino acid not having a side chain capable of forming a hydrogen bond with a Gin, Asn, Tyr, Ser or Thr.
(4) In the helix connecting the E strand with the F strand, a free amino acid having a side chain capable of forming a hydrogen bond is present. In this case, several options are again available:
   (4-1) Said helix does not need to be modified and the free amino acid already present in the helix may be used as a basis for introducing an additional hydrogen bond by replacing an amino acid in the loop separating the C strand from the D strand.
   (4-2) Said amino acid is replaced by an amino acid having a different size, i.e. a larger or smaller amino acid. This option applies where such an amendment is advantageous under sterical view points and is chosen based on the nature of the free amino acid in the loop separating the C strand from the D strand, which may be naturally present or introduced.

Accordingly, option (a-ii) of the method of the present invention includes the replacement of at least one amino acid such that a hydrogen bond is formed between an amino acid pair that consists of (1) an amino acid having a side chain capable of forming a hydrogen bond in the C-D-connecting-loop, and (2) an amino acid having a side chain capable of forming a hydrogen bond in the E-F-connecting-helix. It is particularly preferred that this amino acid pair is selected from the amino acids of the group consisting of Gln, Asn and Tyr. More preferably, the amino acid pair is selected from the group consisting of the amino acid pairs Gin-Gin or Asn-Asn. Most preferably, the amino acid pair is Gln-Gln.

Similar considerations further apply to option (a-iii) of the method of the invention, which comprises at least the following options:
(1) In the loop separating the C strand from the D strand, no free Cys is present. In this case, said loop is modified by replacing an amino acid with Cys.
(2) In the loop separating the C strand from the D strand, a free Cys is present. In this case, said loop does not need to be modified and the free Cys already present in the loop may be used as a basis for introducing an additional disulfide bond by replacing an amino acid in the helix connecting the E strand with the F strand.
(3) In the helix connecting the E strand with the F strand, no free Cys is present. In this case, said helix is modified by replacing an amino acid with Cys.
(4) In the helix connecting the E strand with the F strand, a free Cys is present. In this case, said helix does not need to be modified and the free Cys already present in the helix may be used as a basis for introducing an additional disulfide bond by replacing an amino acid in the loop separating the C strand from the D strand.

In accordance with option (b), at least one non-hydrophobic amino acid is replaced by a hydrophobic amino acid selected from the group consisting of Val, Ile, Leu, Met, Phe, Try, and Pro. In a preferred embodiment of the method of the invention, the hydrophobic amino acid is selected from the group consisting of Val, Ile, Leu, Met and Phe. The term "non-hydrophobic amino acid", in accordance with the present invention, also encompasses weakly hydrophobic amino acids. Thus, the non-hydrophobic amino acids in accordance with the present invention can be, e.g. Ala, Arg, Asn, Asp, Cys, Glu, Gln, Gly, His, Lys, Ser, Tyr and Thr.

In accordance with option (b) of the method of the invention, the existing hydrophobic core of a protein is extended by replacing one or more non-hydrophobic or weakly-hydrophobic amino acid(s) with one or more hydrophobic amino acid(s) in a position within the protein that is suitable to participate in the formation of the hydrophobic core. Preferably, one non-hydrophobic or weakly-hydrophobic amino acid is replaced with one hydrophobic amino acid, as defined herein. Even more preferably, one non-hydrophobic amino acid is replaced with one hydrophobic amino acid.

Many proteins have a hydrophobic core, i.e. a core inside the three-dimensional, folded structure of the protein in which the side chains of hydrophobic amino acids, such as valine, leucine, isoleucine, phenylalanine, tryptophan, methionine and proline, are clustered together and are thus shielded from a hydrophilic environment, such as water. The side chains of non-hydrophobic amino acids are mainly situated on the solvent-exposed surface where they interact with surrounding water molecules. The hydrophobic core not only stabilises the folded state of these proteins, but the minimization of the number of hydrophobic side chains exposed to water is also the main driving force of the folding process.

Whether a particular position is suitable to participate in the formation of the hydrophobic core depends on the three-dimensional structure of the protein and can be determined by the skilled person without further ado. Methods of determining such suitable positions include, without being limiting, the analysis of three-dimensional structures of the protein, secondary/tertiary structure prediction and homology modelling as well as sequence alignments. For example, based on the IgNAR C4 sequence, where an extended hydrophobic core has been identified (see below), the sequences of IgNAR C4 and another protein in accordance with the method of the invention, which is to be stabilized can be aligned and compared by the skilled person without further ado using available sequence alignment software (e.g. ClustaIW2, (Larkin et al., 2007)), to identify the position for a potential further hydrophobic amino acid that could participate in the formation of the hydrophobic core. Similarly, if the structure of the protein in accordance with the method of invention is known or will be determined (by e.g. nuclear magnetic resonance, X-ray crystallography, or computational structure prediction, e.g. homology modelling), the structures of both proteins can be aligned by the skilled person without further ado (using e.g. SALIGN, (Braberg et al., 2012)), and the position for a potential further hydrophobic amino acid can be located and replaced by the skilled person without further ado.

Based on the example of the stable nurse shark IgNAR C4 as represented in SEQ ID NO:1, the hydrophobic core is formed by the amino acids in positions 5, 7, 9, 25, 29, 37, 39, 41, 43, 52, 66, 70, 72, 77, 83, 87, 96 and 100, while based on the example of nurse shark IgNAR C2 as represented in SEQ ID NO:2, the hydrophobic core is formed by the amino acids in positions 2, 4, 6, 22, 26, 34, 36, 38, 40, 49, 67, 74, 80, and 93.

As compared to these stable protein sequences, for example the human C_{L} domain represented by SEQ ID NO: 3 contains a hydrophobic core formed by the amino acids in position 5, 7, 9, 24, 28, 38, 40, 42, 67, 71, 73, 78, 84, 88, 97 and 101. By replacing the serine in position 51 of SEQ ID NO:3, for example with valine, the hydrophobic core of this molecule can be extended.

Similarly, the human CH2 domain represented by SEQ ID NO: 18 contains a hydrophobic core formed by the amino acids in position 11, 13, 15, 32, 36, 46, 48, 50, 52, 75, 79, 81, 86, 96, 105 and 109. By replacing the alanine in position 60 of SEQ ID NO:18, for example with valine, the hydrophobic core of this molecule can be extended. Likewise, the same position in other proteins comprising immunoglobulin constant domain-like regions, such as e.g. the remaining sequences listed in table 4, can be modified by exchanging a non-hydrophobic amino acid for a hydrophobic amino acid to extend the hydrophobic core and stabilize the protein. As described elsewhere herein, suitable positions can be determined by the skilled person based on structural alignments.

In accordance with the method of the invention, the starting protein can be modified in accordance with either (a), (b) or both. As detailed herein above, there are several possible combinations for (a), which can either be carried out on their own, i.e. without the modification according to (b) or which can be carried out together with the modification according to (b). Preferably, at least one additional salt bridge or hydrogen bond or disulfide bond is introduced and the hydrophobic core is extended, i.e. the combination of both (a) and (b) is preferred.

In accordance with the present invention the term "nucleic acid molecule", also referred to as "polynucleotide" or "nucleic acid sequence" herein, defines a linear molecular chain consisting of more than 30 nucleotides. "Nucleic acid molecules", in accordance with the present invention, include DNA, such as for example cDNA or genomic DNA, and RNA, for example mRNA. Further included are nucleic acid mimicking molecules known in the art such as for example synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see (Braasch and Corey, 2001)). LNA is an RNA derivative in which the ribose ring is constrained by a methylene linkage between the 2'-oxygen and the 4'-carbon. They may contain additional non-natural or derivative nucleotide bases, as is well known in the art.

It will be appreciated that the proteins (e.g. the starting protein or the modified protein) of the present invention may be encoded by a single nucleic acid molecule or by a plurality of nucleic acid molecules encoding parts of the proteins, such as e.g. different chains of an antibody. Upon expression of these nucleic acid molecules, they form the modified protein of the invention via non-covalent bonds such as for example hydrogen bonds, ionic bonds, van der Waals forces or hydrophobic interacts or via covalent bonds such as for example disulfide bonds.

Where the modified protein of the invention is encoded by a plurality of nucleic acid molecules, the modifications as described above can be present in all of the nucleic acid molecules or may only be present in some of the nucleic acid molecules, such as e.g. only one out of two nucleic acid molecules or in two out of three nucleic acid molecule etc.. Similarly, where the modified protein of the invention is encoded by a single nucleic acid molecule encoding for a protein with more than one immunoglobulin constant domain-like regions, the modifications as described above can be present in all of the immunoglobulin constant domain-like regions or may only be present in some of the immunoglobulin constant domain-like regions, such as e.g. only one out of two immunoglobulin constant domain-like regions or in two out of three immunoglobulin constant domain-like regions etc..

As a result, the resulting modified protein may comprise several immunoglobulin constant domain-like regions, wherein either one, or several or even all of said immunoglobulin constant domain-like regions are modified as detailed above.

In a further step of the method of the present invention, the nucleic acid molecule is expressed in order to produce the stabilised protein.

A large number of suitable methods exist in the art to produce proteins from nucleic acid molecules. For example, proteins may be produced in appropriate hosts. If the host is a unicellular organism such as a prokaryote, a mammalian or insect cell, the person skilled in the art can revert to a variety of culture conditions. Conveniently, the produced protein is harvested from the culture medium, lysates of the cultured organisms or from isolated (biological) membranes by established techniques. In the case of a multicellular organism, the host may be a cell, which is part of or derived from a part of the organism, for example said host cell may be the harvestable part of a plant. A preferred method involves the recombinant production of proteins in hosts as indicated above. For example, the nucleic acid molecules encoding the modified proteins according to the invention can be synthesized by PCR and inserted into an expression vector. Subsequently, a suitable host may be transformed with the expression vector. Thereafter, the host is cultured to produce the desired proteins(s), which is/are isolated and, optionally, purified.

An alternative method for producing the protein in accordance with the method of the invention is *in vitro* translation of mRNA. Suitable cell-free expression systems include rabbit reticulocyte lysate, wheat germ extract, canine pancreatic microsomal membranes, *E*. *coli* S30 extract, and coupled transcription/translation systems such as e.g. the TNT-system (Promega). These systems allow the expression of recombinant proteins upon the addition of cloning vectors, DNA fragments, or RNA sequences containing coding regions and appropriate promoter elements.

Furthermore, the proteins may be produced semi-synthetically, for example by a combination of recombinant and synthetic production. Synthetic production can be performed by any method known in the art, e.g. by direct peptide synthesis using solid-phase techniques ((Merrifield, 1969; Stewart, 1969)). Synthetic peptide synthesis may be performed using manual techniques or by automation. Automated synthesis may be achieved, for example, using the Applied Biosystems 431A Peptide Synthesizer (Perkin Elmer, Foster City CA) in accordance with the instructions provided by the manufacturer. Accordingly, various fragments may be chemically synthesized separately and combined using chemical methods to produce the full-length molecule. As indicated above, chemical synthesis, such as the solid phase procedure can be used.

Protein isolation and purification can be achieved by any one of several known techniques; for example and without limitation ion exchange chromatography, gel filtration chromatography and affinity chromatography, high pressure liquid chromatography (HPLC), reversed phase HPLC, and preparative disc gel electrophoresis.

In accordance with this method of the present invention, the thus produced modified protein has an increased stability as compared to the unmodified protein, also referred to herein as the "starting protein". This includes, for example, an increased stability when exposed to chemical or thermal stress as well as protection against protease degradation. As a consequence, the modified proteins of the present invention are more stable as compared to the unmodified protein in terms of e.g. having a reduced formation of aggregates; an increased shelf-life; or reduced protein unfolding. In addition, the modified proteins have an improved folding efficiency, i.e. their folding into their correct three-dimensional structure after unfolding, e.g. when present in inclusion bodies, is improved as compared to the unmodified protein. Due to this increased stability, higher yields of the modified proteins can be obtained, i.e. a higher number of modified proteins are secreted from cells or the amount of correctly refolded protein obtained from inclusion bodies is higher for the modified proteins in accordance with the invention as compared to the unmodified protein.

In accordance with the present invention, structural elements that contribute to the high stability of the so-called "immunoglobulin new antigen receptor" (IgNAR) domains of sharks were identified and transferred to other proteins to improve their stability and secretion.

Cartilaginous fish (Chondrichthyes) such as sharks, skates and rays (Cooper and Alder, 2006; Flajnik and Kasahara, 2010) are the phylogenetically oldest living organisms identified to possess all components of a vertebrate adaptive immune system. They shared the last common ancestor with other jawed vertebrates roughly 500 million years ago (Blair and Hedges, 2005; Flajnik and Kasahara, 2010). Accordingly, shark antibodies can provide unique insights into the molecular evolution of the immune system. Furthermore, due to their adaptation to harsh environments - i.e. the high osmolarity of shark blood is partially upheld by the protein denaturant urea (Dooley and Flajnik, 2006; England and Haran, 2011) - shark antibodies are particularly stable (Henderson et al., 2007; Saerens et al., 2008).

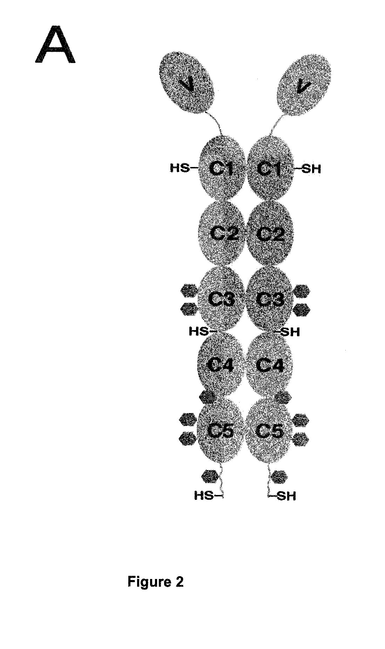

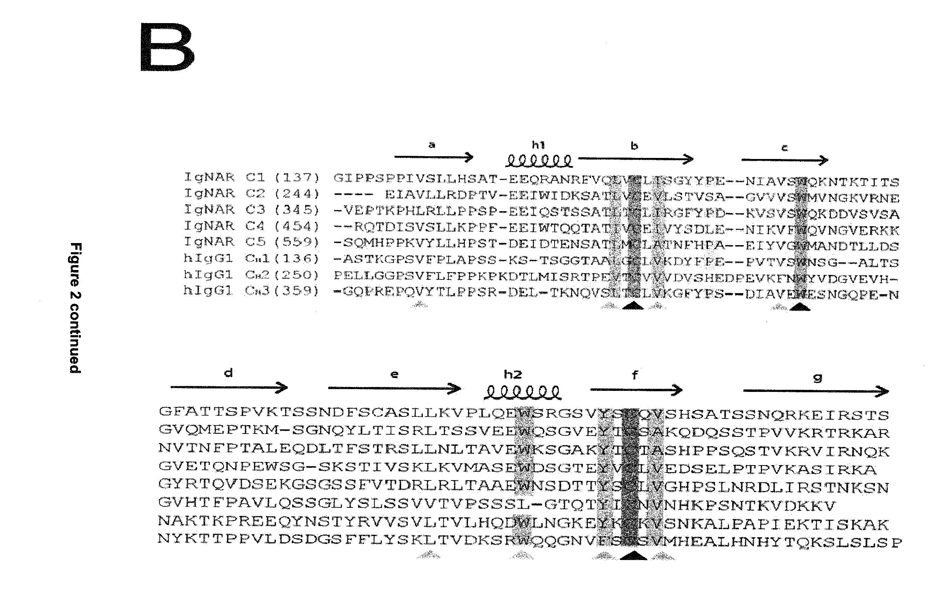

In their secreted form, IgNARs consist of two identical heavy chains composed of one variable (V) and five constant domains each (C1-5) (Dooley and Flajnik, 2006; Greenberg et al., 1995) (Figure 2). The structure of the variable domain of IgNAR has been solved (Stanfield et al., 2004; Streltsov et al., 2004) and was found to show similarity to the variable domains of evolutionary more recent immunoglobulins (Stanfield et al., 2004; Streltsov et al., 2004). In contrast, the constant domains (C1-C5, Figure 2) are most homologous to the primordial IgW of sharks (Berstein et al., 1996) which, out of the five human antibody classes IgA, D, E, G and M, is most related to the evolutionary old IgM (Berstein et al., 1996; Greenberg et al., 1996; Hsu et al., 2006). However, except for low-resolution electron microscopic images (Roux et al., 1998), so far, no structural data were available for any of the constant IgNAR domains.

In accordance with the present invention, the structure of the IgNAR constant domain C4 was determined at atomic resolution by NMR spectroscopy, as detailed in the appended examples. Based on this new structural information, elements were identified that are capable of increasing the stability and secretion of other proteins, such as e.g. mammalian antibodies. As is shown in the examples below, the incorporation of an additional salt bridge in accordance with (a) (also referred to herein as modification 1 = M1) and/or the extension of the hydrophobic core (also referred to herein as modification 2 = M2) led to a significant increase in the stability and the secretion of the thus modified proteins, which therefore provides an approach of generating proteins that, due to the increased secretion, can be produced more easily and that, due to the increased stability, maintain their function even under conditions in which the unmodified proteins would not be capable of maintaining their function.

Accordingly, in a further embodiment, the present invention also relates to a method of producing a modified protein having an improved secretion from cells as compared to the unmodified protein, the method comprising (i) modifying a nucleic acid molecule encoding a protein comprising at least one immunoglobulin constant domain-like region by (a-i) replacing the nucleotides encoding at least one amino acid, preferably an uncharged amino acid, in the loop separating the C strand from the D strand with nucleotides encoding a charged amino acid selected from the group consisting of Arg, Lys, His, Glu and Asp and/or replacing the nucleotides encoding at least one amino acid, preferably an uncharged amino acid, in the helix connecting the E strand with the F strand with nucleotides encoding a charged amino acid selected from the group consisting of Arg, Lys, His, Glu and Asp; (a-ii) replacing the nucleotides encoding at least one amino acid not having a side chain that can form a hydrogen bond in the loop separating the C strand from the D strand with nucleotides encoding an amino acid having a side chain capable of forming a hydrogen bond selected from the group consisting of Gln, Asn, Tyr, Ser and Thr and/or replacing the nucleotides encoding at least one amino acid not having a side chain that can form a hydrogen bond in the helix connecting the E strand with the F strand with nucleotides encoding an amino acid having a side chain capable of forming a hydrogen bond selected from the group consisting of Gin, Asn, Tyr, Ser and Thr; and/or (a-iii) replacing the nucleotides encoding at least one amino acid in the loop separating the C strand from the D strand with nucleotides encoding a cysteine and/or replacing the nucleotides encoding at least one amino acid in the helix connecting the E strand with the F strand with nucleotides encoding a cysteine; and/or (b) replacing the nucleotides encoding at least one non-hydrophobic amino acid at a position suitable to participate in the formation of the hydrophobic core with nucleotides encoding a hydrophobic amino acid selected from the group consisting of Val, Ile, Leu, Met, Phe, Trp and Pro; and (ii) expressing the nucleic acid molecule to produce the protein having improved secretion.

All of the definitions and preferred embodiments provided herein above with regard to the method of producing a modified protein having an increased stability/folding efficiency apply *mutatis mutandis* to this method of producing a modified protein having an improved secretion from cells. For example, the specific definitions and preferred embodiments regarding the modifications and the nature of the protein also apply to this method of producing a modified protein having an improved secretion from cells.

"Secretion" from a cell is a well-known biological process of releasing molecules from a cell. Various mechanisms have been described in the art for secretion from eukaryotic as well as bacterial cells, for example in (Alberts et al., 2007) or (Lodish et al., 2012). Preferably, the cell is a secretory cell. Any secretary cell is suitable in accordance with the present invention. Preferably, the secretory cell is selected from the group consisting of mammalian cells like CHO, 293T, COS or hybridoma cells. Alternatively, protein expression might be performed in other vertebrate cells or in insect cells, plant cells or fungal cells.

As discussed herein above and shown in the appended examples, the introduction of the described structural modifications into proteins comprising at least one immunoglobulin constant domain-like region results in an improved secretion of said modified proteins from cells. By improving the secretion of a protein from a cell, numerous advantages can be achieved, such as e.g. an increase in the amount of properly folded proteins that can be harvested from said cells; a reduction in the formation of inclusion bodies, which are made of aggregated proteins and the facilitation of proper disulfide bond formation, leading to an increase in the percentage of the protein in active form; more efficient isolation of the protein; reduced toxicity to the host cell; and an increased percentage of the recombinant protein in soluble form. Moreover, secretion of the protein into the culture medium allows for continuous, rather than batch culture for recombinant protein production.

The present invention further relates to a protein comprising at least one immunoglobulin constant domain-like region having an additional salt bridge, an additional hydrogen bond, an additional disulfide bond and/or an extended hydrophobic core, wherein the protein comprises one or more modifications selected from the group consisting of (i) a replacement of at least one amino acid, preferably an uncharged amino acid, in the loop separating the C strand from the D strand for a charged amino acid selected from the group consisting of Arg, Lys, His, Glu and Asp and/or a replacement of at least one amino acid, preferably an uncharged amino acid, in the helix connecting the E strand with the F strand for a charged amino acid selected from the group consisting of Arg, Lys, His, Glu and Asp, thereby enabling the formation of an additional salt bridge; (ii) a replacement of at least one amino acid not having a side chain that can form a hydrogen bond in the loop separating the C strand from the D strand for an amino acid having a side chain capable of forming a hydrogen bond selected from the group consisting of Gin, Asn, Tyr, Ser and Thr and/or a replacement of at least one amino acid not having a side chain that can form a hydrogen bond in the helix connecting the E strand with the F strand for an amino acid having a side chain capable of forming a hydrogen bond selected from the group consisting of Gln, Asn, Tyr, Ser and Thr; thereby enabling the formation of an additional hydrogen bond; (iii) a replacement of at least one amino acid in the loop separating the C strand from the D strand for a cysteine and/or a replacement of at least one amino acid in the helix connecting the E strand with the F strand for a cysteine; thereby enabling the formation of an additional disulfide bridge; and/or (iv) a replacement of at least one non-hydrophobic amino acid at a position suitable to participate in the formation of the hydrophobic core for a hydrophobic amino acid selected from the group consisting of Val, Ile, Leu, Met, Phe, Trp and Pro; thereby extending the hydrophobic core.

All of the definitions and preferred embodiments provided herein above with regard to the method of producing a modified protein having an increased stability and the method of producing a modified protein having an improved secretion from cells apply *mutatis mutandis* to this protein in accordance with the present invention. For example, the definitions and preferred embodiments regarding the modifications and the nature of the protein also apply to the claimed protein.

As discussed above, it was surprisingly found in accordance with the present invention that proteins comprising immunoglobulin constant domain-like regions having the recited modifications show an increased stability and improved secretion from cells as compared to said proteins prior to the introduction of the modification(s). These proteins are also referred to herein as "modified protein of the invention".

In a more preferred embodiment of the methods of the invention and of the modified protein of the invention, the at least one amino acid in the loop separating the C strand from the D strand is an amino acid in a position corresponding to position 48 in SEQ ID NO: 1 and the at least one amino acid in the helix connecting the E strand with the F strand is an amino acid in a position corresponding to position 76 in SEQ ID NO: 1.

The molecule represented by SEQ ID NO:1 is the nurse shark IgNAR C4 domain, which was found in accordance with the present invention to be particularly stable and can be efficiently secreted from cells. Accordingly, this molecule serves as a reference molecule herein, in particular for the purpose of defining various structural aspects.

Thus, the term "an amino acid in a position corresponding to position [...] in SEQ ID NO: [...]", as used herein, relates to an amino acid that is identified by any one of the methods mentioned below to have the same position within the protein to be modified as the amino acid at the recited position in the reference protein, such as e.g. SEQ ID NO:1. Thus, this term refers to the same position within the folded protein analysed as compared to the folded reference protein, e.g. the protein of SEQ ID NO:1. Said amino acid thus may be at a different position based on the primary amino acid sequence, but, preferably, is at a position corresponding to the recited position when analysed in a structure or sequence alignment, e.g. based on domain structure and/or homology.

The position in the protein to be modified can be identified by several techniques well known for the skilled person, as detailed herein above. Most preferred, the three-dimensional structure of the protein to be modified is available and the position can be identified by e.g. a superimposition with the reference protein. Otherwise, a homology model of the protein to be modified may be used to identify the corresponding residue in the reference protein. Additionally, a sequence alignment of the protein to be modified can be used to identify the corresponding residue in the reference protein. Such methods have been described herein above.

The immunoglobulin constant domain represented in SEQ ID NO:2 may also serve as a reference molecule. Position 48 of SEQ ID NO:1 corresponds to position 45 of SEQ ID NO:2 and position 76 of SEQ ID NO:1 corresponds to position 73 of SEQ ID NO:2. Accordingly, in an additional, or alternative, preferred embodiment of the methods of the invention and of the modified protein of the invention, the at least one amino acid in the loop separating the C strand from the D strand is an amino acid in a position corresponding to position 45 in SEQ ID NO: 2 and the at least one amino acid in the helix connecting the E strand with the F strand is an amino acid in a position corresponding to position 73 in SEQ ID NO: 2.

Applied to, for example, the C_{L} domain represented by SEQ ID NO:3, the at least one amino acid in the loop separating the C strand from the D strand corresponding to position 48 in SEQ ID NO: 1 (or position 45 in SEQ ID NO:2) is the glutamine at position 47 of SEQ ID NO:3; and the at least one amino acid in the helix connecting the E strand with the F strand corresponding to position 76 in SEQ ID NO: 1 (or position 73 in SEQ ID NO:2) is the aspartic acid at position 77 of SEQ ID NO:3. Applied to, for example, the CH2 domain represented by SEQ ID NO:18, the at least one amino acid in the loop separating the C strand from the D strand corresponding to position 48 in SEQ ID NO: 1 (or position 45 in SEQ ID NO:2) is the valine at position 57 of SEQ ID NO:18; and the at least one amino acid in the helix connecting the E strand with the F strand corresponding to position 76 in SEQ ID NO: 1 (or position 73 in SEQ ID NO:2) is the aspartic acid at position 85 of SEQ ID NO:18.

Further non-limiting examples include: replacement of the valine at position 49 and the glutamine at position 78 of SEQ ID NO:4; replacement of the valine at position 49 and the glutamine at position 78 of SEQ ID NO:5; replacement of the alanine at position 49 and the glutamine at position 78 of SEQ ID NO:6; replacement of the leucine at position 46 and the serine at position 75 of SEQ ID NO:17; replacement of the leucine at position 46 and the asparagine at position 75 of SEQ ID NO:20; replacement of the valine at position 53 and the aspartic acid at position 81 of SEQ ID NO:21; replacement of the leucine at position 46 and the serine at position 75 of SEQ ID NO:23; replacement of the valine at position 54 and the aspartic acid at position 82 of SEQ ID NO:24; replacement of the leucine at position 46 and the serine at position 75 of SEQ ID NO:26; and replacement of the valine at position 54 and the aspartic acid at position 82 of SEQ ID NO:27.

Likewise, the same position in other proteins comprising immunoglobulin constant domain-like regions, such as e.g. the remaining sequences listed in tables 2 and 3, can be modified. As described elsewhere herein, suitable positions can be determined by the skilled person based on structural alignments.

In a further more preferred embodiment of the methods of the invention and of the modified protein of the invention, the at least one non-hydrophobic amino acid in a position suitable to participate in the formation of the hydrophobic core is an amino acid in the D strand.

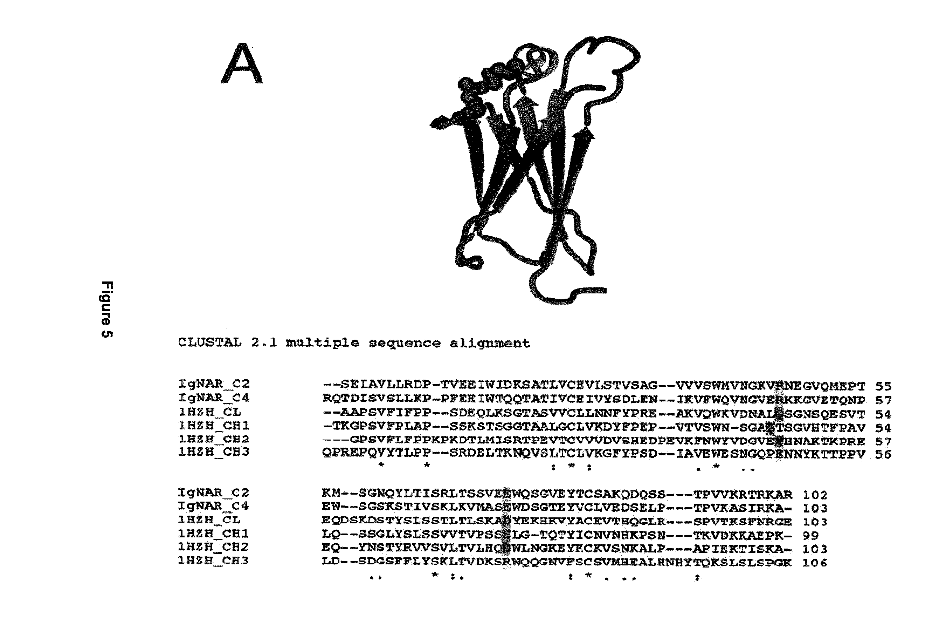

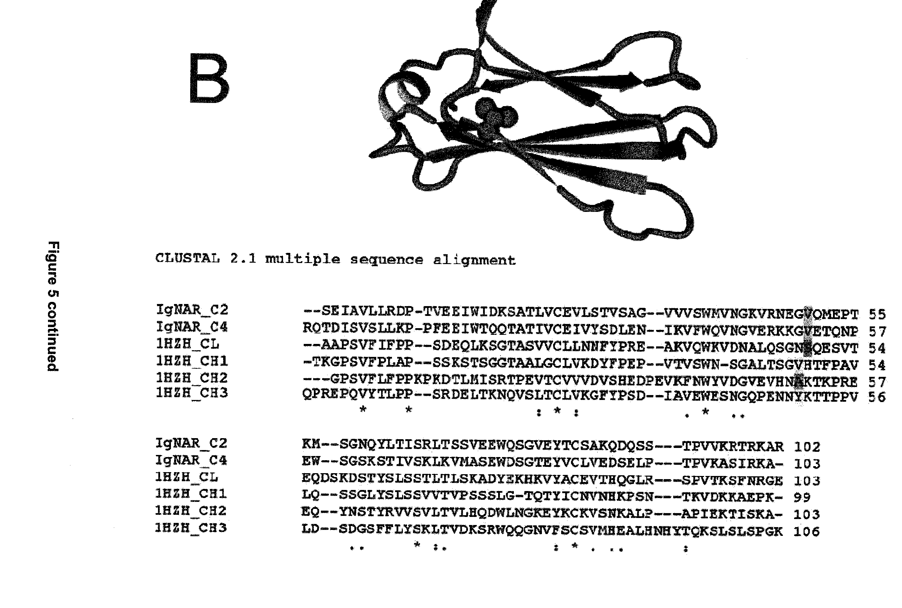

As described herein above, the skilled person is aware of suitable methods for identifying the sequence corresponding to the D strand within an immunoglobulin constant domain or an immunoglobulin constant domain-like region. For example, in the reference sequences shown in SEQ ID NOs: 1 and 2, the D strand extends from position 52 to 60 and position 49 to 57, respectively (also shown in Figure 5). The following table 4 provides an overview of selected immunoglobulin constant domains, their SEQ ID number as well as the position of the D strand.

**Table 4: The position of the D strand in selected immunoglobulin constant domains.**

| **Ig constant domain (UniProtKB accession number)** | **SEQ ID NO:** | **Position of the D strand in the indicated SEQ ID NO** | **Position of the D strand in the indicated UniProtKB accession number** |
|---|---|---|---|
| Nurse Shark C4 (Q90544) | 1 | 52- 60 | 505-514 |
| Nurse Shark C2 (Q90544) | 2 | 49-57 | 292-301 |
| Human Ig Kappa LC (P01834) | 3 | 51-60 | 51-60 |
| Human Ig lambda-1 (P0CG04) | 4 | 53-62 | 53-62 |
| Human Ig lambda-2 (P0CG05) | 5 | 53-62 | 53-62 |
| Human Ig lambda-3 (P0CG06) | 6 | 53-62 | 53-62 |
| Mouse Ig kappa (P01837) | 7 | 52-62 | 52-62 |
| Mouse Ig lambda-1 (P01843) | 8 | 50-59 | 50-59 |
| Mouse Ig lambda-2 (P01844) | 9 | 50-59 | 50-59 |
| Mouse Ig lambda-3 (P01845) | 10 | 50-59 | 50-59 |
| Rabbit Ig kappa b4 (P01840) | 11 | 50-59 | 50-59 |
| Rabbit Ig kappa b5 (P01841) | 12 | 50-59 | 50-59 |
| Rabbit Ig kappa b5 var (P03984) | 13 | 50-59 | 50-59 |
| Rabbit Ig kappa b9 (P01838) | 14 | 51-60 | 51-60 |
| Rabbit Ig kappa b4 (P01839) | 15 | 52-61 | 52-61 |
| Rabbit Ig lambda (P01847) | 16 | 52-61 | 52-61 |
| Human Ig gamma 1 CH1 (P01857) | 17 | 52-61 | 52-61 |
| Human Ig gamma 1 CH2 (P01857) | 18 | 61-70 | 171-180 |
| Human Ig gamma 1 CH3 (P01857) | 19 | 51-60 | 274-283 |
| Human Ig gamma 2 CH1 (P01859) | 20 | 52-61 | 52-61 |
| Human Ig gamma 2 CH2 (P01859) | 21 | 57-66 | 167-176 |
| Human Ig gamma 2 CH3 (P01859) | 22 | 51-60 | 270-279 |
| Human Ig gamma 3 CH1 (P01860) | 23 | 52-61 | 52-61 |
| Human Ig gamma 3 CH2 (P01860) | 24 | 58-67 | 218-227 |
| Human Ig gamma 3 CH3 (P01860) | 25 | 51-60 | 321-330 |
| Human Ig gamma 4 CH1 (P01861) | 26 | 52-61 | 52-61 |
| Human Ig gamma 4 CH2 (P01861) | 27 | 58-67 | 168-177 |
| Human Ig gamma 4 CH3 (P01861) | 28 | 51-60 | 271-280 |
| Mouse Ig gamma 1 CH1 (P01868) | 29 | 52-61 | 52-61 |
| Mouse Ig gamma 1 CH2 (P01868) | 30 | 62-71 | 172-181 |
| Mouse Ig gamma 1 CH3 (P01868) | 31 | 51-60 | 268-277 |
| Mouse Ig gamma 2A CH1 (P01863) | 32 | 52-61 | 52-61 |
| Mouse Ig gamma 2A CH2 (P01863) | 33 | 51-60 | 171-180 |
| Mouse Ig gamma 2A CH3 (P01863) | 34 | 46-55 | 274-283 |
| Mouse Ig gamma 2B CH1 (P01867) | 35 | 51-60 | 51-60 |
| Mouse Ig gamma 2B CH2 (P01867) | 36 | 51-60 | 176-185 |
| Mouse Ig gamma 2B CH3 (P01867) | 37 | 46-55 | 279-288 |
| Mouse Ig gamma 3 CH1 (P03987) | 38 | 51-60 | 51-60 |
| Mouse Ig gamma 3 CH2 (P03987) | 39 | 58-67 | 171-180 |
| Mouse Ig gamma 3 CH3 (P03987) | 40 | 50-59 | 273-282 |
| Rabbit Ig gamma CH1 (P01870) | 41 | 52-61 | 52-61 |
| Rabbit Ig gamma CH2 (P01870) | 42 | 50-59 | 163-172 |
| Rabbit Ig gamma CH3 (P01870) | 43 | 46-55 | 267-276 |
| Human Ig epsilon CH1 (P01854) | 44 | 54-63 | 54-63 |
| Human Ig epsilon CH2 (P01854) | 45 | 49-58 | 160-169 |
| Human Ig epsilon CH3 (P01854) | 46 | 53-62 | 266-275 |
| Human Ig epsilon CH4 (P01854) | 47 | 47-56 | 370-379 |
| Human Ig delta CH1 (P01880) | 48 | 53-62 | 53-62 |
| Human Ig delta CH2 (P01880) | 49 | 39-48 | 214-223 |
| Human Ig delta CH3 (P01880) | 50 | 53-62 | 319-328 |
| Human Ig mu CH1 (P01871) | 51 | 53-62 | 53-62 |
| Human Ig mu CH2 (P01871) | 52 | 55-64 | 160-169 |
| Human Ig mu CH3 (P01871) | 53 | 53-62 | 270-279 |
| Human Ig mu CH4 (P01871) | 54 | 53-62 | 376-385 |
| Human Ig alpha CH1 (P01876) | 55 | 52-61 | 52-61 |
| Human Ig alpha CH2 (P01876) | 56 | 49-58 | 173-182 |
| Human Ig alpha CH3 (P01876) | 57 | 48-57 | 275-284 |
| Human Ig alpha 2 CH1 (P01877) | 58 | 52-61 | 52-61 |
| Human Ig alpha 2 CH2 (P01877) | 59 | 49-58 | 160-169 |
| Human Ig alpha 2 CH3 (P01877) | 60 | 48-57 | 262-271 |
| Rat Ig Kappa chain C (P01836) | 61 | 51-60 | 51-60 |
| Chicken Ig lambda C (P20763) | 62 | 53-62 | 53-62 |
| Horse Ig gamma (Q95M34) | 63 | 50-59 | 176-185 |
| Giant Panda CH1 (D2I829) | 64 | 57-66 | 57-66 |
| Giant Panda CH2 (D2I829) | 65 | 57-66 | 162-171 |
| Giant Panda CH3 (D2I829) | 66 | 51-60 | 266-275 |
| African clawed frog (Q6INK3) | 67 | 49-59 | 409-418 |

In an even more preferred embodiment of the methods of the invention and of the modified protein of the invention, the at least one non-hydrophobic amino acid is an amino acid at a position corresponding to position 52 in SEQ ID NO: 1.

The immunoglobulin constant domain represented in SEQ ID NO:2 may alternatively, or additionally, serve as a reference molecule. Position 52 of SEQ ID NO:1 corresponds to position 49 of SEQ ID NO:2. Accordingly, in an additional, or alternative, preferred embodiment of the methods of the invention and of the modified protein of the invention, the at least one non-hydrophobic amino acid is an amino acid at a position corresponding to position 49 in SEQ ID NO: 2.

Applied to for example the C_{L} domain represented in SEQ ID NO:3, the at least one non-hydrophobic amino acid at a position corresponding to position 52 in SEQ ID NO: 1 (or position 49 in SEQ ID NO:2) is the serine at position 51 of SEQ ID NO:3. Applied to for example the CH2 domain represented in SEQ ID NO:18, the at least one non-hydrophobic amino acid at a position corresponding to position 52 in SEQ ID NO: 1 (or position 49 in SEQ ID NO:2) is the alanine at position 60 of SEQ ID NO:18. Other suitable positions for an amino acid exchange for a hydrophobic amino acid in order to extend the hydrophobic core include, without being limiting: the alanine at position 56 of SEQ ID NO:21; the alanine at position 57 of SEQ ID NO:24; or the alanine at position 57 of SEQ ID NO:27.

Likewise, the same position in other proteins comprising immunoglobulin constant domain-like regions, such as e.g. the remaining sequences listed in table 4, can be modified by exchanging a non-hydrophobic amino acid for a hydrophobic amino acid to extend the hydrophobic core and stabilize the protein. As described elsewhere herein, suitable positions can be determined by the skilled person based on structural alignments.

In a particularly preferred embodiment of the method of the invention or the modified protein of the invention, the amino acids to be modified in the specifically recited amino acid sequences are:
(i) replacement of the glutamine at position 47 and the aspartic acid at position 77 of SEQ ID NO:3 and/or replacement of the serine at position 51 of SEQ ID NO:3;
(ii) replacement of the valine at position 49 and the glutamine at position 78 of SEQ ID NO:4;
(iii) replacement of the valine at position 49 and the glutamine at position 78 of SEQ ID NO:5;
(iv) replacement of the alanine at position 49 and the glutamine at position 78 of SEQ ID NO:6;
(v) replacement of the leucine at position 46 and the serine at position 75 of SEQ ID NO:17;
(vi) replacement of the valine at position 57 and the aspartic acid at position 85 of SEQ ID NO:18 and/or replacement of the alanine at position 60 of SEQ ID NO:18;
(vii) replacement of the leucine at position 46 and the asparagine at position 75 of SEQ ID NO:20;
(viii) replacement of the valine at position 53 and the aspartic acid at position 81 of SEQ ID NO:21 and/or replacement of the alanine at position 56 of SEQ ID NO:21;
(ix) replacement of the leucine at position 46 and the serine at position 75 of SEQ ID NO:23;
(x) replacement of the valine at position 54 and the aspartic acid at position 82 of SEQ ID NO:24 and/or replacement of the alanine at position 57 of SEQ ID NO:24;
(xi) replacement of the leucine at position 46 and the serine at position 75 of SEQ ID NO:26;
(xii) replacement of the valine at position 54 and the aspartic acid at position 82 of SEQ ID NO:27 and/or replacement of the alanine at position 57 of SEQ ID NO:27.

Most preferably, the modified protein of the invention is a protein or the method of the present invention yields a protein comprising the amino acid sequence of SEQ ID NO: 3, wherein the serine at position 51 of SEQ ID NO:3 has been replaced by a hydrophobic amino acid, preferable valine; the glutamine at position 47 of SEQ ID NO:3 is replaced by a charged amino acid, preferably an arginine; and the aspartic acid at position 77 of SEQ ID NO:3 has been replaced by a charged amino acid, preferably a glutamic acid.

In a preferred embodiment of the methods or the modified protein of the invention, the protein comprising at least one immunoglobulin constant domain-like region is an antibody. In an even more preferred embodiment of the methods or the modified protein of the invention, the antibody is a human or humanised antibody.

In a further preferred embodiment of the methods or the modified protein of the invention, the protein comprising at least one immunoglobulin constant domain-like region is a eukaryotic protein.

The present invention further relates to a nucleic acid molecule encoding the modified protein of the invention.

The definition and preferred embodiments with regard to "nucleic acid molecules" has been provided herein above. As also detailed above, the term "nucleic acid molecule of the invention" encompasses both a single nucleic acid molecule as well as a plurality of nucleic acid molecules, as long as all the components of the modified protein of the invention are encoded thereby.

The present invention further relates to a vector comprising the nucleic acid molecule of the invention.

Preferably, the vector is a plasmid, cosmid, virus, bacteriophage or another vector used conventionally e.g. in genetic engineering.

The nucleic acid molecule of the present invention may be inserted into several commercially available vectors. Non-limiting examples include prokaryotic plasmid vectors, such as pQE-12, the pUC-series, pBluescript (Stratagene), the pET-series of expression vectors (Novagen) or pCRTOPO (Invitrogen), lambda gt11, pJOE, the pBBR1-MCS series, pJB861, pBSMuL, pBC2, pUCPKS, pTACT1 and vectors compatible with expression in mammalian cells like pSVL (Amersham), E-027 pCAG Kosak-Cherry (L45a) vector system, pREP (Invitrogen), pCEP4 (Invitrogen), pMC1 neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, Okayama-Berg cDNA expression vector pcDV1 (Pharmacia), pRc/CMV, pcDNA1, pcDNA3 (Invitrogen), pcDNA3.1, pSPORT1 (GIBCO BRL), pGEMHE (Promega), pLXIN, pSIR (Clontech), pIRES-EGFP (Clontech), pEAK-10 (Edge Biosystems) pTriEx-Hygro (Novagen) and pCINeo (Promega). A non-limiting example of a suitable expression vector for insect cells is pACGP67 (BD), as employed in the appended Examples. Examples for plasmid vectors suitable for *Pichia pastoris* comprise e.g. the plasmids pAO815, pPIC9K and pPIC3.5K (all Invitrogen). Another vector suitable for expressing proteins in *Xenopus* embryos, zebrafish embryos as well as a wide variety of mammalian and avian cells is the multipurpose expression vector pCS2+.

The nucleic acid molecule of the present invention referred to above may also be inserted into vectors such that a translational fusion with another nucleic acid molecule is generated. The other nucleic acid molecules may e.g. encode a protein that increases the solubility and/or facilitates the purification of the modified proteins encoded by the nucleic acid molecule of the invention. Non-limiting examples of such vectors include pET32, pET41, pET43. The vectors may also contain an additional expressible polynucleotide coding for one or more chaperones to facilitate correct protein folding. Suitable bacterial expression hosts comprise e. g. strains derived from TG1, BL21 (such as BL21(DE3), BL21(DE3)PlysS, BL21 (DE3)RIL, BL21 (DE3)PRARE) or Rosetta.

For vector modification techniques, see (Green and Sambrook, 2012). Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Such regulatory sequences are well known to those skilled in the art and include, without being limiting, regulatory sequences ensuring the initiation of transcription, internal ribosomal entry sites (IRES) ((Owens et al., 201)) and optionally regulatory elements ensuring termination of transcription and stabilization of the transcript. Non-limiting examples for regulatory elements ensuring the initiation of transcription comprise a translation initiation codon, enhancers such as e.g. the SV40-enhancer, insulators and/or promoters, such as for example the cytomegalovirus (CMV) promoter, SV40-promoter, RSV-promoter (Rous sarcome virus), the lacZ promoter, chicken beta-actin promoter, CAG-promoter (a combination of chicken beta-actin promoter and cytomegalovirus immediate-early enhancer), the gai10 promoter, human elongation factor 1α-promoter, AOX1 promoter, GAL1 promoter CaM-kinase promoter, the lac, trp or tac promoter, the lacUV5 promoter, the autographa californica multiple nuclear polyhedrosis virus (AcMNPV) polyhedral promoter or a globin intron in mammalian and other animal cells. The lac promoter is a typical inducible promoter, useful for prokaryotic cells, which can be induced using the lactose analogue isopropylthiol-b-D-galactoside ("IPTG"). Non-limiting examples for regulatory elements ensuring transcription termination include the V40-poly-A site, the tk-poly-A site or the SV40, lacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing, nucleotide sequences encoding secretion signals or, depending on the expression system used, signal sequences capable of directing the expressed polypeptide to a cellular compartment. For example an N-terminal flanking sequence or "leader sequence", which is also referred to as "signal peptide" in the art, may be included. The skilled person can choose suitable leader sequences without further ado. A leader sequence is preferably employed for the expression of any antibody chain (including light chain, heavy chain) or domain but is no longer required in the expressed, mature construct. Moreover, elements such as origin of replication, drug resistance gene, regulators (as part of an inducible promoter) may also be included.

An expression vector according to this invention is capable of directing the replication of the nucleic acid molecule of the invention and the expression of the modified protein encoded thereby.

The co-transfection with a selectable marker such as DHFR (dihydrofolate reductase), gpt, neomycin, hygromycin allows the identification and isolation of the transfected cells. The transfected nucleic acid can also be amplified to express large amounts of the encoded modified protein. The DHFR marker is useful to develop cell lines that carry several hundred or even several thousand copies of the gene of interest. Another useful selection marker is the enzyme glutamine synthase (GS) ((Bebbington et al., 1992; Murphy et al., 1991)). Using these markers, the cells are grown in selective medium and the cells with the highest resistance are selected. Expression vectors will preferably include at least one selectable marker. Such markers include dihydrofolate reductase, G418 or neomycin resistance for eukaryotic cell culture and tetracycline, kanamycin or ampicillin resistance genes for culturing in *E*. *coli* and other bacteria.

The nucleic acid molecules of the invention as described herein above may be designed for direct introduction or for introduction via electroporation (using for example Multiporator (Eppendorf) or Genepulser (BioRad)), PEI (Polysciences Inc. Warrington, Eppelheim), Ca²⁺-mediated transfection or via liposomes (for example: "Lipofectamine" (Invitrogen)), non-liposomal compounds (for example: "Fugene" (Roche)), liposomes, phage vectors or viral vectors (e.g. adenoviral, retroviral, lentiviral) into cells. Additionally, baculoviral systems or systems based on Vaccinia Virus or Semliki Forest Virus can also be used as vector in eukaryotic expression system for the nucleic acid molecules of the invention. Expression vectors derived from viruses such as retroviruses, vaccinia virus, adeno-associated virus, herpes viruses, or bovine papilloma virus, may be used for delivery of the nucleic acid molecules or vector into targeted cell population. Methods which are well known to those skilled in the art can be used to construct recombinant viral vectors; see, for example, the techniques described in (Ausubel et al., 2012; Green and Sambrook, 2012).

It will be appreciated that where the modified protein of the invention is encoded by more than one nucleic acid molecule, said plurality of nucleic acid molecules may be comprised in one or in a plurality of vectors. The term "the vector of the invention" encompasses both a single vector as well as a plurality of vectors, as long as all the components of the modified protein of the invention are encoded thereby.

The present invention further relates to a host cell or a non-human host transformed with the vector of the invention.

Said host or host cell may be produced by introducing the vector of the invention into a host or host cell, which upon its presence mediates the expression of the modified protein encoded by the vector.

In accordance with the present invention, the host may be a transgenic non-human animal transfected with and/or expressing the vector of the present invention. In a preferred embodiment, the transgenic animal is a mammal, e.g. a mouse, rabbit, rat, hamster, cow, cat, pig, dog or horse.

In a preferred embodiment, the host is a cell, such as an isolated cell, which may be part of a cell culture.

Suitable prokaryotic host cells comprise e.g. bacteria of the species *Escherichia, Bacillus, Streptomyces* and *Salmonella typhimurium.* Suitable eukaryotic host cells are e.g. fungal cells, inter alia, yeasts such as *Saccharomyces cerevisiae* or *Pichia pastoris* or insect cells such as Drosophila S2 and Spodoptera Sf9 cells and plant cells as well as mammalian cells. Appropriate culture mediums and conditions for the above-described host cells are known in the art.

Mammalian host cells include without being limiting human Hela, HEK293, H9 and Jurkat cells, mouse NIH3T3 and C127 cells, COS 1, COS 7 and CV1, quail QC1-3 cells, mouse L cells, Chinese hamster ovary (CHO) cells and Bowes melanoma cells. Alternatively, the modified protein of the invention can be expressed in stable cell lines that contain the gene construct encompassing the nucleic acid molecule or the vector of the invention integrated into a chromosome. In addition, the modified protein of the invention can be expressed in hybridoma cells that contain the gene construct encompassing the nucleic acid molecule or the vector of the invention integrated into a chromosome.

In another preferred embodiment, said cell is a primary cell or primary cell line. Primary cells are cells, which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

The present invention also relates to a method for the production of a modified protein according to the invention comprising (i) culturing the host cell of the invention under suitable conditions and (ii) isolating the modified protein of the invention produced by said host cell.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E*. *coli* can be cultured from 4 to about 37 °C, the exact temperature or sequence of temperatures depends on the molecule to be over-expressed. In general, the skilled person is also aware that these conditions may have to be adapted to the needs of the host and the requirements of the modified protein expressed. In case an inducible promoter controls the nucleic acid molecule of the invention in the vector present in the host cell, expression of the modified protein can be induced by addition of an appropriate inducing agent. Suitable expression protocols and strategies are known to the skilled person.

Depending on the cell type and its specific requirements, mammalian cell cultures can e.g. be carried out in RPMI or DMEM medium containing 10% (v/v) FCS, 2mM L-glutamine and 100 U/ml penicillin/streptomycine. The cells can be kept at 37 °C in a 5% CO₂, water saturated atmosphere. Suitable media for insect cell culture is e.g. TNM + 10% FCS or SF900 medium. Insect cells are usually grown at 27 °C as adhesion or suspension culture. Suitable expression protocols for eukaryotic cells are well known to the skilled person and can be retrieved e.g. from (Green and Sambrook, 2012), loc cit.

Methods of isolating the modified protein produced are well-known in the art and comprise, without being limiting, method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in (Green and Sambrook, 2012), loc. cit.

The present invention also provides a composition comprising at least one of (i) the modified protein of the invention, (ii) the nucleic acid molecule of the invention; (iii) the vector of the invention or (iv) the host cell of the invention. Preferably, the composition comprises a modified antibody in accordance with the invention.

The term "composition", as used in accordance with the present invention, relates to a composition, which comprises at least one of the recited compounds. It may, optionally, comprise further molecules capable of altering the characteristics of the compounds of the invention thereby, for example, stabilizing, modulating and/or enhancing their function. The composition may be in solid or liquid form and may be, inter alia, in the form of (a) powder(s), (a) tablet(s) or (a) solution(s).

In a preferred embodiment, the composition is a pharmaceutical composition optionally further comprising a pharmaceutically acceptable carrier.

In accordance with the present invention, the term "pharmaceutical composition" relates to a composition for administration to a patient, preferably a human patient. The pharmaceutical composition of the invention comprises the compounds recited above. The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art and include sodium chloride solutions, phosphate buffered sodium chloride solutions, water, emulsions, such as oil/water emulsions, various types of wetting agents, sterile solutions, organic solvents etc. Preferably the carrier is a parenteral carrier, more preferably a solution that is isotonic with the blood of the recipient. The term "parenteral" as used herein refers to modes of administration, which include intravenous, intramuscular, intraperitoneal, intrasternal, subcutaneous and intraarticular injection and infusion. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or further immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

Compositions comprising such carriers can be formulated by well known conventional methods. Generally, the formulations are prepared by contacting the components of the pharmaceutical composition uniformly and intimately with liquid carriers or finely divided solid carriers or both. Then, if necessary, the product is shaped into the desired formulation.

These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. The pharmaceutical composition may be for administration once or for a regular administration over a prolonged period of time. Generally, the administration of the pharmaceutical composition should be in the range of for example 10µg/kg of body weight to 2g/kg of body weight for a single dose. However, a more preferred dosage might be in the range of 100µg /kg to 1.5g/kg of body weight, even more preferably 1 mg/kg to 1 g/kg of body weight and even more preferably 5mg/kg to 500mg/kg of body weight for a single dose.

Administration of pharmaceutical compositions of the invention may be effected by different ways, e.g., by intravenous, intraperitoneal, subcutaneous, intramuscular, intradermal, intranasal or intrabronchial administration.

The components of the pharmaceutical composition to be used for therapeutic administration must be sterile. Sterility can for example be accomplished by filtration through sterile filtration membranes (e.g., 0.2 micron membranes).

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic water-for-injection. Preservatives and other additives may also be present such as, for example, antimicrobials, anti oxidants, chelating agents, and inert gases and the like. The pharmaceutical composition may comprise further agents depending on the intended use of the pharmaceutical composition.

In another preferred embodiment, the composition of the invention is a diagnostic composition.

In accordance with the present invention, the term "diagnostic composition" relates to compositions for diagnosing individual patients for their potential response to or curability by the pharmaceutical compositions of the invention. The diagnostic composition of the invention comprises the compounds recited above. The diagnostic composition may further comprise appropriate buffer(s) etc.. The diagnostic compositions may be packaged in a container or a plurality of containers.

The present invention further relates to a kit comprising at least one of (i) the modified protein of the invention, (ii) the nucleic acid molecule of the invention; (iii) the vector of the invention or (iv) the host cell of the invention. Preferably, the composition comprises a modified antibody in accordance with the invention.

In its broadest sense, the term "kit" does not require the presence of any other compounds, vials, containers and the like. Preferably, the various components of the kit may be packaged in one or more containers such as one or more vials. Consequently, the various components of the kit may be present in isolation or combination. The containers or vials may, in addition to the components, comprise preservatives or buffers for storage. In addition, the kit may contain instructions for use.

Unless otherwise defined, all technical and scientific terms used herein have the same meaning as commonly understood by one of ordinary skill in the art to which this invention belongs. In case of conflict, the patent specification, including definitions, will prevail.

As regards the embodiments characterised in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims. To give a non-limiting example, the combination of claims 7 and 1 is clearly and unambiguously envisaged in view of the claim structure. The same applies for example to the combination of claims 7, 6, 5 and 1, etc..

The figures show:
**Figure 1****. The Ig fold.** Schematic of the C-type Ig fold with important structural elements highlighted (helices are marked, strands are labeled and the internal disulfide bridge is indicated with an arrow).
**Figure 2****. IgNAR sequence and schematic structure** (A) Schematic of the secreted dimeric IgNAR molecule, comprising one variable (V) and five constant domains (C1-5). Predicted glycosylation sites are shown as grey hexagons. Cysteines that are not part of the intradomain disulfide bridges are indicated. The secretory tail is C-terminally of the C5 domain. (B) Sequence alignment of IgNAR C1-5 with the human IgG1 heavy chain domains C_{H}1-C_{H}3. Conserved cysteines are highlighted in dark grey, conserved hydrophobic residues of a YxCxY (Y=hydrophobic residue) motif around the disulfide bridge are highlighted in light grey. Conserved tryptophans (W) in strand c and the second helix are highlighted in grey, the cis-proline residue in the loop between strand b and c is boxed in grey. Secondary structure elements are indicated above the alignment. Black arrow indicates strictly conserved residues, grey arrows indicate homologous residues.

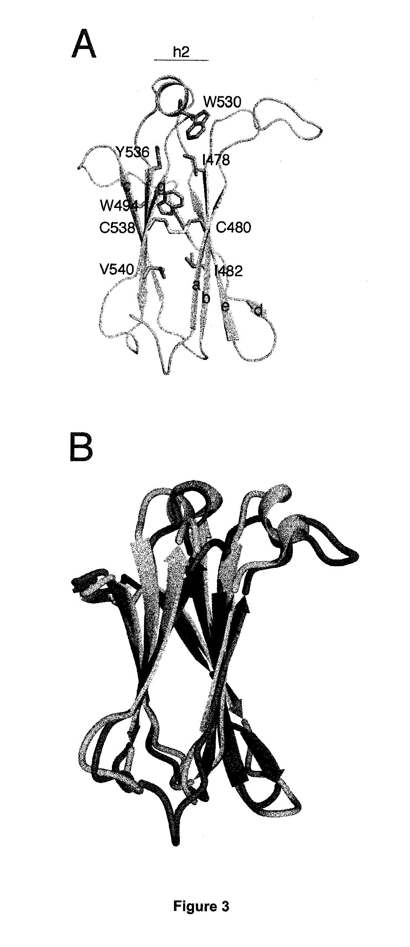

**Figure 3****. Atomic resolution structure of the IgNAR C4 domain.** (A) Ribbon diagram of the isolated constant IgNAR domains C4 (Residues marked in the alignment in Figure 2 are shown in stick representation, the small helix is indicated). (B) Superposition of the IgNAR C4 domain (C4: black) on a human IgG C_{L} domain (gray, pdb entry code: 1 HZH).

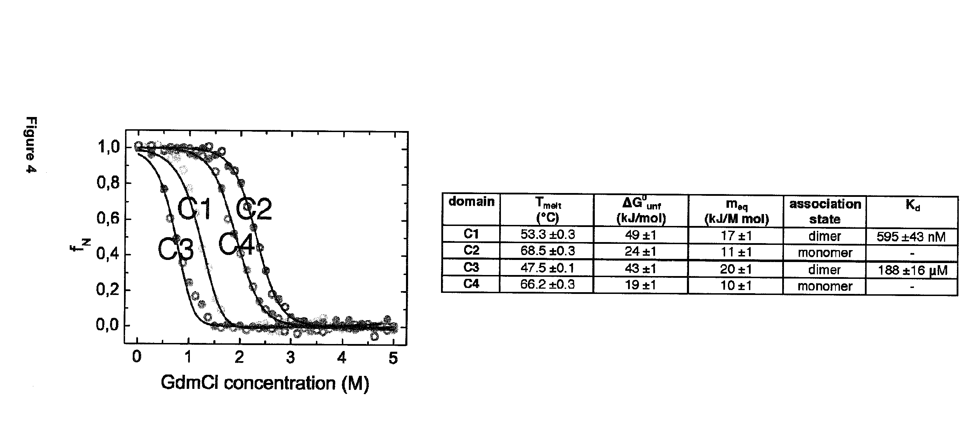

**Figure 4****. Stability and oligomerization state of the constant IgNAR domains.** C1-C4 were reversibly unfolded by the chemical denaturant GdmCl (unfolding: closed circles, refolding: open circles). Midpoints of thermal transitions and thermodynamic parameters obtained by GdmCl-induced unfolding transitions are listed in the table. The association state and dissociation constant K_{d} of the individual domains in solution was obtained by analytical ultracentrifugation. All data are shown ±1SD.
**Figure 5****. Design of mutant M1 ((A), additional salt bridge) and M2 ((B), extension of the hydrophobic core).** On the top of each panel, C4 is shown. The residues that are to be implemented into the human domains are shown in a ball representation and highlighted in the multiple sequence alignment below. If marked in the multiple alignment in dark grey, the corresponding human domain is lacking the structural motif found in the shark domains and hence amenable to optimization at this site.

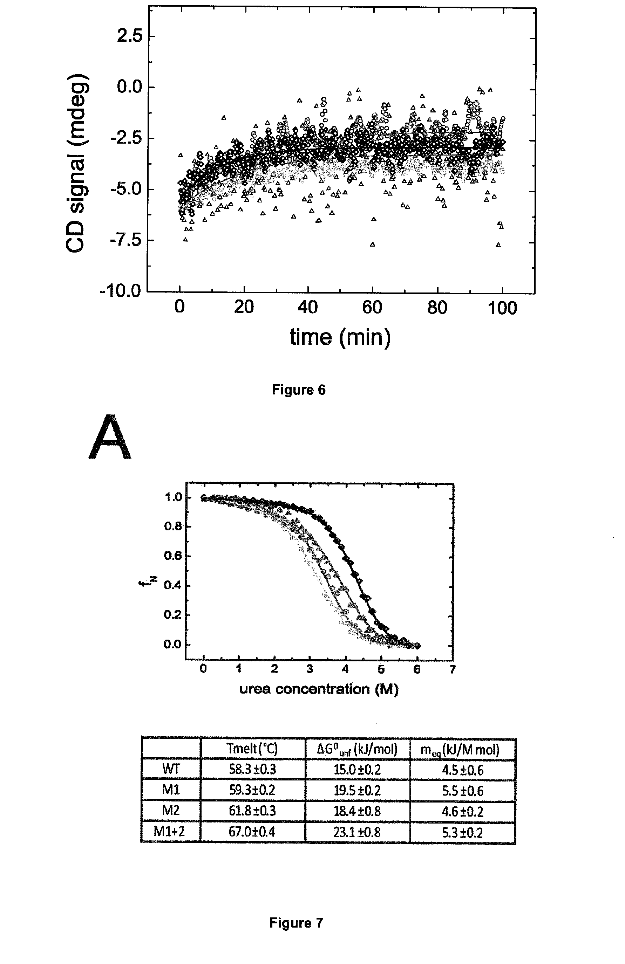

**Figure 6****.** Association-coupled folding of the human IgG1 C_{H}1 domain by human k C_{L} and the mutant M1, M2 and M3. Folding of 10 µM C_{H}1 was followed by CD spectroscopy upon addition of 10 µM of human k C_{L} wt (squares), M1 (circles), M2 (triangles) and the M3 mutant (diamonds). All kinetics were fitted with a single exponential.

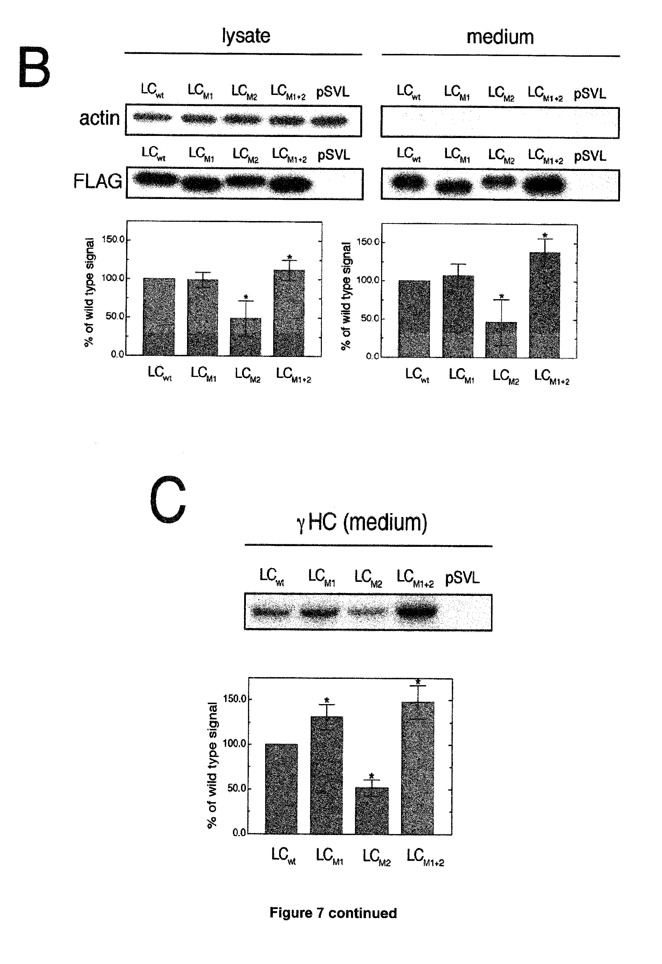

**Figure 7****. The impact of shark-based mutants of a human C_{L} domain on domain stability and antibody secretion.** (A) Thermal stabilities and thermodynamic parameters derived from urea melts are shown for the wt C_{L} domain and mutants M1, M2 and M1+2 on the right. Urea melts are shown on the left (wt: squares, M1: circles, M2: triangles, M1+2: diamonds; filled symbols represent unfolding transitions and open symbols refolding transitions). (B) Expression (lysate) and secretion (medium) of FLAG-tagged human K LC's, either wt or comprising mutations as indicated, were analyzed. Immunoblots of the lysates are shown on the left; immunoblots of the media are shown on the right.

Actin was used as a control. Constructs transfected are indicated above the lanes. Expression and secretion levels were quantified (n=7±SD; data that are statistically significantly different from the wt (p≤.05) are marked with an asterisk). (C) LC_{wt}, LC_{M1}, LC_{M2} and LCₘ₁₊₂ or empty pSVL vector were co-expressed with γHCs and the amount of secreted γHCs was analyzed. Cells were metabolically labeled for 1h and subsequently chased for 24h before analysis of the medium. Data were quantified by phosphorimager analysis (n=4±SD; data that are statistically significantly different from the wt (pH0.05) are marked with an asterisk).

The examples illustrate the invention:

### Example 1: Materials and methods

### Cloning, expression and purification of the constant domains of IgNAR, the human κ C_{L} domain and mutants of the human K C_{L} domain

The genes of the constant IgNAR domains C2 and C4 were amplified by PCR using the IgNAR cDNA of nurse shark as a template. The genes of the C1, C3 and C5 domains were amplified by PCR using a synthetic, *E. coli* codon-usage optimized gene encoding the complete nurse shark IgNAR (Geneart, Regensburg, Germany). Domain boundaries were derived from (Greenberg et al., 1995). PCR products were cloned into the pET28a expression vector (Novagen, Gibbstown, NJ, USA) with an N-terminal Thrombin-cleavable His-tag (C1) or without a tag (C2-C5). For the wt human K C_{L} domain as well as mutants thereof synthetic, *E. coli* codon-usage optimized genes were used (Geneart, Regensburg, Germany).

Expression was carried out in *E. coli* BL21 Star DE3 (Invitrogen, Darmstadt, Germany) overnight at 37°C in selective LB medium. All proteins were expressed as inclusion bodies. For C1, inclusion bodies were solubilized in 100 mM sodium phosphate, pH 7.5, 5 M GdmCl, 5 mM β-mercaptoethanol and applied to a Ni-sepharose FF column (GE Healthcare, Munich, Germany) equilibrated in 100 mM sodium phosphate, pH 7.5, 3 M GdmCl. Elution was carried out by a shift to pH 3.5 in the same buffer. For C2-C5 and K C_{L} constructs, inclusion bodies were solubilized in 100 mM Tris/HCl, pH 8.0, 8 M urea, 10 mM EDTA, 10 mM β-mercaptoethanol and applied to a Q-sepharose column equilibrated in 100 mM Tris/HCl, pH 8.0, 5 M urea, 10 mM EDTA. All proteins of interest did not bind to the Q-sepharose column. For C2-C5, the flowthrough was applied to a SP-sepharose column equilibrated in 100 mM Tris/HCl, pH 8.0, 5 M urea, 10 mM EDTA. C2 and C3 bound to the SP-sepharose column whereas C4 and C5 did not bind to the column. C2 and C3 were eluted with 1 M NaCl in the same buffer. C1-C3 and C5 were subsequently dialyzed against 100 mM Tris/HCl, pH 8.2, 3 M GdmCl, 10 mM EDTA, 1 mM DTT and applied to a Superdex 200pg (26/60) gel filtration column (GE Healthcare, Munich, Germany) equilibrated in the same buffer. All proteins were refolded *via* dialysis in 250 mM Tris/HCl, 100 mM L-Arg, pH 8.0, 5 mM EDTA, 1 mM oxidized glutathione, 0.5 mM reduced glutathione at 4°C over night. Before refolding, C2 and the C_{L} double mutant (hydrophobic core and salt bridge, M1+2) were incubated with 10 mM oxidized glutathione for 1 h at RT. The His-tag of C1 was cleaved with Thrombin over night at 4°C after refolding. Thereafter, all proteins were applied to a Superdex 75pg (26/60) gel filtration column (GE Healthcare, Munich, Germany) equilibrated in PBS. If necessary, proteins were further purified using a Superdex 75GL (10/300) HPLC column (GE Healthcare, Munich, Germany). For NMR samples, bacteria were grown in M9 minimal medium supplemented with ¹³C-labeled glucose and ¹⁵N-labeled ammonium chloride for uniformly labeled samples. Samples used for stereospecific assignments were prepared by using a ratio of 1:9 of ¹³C-labeled glucose and unlabeled glucose during bacterial expression (Neri et al., 1989). Proteins were purified as described above. The NMR samples had concentrations ranging from 300-500 µM and contained 0.02 % sodium azide and protease inhibitors. All vectors were sequenced and the masses of the purified proteins were verified by matrix assisted laser desorption ionization time of flight mass spectrometry.

### Optical spectroscopy and protein stability measurements

Circular dichroism (CD) measurements were performed in Jasco J-715 and Jasco J-720 spectropolarimeters (Jasco, Grossumstadt, Germany). Far-UV CD spectra were recorded from 194-260 nm in 0.2 mm quartz cuvettes at 25°C and a protein concentration of 50 µM. Near-UV CD spectra were recorded from 250-320 nm at 25°C and protein concentrations of 120-150 µM in 2 mm quartz cuvettes. All spectra were accumulated 16 times and buffer corrected.

Temperature-induced unfolding was followed by far-UV CD spectroscopy. 10 µM protein were unfolded at a heating rate of 30°C/h in a 1 mm quartz cuvette, and the signal change at 205 nm was recorded.

GdmCl- or urea-induced unfolding transitions were followed by either changes in the intrinsic fluorescence signal (all human κ C_{L} constructs; excited at 280 nm, detected at 354 nm) or by changes in the far-UV CD signal at 220 nm (C4). Fluorescence measurements were carried out in a 1 mm quartz cuvette in a Fluoromax-4 spectrometer (Horiba Jobin Yvon); for far-UV CD measurements, a cuvette with 1 mm path-length was used. Protein concentrations of 10 µM (C4), were used. At each GdmCl/urea concentration, 50 individual data points were recorded with an integration time of 1 s at the observation wavelength and averaged. For unfolding transitions, samples were incubated overnight at 25°C at the final GdmCl/urea concentration prior to measurements. For refolding transitions, proteins were first unfolded in 5 M GdmCl for 3 h or 8 M urea overnight, respectively, before refolding at different GdmCl/urea concentrations was initiated. Subsequently, samples were incubated overnight at 25°C at the final GdmCl/urea concentration prior to measurements. All measurements were carried out at 25°C. Data were evaluated according to a two-state monomer unfolding model (Santoro and Bolen, 1992) (C4 and human k C_{L} constructs) Analysis of association-induced folding of a human IgG C_{H}1 domain by the human κ C_{L} constructs by CD spectroscopy was performed as described previously (Feige et al., 2009).

### NMR spectroscopy

### NMR spectroscopy

NMR measurements were carried out at 298 K on a Bruker Avance III 600 MHz spectrometer equipped with a TCl cryo-probe head, a 750 MHz spectrometer equipped with a TXI probe head, an 800 MHz spectrometer with a TCl cryo-probe head, or a Bruker Avance 900 MHz spectrometer equipped with a TXI cryo-probe head (Bruker Biospin GmbH, Rheinstetten, Germany). Spectra were processed with NMRPipe (Delaglio et al., 1995) and analyzed with CARA (see the world wide web at cara.nmr.ch). For backbone and side chain assignment CBCA(CO)NH, CBCANH and HCCH-TOCSY spectra were recorded (Sattler, 1999). Distance restraints were obtained from ¹⁵N- and ¹³C-edited NOESY spectra with mixing times of 120 ms and 150 ms, respectively. ¹⁵N T₁ and T₂ relaxation times and {¹H}-¹⁵N heteronuclear NOE data were measured at 800 MHz proton Larmor frequency at 298 K (Farrow et al., 1994). N-C' and H^{N}-N residual dipolar couplings were recorded using an IPAP-HSQC (Cordier et al., 1999) and HNCO-based NMR experiments (Yang and Kay, 1999) with a 300 µM sample that was aligned in a medium containing 10 mg/ml Pf1 phage (Profos AG, Regensburg, Germany) as described (Otting et al., 2000). The alignment tensor was determined from a power pattern analysis, with Dₐ = - 18.2 and R= 0.145.

### Structure Calculation

Automated NOE cross-peak assignment was performed using the software CYANA 3.0 (Guntert, 2009). This assignment was assisted by manually assigning intra-residual NOEs. Automatically assigned NOEs and completeness of the NOE cross-peaks were manually inspected. Distance restraints from the CYANA calculation and TALOS+-derived (Shen et al., 2009) torsion angles and the residual dipolar couplings were used in a water refinement calculation (Linge et al., 2003) using Aria 2.0 (Rieping et al., 2007). The quality of the structure ensemble, consisting of twenty structures with lowest energy, was validated using the iCING web server (Doreleijers et al., 2012). Molecular images were generated using PyMOL.

### Cell culture, metabolic labeling and western blots

Individual constructs were amplified from synthetic genes optimized for human expression (Geneart, Regensburg, Germany) and cloned into the pSVL vector (GE Healthcare, Pittsburg, PA, USA) via Xhol/BamHl for expression in COS-1 cells. Mutants were generated by site directed mutagenesis using the QuikChange site-directed mutagenesis kit (Agilent Technologies, Santa Clara, CA, USA). All constructs were sequenced. DNA concentrations were determined in triplicates using a Qubit fluorometer (Invitrogen, Grand Island, NY, USA) with standard deviations smaller than 2%.

COS-1 cells were grown in Dulbecco's modified Eagle's medium (DMEM) supplemented with 10% (v/v) fetal bovine serum, 2 mM L-glutamine, and a 1% (v/v) antibiotic-antimycotic solution (25 µg/ml amphotenicin B, 10,000 µg/ml streptomycin, and 10,000 units of penicillin; Cellgro/Mediatech, Manassas, VA) (complete DMEM) at 37°C and 3% CO₂. COS-1 transfections were carried out for 24h in p60 dishes using GeneCellin (BioCellChallenge, Toulon, France) according to the manufacturer's protocol. Either four microgram LC DNA only or two microgram LC DNA together with two microgram γHC DNA were transfected.

For western blots, cells were transfected for 24 h and subsequently the medium was changed for another 26h (1.5 ml/p60) to analyze secreted proteins. Medium was prepared as outlined below, cells were lysed in RIPA buffer (50 mM Tris/HCl, pH 7.5, 150 mM NaCl, 1.0% Nonidet P40 substitute, 0.5% sodium deoxycholate, 0.1% SDS, 0.1 mM PMSF, 1x Roche complete protease inhibitor tablets w/o EDTA (Roche Applied Science, Indianapolis, IN, USA)). The same amount of sample, medium or lysate, was separated on SDS-PAGE gels, transferred to PVDF membranes and blotted with anti-FLAG-antibody (rabbit polyclonal, F7425, Sigma, St. Louis, MO, USA) according to the manufacturer's protocol. Data were quantified from film with the ImageQuant TL software (GE Healthcare, Pittsburg, PA, USA).

For metabolic labeling, cells were washed twice with PBS, then starved for 30 min in complete DMEM without Met and Cys and subsequently labeled for 60 min with 100µCi/p60 dish of EasyTag™ EXPRESS35S Protein Labeling Mix (Perkin Elmer, Waltham, MA, USA). Cells were subsequently chased in complete DMEM (1.5 ml/p60) additionally supplemented with 2 mM cold Met and Cys. To analyze secreted heavy chains, 1.2 ml of the medium were centrifuged for 5 min, 300 g, 4°C. Subsequently, 1.1 ml of the sample were supplemented with protease inhibitor and the sample centrifuged for 15 min, 20.000 g, 4°C. Afterwards, 1.0 ml of the sample were immunoprecipitated with CaptivA™ PriMAB proteinA agarose (Repligen Bioprocessing, Waltham, MA, USA) overnight at 4°C under rotation. Immunoprecipitated proteins were washed three times with RIPA buffer and eluted with Laemmli buffer for 10 min at 95°C. Samples were run on 10% SDS-PAGE gels, signals were amplified with Amplify (GE Healthcare, Pittsburg, PA, USA) and subsequently gels were dried and used in phosphorimager analysis. Phosphorimager scans were carried out on a Storm 860 scanner (GE Healthcare, Pittsburg, PA, USA) and quantified with the ImageQuant TL software (GE Healthcare, Pittsburg, PA, USA).

### Statistical analysis

Results are shown as means ±one standard deviation. Where indicated, a two-tailed Student's t test was used to analyze the data.

### Example 2: Determination of the structures of the IgNAR constant domain C4 at atomic resolution

The structure of the C4 domain was solved by NMR spectroscopy and is well-defined based on 1265 NOE-derived distance restraints, orientational restraints from residual dipolar couplings, and dihedral angle restraints.. Details can be found in the Methods described above and in Table 5.

Even though the IgNAR domains have only limited sequence conservation in comparison to human Ig domains (Fig. 1B and Fig. 5), C4 shows a typical constant domain immunoglobulin fold (c-type). It consists of a two-layer sandwich structure with strands b, c, e and f forming the common core (Bork et al., 1994; Hamill et al., 2000). The two layers of the β-sandwich are covalently linked by a buried disulfide bridge that is orientated roughly perpendicular to the sheets (Figure 3) (Bork et al., 1994). Around this disulfide bridge, several hydrophobic residues form a tight core with a highly conserved tryptophan at its center, which is present in all IgNAR domains (Figure 2). Essentially all known modern antibody domain structures exhibit this core. Thus, this feature developed very early on and remained one of its defining characteristics. In the C4 domain a helix between strand e and f is formed (Figure 3). C4 contains a tryptophan in this helix, which is highly conserved within chordata (Figure 2 and Figure 6). This tryptophan interacts with an almost equally conserved tyrosine or phenylalanine located C-terminally (Figure 6). The evolutionary conservation of this motif is in agreement with an important structural role in the initial phases of the antibody domain folding process (Feige et al., 2008) and suggests that, together with the core of the fold, it has developed early on in antibody domains. Of note, in C4 a salt bridge exists between the second helix and the loop connecting strand c and d that is likely to stabilize the helix and this loop.

### Example 3: Determination of stabilizing features

Taken together, the structure of C4 shows that the Ig fold has been conserved for ca. 500 million years since the last common ancestor of shark and men existed. Comparison of Ig domains from both species, which are not well conserved at the sequence level, allows the identification of key structural features of the Ig fold. Given the very different environments in which antibodies from these two species must function, it appeared likely that such comparisons might reveal structural differences that could influence the folding pathway and stability of shark antibody domains. Indeed, it was found that the C4 domain is more resistant to chemical denaturation than typical mammalian antibody domains (Figure 4) (Feige et al., 2010). In agreement with their high chemical stability, C4 also showed superior stabilities against elevated temperatures (Figure 4). In a search for motifs that are not present in human Ig domains that might serve to stabilize the shark Ig fold, two structural elements were identified: i) the salt bridge between the loop separating strand c and d and the second helix connecting strand e and f and ii) an extended hydrophobic core in C4 due to an additional Val residue in strand d (Figure 7). With a view to test whether these elements could improve the properties of modern Ig domains, these motifs were introduced into the monomeric human Ig kappa light chain constant domain (C_{L}) and their effect on domain stability and antibody secretion was assessed. When the additional salt bridge (M1) and the extended hydrophobic core (M2) were introduced together into the C_{L} domain (M1+2) a significant stabilization was achieved. The melting point of M1+2 was almost 10°C higher (67.0 ±0.4°C) than that of the wt C_{L} domain and its stability against urea was markedly increased (Figure 5). Importantly, all C_{L} mutants tested still associated with the heavy chain C_{H}1 domain, its authentic partner domain, and induced its folding (Feige et al., 2009), which is a critical test for their structural integrity (Figure 6).

Next, the ability of the stabilized C_{L} mutants to exert their advantageous characteristics in cells was assessed, where the accuracy of antibody folding and assembly are carefully monitored by quality control processes in the endoplasmic reticulum (ER). The different LC variants, comprising mutants M1, M2 or M1+2, were expressed either alone or together with Ig γ heavy chains (yHCs) in COS-1 cells (Lee et al., 1999). When the LCs were expressed alone, an increase was observed in secretion of the combined mutant (LC_{M1+2}) compared to wild-type LC (LC_{wt}), (Figure 7). Importantly, when the different LCs were co-expressed with the γHC, a significant increase in the assembly and subsequent secretion of complete IgG antibody molecules was observed for LC_{M1}, which was even more pronounced with the double mutant (LC_{M1+2}) (Figure 5). This demonstrates that the IgNAR-based optimization of a human C_{L} domain, the scaffold on which the heavy chain C_{H}1 domain has to fold for antibodies to pass ER quality control (Feige et al., 2009; Lee et al., 1999), positively influences limiting steps in antibody biosynthesis.

**Table 5 - Structural statistics for the NMR structure calculation of C4**

| **NOE-based distance restraints** | |
|---|---|
| Intraresidual, sequential | 756 |
| | |
| Medium range (1 ≤ \|i-j\| ≤ 5) | 80 |
| Long range (\|i-j\| ≥ 5) | 429 |
| Total | 1265 |

| **Other restraints** | |
|---|---|
| φ+ψ dihedral angle restraints | 156 |
| Residual dipolar coupling restraints (H^{N}-N, N-C') | 95 |

| **Coordinate precision r.m.s.d** | |
|---|---|
| Backbone (Å) | 0.31 ± 0.08 |
| Heavy atom (Å) | 0.87 ± 0.05 |

| **Consistency (structures vs. Restraints)** | |
|---|---|
| r.m.s.d (Å) from experimental distance restraints^{a} | 0.007 ± 0.002 |
| r.m.s.d (°) from experimental torsion angle restraints^{b} | 0.703 ± 0.061 |
| RDC Q-factor^{c} | 0.243 ± 0.011 |

| **After water refinement** | **Structure Z-score** |
|---|---|
| **WHATCHECK^{d}** | |
| First generation packing quality | 1.640 |
| Second generation packing quality | 5.668 |
| Ramachandran plot appearance | -0.279 |
| X₁/X₂ rotamer normality | -3.969 |
| Backbone conformation | 0.987 |

| **Ramachandran plot^{d}** | |
|---|---|
| Most favoured regions | 93.6% |
| Allowed regions | 6.4 % |
| Generously allowed regions | 0.0% |
| Disallowed regions | 0.0% |

| | |
|---|---|
| ^{a}distance restraints were employed with a soft square well potential using an energy constant of 50 kcal mol⁻¹Å⁻². No distance restraint was violated by more than 0.5 Å. ^{b}Torsion angle restraints derived from TALOS+ (Shen et al., 2009) were applied to, backbone angles using energy constants of 200 kcal mol⁻¹ radians⁻². One dihedral angle restraint was violated by more than 5°. ^{c}Residual dipolar couplings (RDCs) were employed with a harmonic potential using an energy constant of 0.5 kcal mol⁻¹ Hz⁻². Q-factor as defined in (Lipsitz and Tjandra, 2004). ^{d}PROCHECK (Laskowski et al., 1996) and WHATCHECK (Hooft et al., 1996) were used to determine the quality of the structure . Positive WHATCHECK Z-scores indicate that the structure is better than average. The low _{X1}/_{X2} rotamer normality is due to Leu523 and Leu539. For these residues the floating stereospecific assignments of the side chain methyl groups did not converge. Quality assessment was done only for residue ranges 456-464 and 478-556 due to the lack of restraints in loop 465-477, in the four N-terminal residues and their high flexibility. | |

### References

Alberts, B., Johnson, A., Lewis, A., Raff, M., Roberts, K., and Walter, P. (2007). Molecular Biology of the Cell (Garland Science).
Altshuler, E.P., Serebryanaya, D.V., and Katrukha, A.G. (2010). Generation of recombinant antibodies and means for increasing their affinity. Biochemistry (Mosc) 75,1584-1605.
Ausubel, F.M., Brent, R., Kingston, R.E., Moore, D.D., Seidman, J.G., Smith, J.A., and Struhl, K. (2012). Short Protocols in Molecular Biology (Wiley Interscience).
Bebbington, C.R., Renner, G., Thomson, S., King, D., Abrams, D., and Yarranton, G.T. (1992). High-level expression of a recombinant antibody from myeloma cells using a glutamine synthetase gene as an amplifiable selectable marker. Biotechnology (N Y) 10, 169-175.
Berstein, R.M., Schluter, S.F., Shen, S., and Marchalonis, J.J. (1996). A new high molecular weight immunoglobulin class from the carcharhine shark: implications for the properties of the primordial immunoglobulin. Proc Natl Acad Sci U S A 93, 3289-3293.
Blair, J.E., and Hedges, S.B. (2005). Molecular phylogeny and divergence times of deuterostome animals. Mol Biol Evol 22, 2275-2284.
Bork, P., Holm, L., and Sander, C. (1994). The immunoglobulin fold. Structural classification, sequence patterns and common core. J Mol Biol 242, 309-320.
Braasch, D.A., and Corey, D.R. (2001). Locked nucleic acid (LNA): fine-tuning the recognition of DNA and RNA. Chem Biol 8, 1-7.
Braberg, H., Webb, B.M., Tjioe, E., Pieper, U., Sali, A., and Madhusudhan, M.S. (2012). SALIGN: a web server for alignment of multiple protein sequences and structures. Bioinformatics 28, 2072-2073.
Cole, S.P., Vreeken, E.H., and Roder, J.C. (1985). Antibody production by human X human hybridomas in serum-free medium. J Immunol Methods 78, 271-278.
Cooper, M.D., and Alder, M.N. (2006). The evolution of adaptive immune systems. Cell 124, 815-822.
Cordier, F., Dingley, A.J., and Grzesiek, S. (1999). A doublet-separated sensitivity-enhanced HSQC for the determination of scalar and dipolar one-bond J-couplings. J Biomol NMR 13, 175-180.
Delaglio, F., Grzesiek, S., Vuister, G.W., Zhu, G., Pfeifer, J., and Bax, A. (1995). NMRPipe: a multidimensional spectral processing system based on UNIX pipes. J Biomol NMR 6, 277-293.
Dooley, H., and Flajnik, M.F. (2006). Antibody repertoire development in cartilaginous fish. Dev Comp Immunol 30, 43-56.
Doreleijers, J.F., Sousa da Silva, A.W., Krieger, E., Nabuurs, S.B., Spronk, C.A., Stevens, T.J., Vranken, W.F., Vriend, G., and Vuister, G.W. (2012). CING: an integrated residue-based structure validation program suite. J Biomol NMR 54, 267-283.
England, J.L., and Haran, G. (2011). Role of solvation effects in protein denaturation: from thermodynamics to single molecules and back. Annu Rev Phys Chem 62, 257-277.
Ewert, S., Honegger, A., and Pluckthun, A. (2004). Stability improvement of antibodies for extracellular and intracellular applications: CDR grafting to stable frameworks and structure-based framework engineering. Methods 34, 184-199.
Farrow, N.A., Muhandiram, R., Singer, A.U., Pascal, S.M., Kay, C.M., Gish, G., Shoelson, S.E., Pawson, T., Forman-Kay, J.D., and Kay, L.E. (1994). Backbone dynamics of a free and phosphopeptide-complexed Src homology 2 domain studied by 15N NMR relaxation. Biochemistry 33, 5984-6003.
Feige, M.J., Groscurth, S., Marcinowski, M., Shimizu, Y., Kessler, H., Hendershot, L.M., and Buchner, J. (2009). An unfolded CH1 domain controls the assembly and secretion of IgG antibodies. Mol Cell 34, 569-579.
Feige, M.J., Groscurth, S., Marcinowski, M., Yew, Z.T., Truffault, V., Paci, E., Kessler, H., and Buchner, J. (2008). The structure of a folding intermediate provides insight into differences in immunoglobulin amyloidogenicity. Proc Natl Acad Sci U S A 105, 13373-13378.
Feige, M.J., Simpson, E.R., Herold, E.M., Bepperling, A., Heger, K., and Buchner, J. (2010). Dissecting the alternatively folded state of the antibody Fab fragment. J Mol Biol 399, 719-730.
Flajnik, M.F., and Kasahara, M. (2010). Origin and evolution of the adaptive immune system: genetic events and selective pressures. Nat Rev Genet 11, 47-59.
Green, M.R., and Sambrook, J. (2012). Molecular Cloning: A Laboratory Manual (CSH Press). Greenberg, A.S., Avila, D., Hughes, M., Hughes, A., McKinney, E.C., and Flajnik, M.F. (1995). A new antigen receptor gene family that undergoes rearrangement and extensive somatic diversification in sharks. Nature 374, 168-173.
Greenberg, A.S., Hughes, A.L., Guo, J., Avila, D., McKinney, E.C., and Flajnik, M.F. (1996). A novel "chimeric" antibody class in cartilaginous fish: IgM may not be the primordial immunoglobulin. Eur J Immunol 26, 1123-1129.
Guntert, P. (2009). Automated structure determination from NMR spectra. Eur Biophys J 38, 129-143.
Hamill, S.J., Steward, A., and Clarke, J. (2000). The folding of an immunoglobulin-like Greek key protein is defined by a common-core nucleus and regions constrained by topology. J Mol Biol 297, 165-178.
Harlow, E., and Lane, D.P. (1988). Antibodies, A Laboratory Manual (CSH Press).
Harlow, E., and Lane, D.P. (1999). Using Antibodies: A Laboratory Manual (CSH Press).
Henderson, K.A., Streltsov, V.A., Coley, A.M., Dolezal, O., Hudson, P.J., Batchelor, A.H., Gupta, A., Bai, T., Murphy, V.J., Anders, R.F., et al. (2007). Structure of an IgNAR-AMA1 complex: targeting a conserved hydrophobic cleft broadens malarial strain recognition. Structure 15, 1452-1466.
Holliger, P., and Hudson, P.J. (2005). Engineered antibody fragments and the rise of single domains. Nat Biotechnol 23,1126-1136.
Hooft, R.W., Vriend, G., Sander, C., and Abola, E.E. (1996). Errors in protein structures. Nature 381, 272.
Hsu, E., Pulham, N., Rumfelt, L.L., and Flajnik, M.F. (2006). The plasticity of immunoglobulin gene systems in evolution. Immunol Rev 210, 8-26.
Kohler, G., and Milstein, C. (1975). Continuous cultures of fused cells secreting antibody of predefined specificity. Nature 256, 495-497.
Kozbor, D., Dexter, D., and Roder, J.C. (1983). A comparative analysis of the phenotypic characteristics of available fusion partners for the construction of human hybridomas. Hybridoma 2, 7-16.
Larkin, M.A., Blackshields, G., Brown, N.P., Chenna, R., McGettigan, P.A., McWilliam, H., Valentin, F., Wallace, I.M., Wilm, A., Lopez, R., et al. (2007). Clustal W and Clustal X version 2.0. Bioinformatics 23, 2947-2948.
Laskowski, R.A., Rullmannn, J.A., MacArthur, M.W., Kaptein, R., and Thornton, J.M. (1996). AQUA and PROCHECK-NMR: programs for checking the quality of protein structures solved by NMR. J Biomol NMR 8, 477-486.
Lee, Y.K., Brewer, J.W., Hellman, R., and Hendershot, L.M. (1999). BiP and immunoglobulin light chain cooperate to control the folding of heavy chain and ensure the fidelity of immunoglobulin assembly. Mol Biol Cell 10, 2209-2219.
Linge, J.P., Williams, M.A., Spronk, C.A., Bonvin, A.M., and Nilges, M. (2003). Refinement of protein structures in explicit solvent. Proteins 50, 496-506.
Lipsitz, R.S., and Tjandra, N. (2004). Residual dipolar couplings in NMR structure analysis. Annu Rev Biophys Biomol Struct 33, 387-413.
Lodish, H., Berk, A., Kaiser, C.A., and Krieger, M. (2012). Molecular Cell Biology (W. H. Freeman).
Merrifield, R.B. (1969). Solid-phase peptide synthesis. Adv Enzymol Relat Areas Mol Biol 32, 221-296.
Muller, C.I., Blumbach, B., Krasko, A., and Schroder, H.C. (2001). Receptor protein-tyrosine phosphatases: origin of domains (catalytic domain, Ig-related domain, fibronectin type III module) based on the sequence of the sponge Geodia cydonium. Gene 262, 221-230.
Murphy, G., Cockett, M.I., Ward, R.V., and Docherty, A.J. (1991). Matrix metalloproteinase degradation of elastin, type IV collagen and proteoglycan. A quantitative comparison of the activities of 95 kDa and 72 kDa gelatinases, stromelysins-1 and -2 and punctuated metalloproteinase (PUMP). Biochem J 277 ( Pt 1), 277-279.
Neri, D., Szyperski, T., Otting, G., Senn, H., and Wuthrich, K. (1989). Stereospecific nuclear magnetic resonance assignments of the methyl groups of valine and leucine in the DNA-binding domain of the 434 repressor by biosynthetically directed fractional 13C labeling. Biochemistry 28, 7510-7516.
Otting, G., Ruckert, M., Levitt, M.H., and Moshref, A. (2000). NMR experiments for the sign determination of homonuclear scalar and residual dipolar couplings. J Biomol NMR 16, 343-346.
Owens, G.C., Chappell, S.A., Mauro, V.P., and Edelman, G.M. (2001). Identification of two short internal ribosome entry sites selected from libraries of random oligonucleotides. Proc Natl Acad Sci U S A 98,1471-1476.
Rieping, W., Habeck, M., Bardiaux, B., Bernard, A., Malliavin, T.E., and Nilges, M. (2007). ARIA2: automated NOE assignment and data integration in NMR structure calculation. Bioinformatics 23, 381-382.
Roux, K.H., Greenberg, A.S., Greene, L., Strelets, L., Avila, D., McKinney, E.C., and Flajnik, M.F. (1998). Structural analysis of the nurse shark (new) antigen receptor (NAR): molecular convergence of NAR and unusual mammalian immunoglobulins. Proc Natl Acad Sci U S A 95,11804-11809.
Saerens, D., Ghassabeh, G.H., and Muyldermans, S. (2008). Single-domain antibodies as building blocks for novel therapeutics. Curr Opin Pharmacol 8, 600-608.
Santoro, M.M., and Bolen, D.W. (1992). A test of the linear extrapolation of unfolding free energy changes over an extended denaturant concentration range. Biochemistry 31, 4901-4907. Sattler, M.S., J.; and Griesinger, C. (1999). Heteronuclear multidimensional NMR experiments for the structure determination of proteins in solution employing pulsed field gradients. Prog Nucl Magn Res Spectrosc 34, 93-158.
Shen, Y., Delaglio, F., Cornilescu, G., and Bax, A. (2009). TALOS+: a hybrid method for predicting protein backbone torsion angles from NMR chemical shifts. J Biomol NMR 44, 213-223.
Stanfield, R.L., Dooley, H., Flajnik, M.F., and Wilson, I.A. (2004). Crystal structure of a shark single-domain antibody V region in complex with lysozyme. Science 305, 1770-1773.
Stewart, J.M. (1969). Solid Phase Peptide Synthesis (Freeman Co. San Francisco).
Streltsov, V.A., Varghese, J.N., Carmichael, J.A., Irving, R.A., Hudson, P.J., and Nuttall, S.D. (2004). Structural evidence for evolution of shark Ig new antigen receptor variable domain antibodies from a cell-surface receptor. Proc Natl Acad Sci U S A 101, 12444-12449.
Yang, D., and Kay, L.E. (1999). Improved 1 HN-detected triple resonance TROSY-based experiments. J Biomol NMR 13, 3-10.

## Claims

1. A method of producing a modified protein having an increased stability and/or improved folding efficiency as compared to the unmodified protein, the method comprising
(i) modifying a nucleic acid molecule encoding a protein comprising at least one immunoglobulin constant domain-like region by
(a-i) replacing the nucleotides encoding at least one amino acid, preferably an uncharged amino acid, in the loop separating the C strand from the D strand with nucleotides encoding a charged amino acid selected from the group consisting of Arg, Lys, His, Glu and Asp and/or replacing the nucleotides encoding at least one amino acid, preferably an uncharged amino acid, in the helix connecting the E strand with the F strand with nucleotides encoding a charged amino acid selected from the group consisting of Arg, Lys, His, Glu and Asp;
(a-ii) replacing the nucleotides encoding at least one amino acid not having a side chain that can form a hydrogen bond in the loop separating the C strand from the D strand with nucleotides encoding an amino acid having a side chain capable of forming a hydrogen bond selected from the group consisting of Gin, Asn, Tyr, Ser and Thr and/or replacing the nucleotides encoding at least one amino acid not having a side chain that can form a hydrogen bond in the helix connecting the E strand with the F strand with nucleotides encoding an amino acid having a side chain capable of forming a hydrogen bond selected from the group consisting of Gln, Asn, Tyr, Ser and Thr; and/or
(a-iii) replacing the nucleotides encoding at least one amino acid in the loop separating the C strand from the D strand with nucleotides encoding a cysteine and/or replacing the nucleotides encoding at least one amino acid in the helix connecting the E strand with the F strand with nucleotides encoding a cysteine; and/or
(b) replacing the nucleotides encoding at least one non-hydrophobic amino acid at a position suitable to participate in the formation of the hydrophobic core with nucleotides encoding a hydrophobic amino acid selected from the group consisting of Val, Ile, Leu, Met, Phe, Trp and Pro; and
(ii) expressing the nucleic acid molecule to produce the stabilised protein.

2. A method of producing a modified protein having an improved secretion from cells as compared to the unmodified protein, the method comprising
(i) modifying a nucleic acid molecule encoding a protein comprising at least one immunoglobulin constant domain-like region by
(a-i) replacing the nucleotides encoding at least one amino acid, preferably an uncharged amino acid, in the loop separating the C strand from the D strand with nucleotides encoding a charged amino acid selected from the group consisting of Arg, Lys, His, Glu and Asp and/or replacing the nucleotides encoding at least one amino acid, preferably an uncharged amino acid, in the helix connecting the E strand with the F strand with nucleotides encoding a charged amino acid selected from the group consisting of Arg, Lys, His, Glu and Asp;
(a-ii) replacing the nucleotides encoding at least one amino acid not having a side chain that can form a hydrogen bond in the loop separating the C strand from the D strand with nucleotides encoding an amino acid having a side chain capable of forming a hydrogen bond selected from the group consisting of Gln, Asn, Tyr, Ser and Thr and/or replacing the nucleotides encoding at least one amino acid not having a side chain that can form a hydrogen bond in the helix connecting the E strand with the F strand with nucleotides encoding an amino acid having a side chain capable of forming a hydrogen bond selected from the group consisting of Gln, Asn, Tyr, Ser and Thr; and/or
(a-iii) replacing the nucleotides encoding at least one amino acid in the loop separating the C strand from the D strand with nucleotides encoding a cysteine and/or replacing the nucleotides encoding at least one amino acid in the helix connecting the E strand with the F strand with nucleotides encoding a cysteine; and/or
(b) replacing the nucleotides encoding at least one non-hydrophobic amino acid at a position suitable to participate in the formation of the hydrophobic core with nucleotides encoding a hydrophobic amino acid selected from the group consisting of Val, Ile, Leu, Met, Phe, Trp and Pro; and
(ii) expressing the nucleic acid molecule to produce the protein having improved secretion.

3. A protein comprising at least one immunoglobulin constant domain-like region having an additional salt bridge, an additional hydrogen bond, an additional disulfide bond and/or an extended hydrophobic core, wherein the protein comprises one or more modifications selected from the group consisting of
(i) a replacement of at least one amino acid, preferably an uncharged amino acid, in the loop separating the C strand from the D strand for a charged amino acid selected from the group consisting of Arg, Lys, His, Glu and Asp and/or a replacement of at least one amino acid, preferably an uncharged amino acid, in the helix connecting the E strand with the F strand for a charged amino acid selected from the group consisting of Arg, Lys, His, Glu and Asp, thereby enabling the formation of an additional salt bridge;
(ii) a replacement of at least one amino acid not having a side chain that can form a hydrogen bond in the loop separating the C strand from the D strand for an amino acid having a side chain capable of forming a hydrogen bond selected from the group consisting of Gln, Asn, Tyr, Ser and Thr and/or a replacement of at least one amino acid not having a side chain that can form a hydrogen bond in the helix connecting the E strand with the F strand for an amino acid having a side chain capable of forming a hydrogen bond selected from the group consisting of Gin, Asn, Tyr, Ser and Thr; thereby enabling the formation of an additional hydrogen bond;
(iii) a replacement of at least one amino acid in the loop separating the C strand from the D strand for a cysteine and/or a replacement of at least one amino acid in the helix connecting the E strand with the F strand for a cysteine; thereby enabling the formation of an additional disulfide bridge; and/or
(iv) a replacement of at least one non-hydrophobic amino acid at a position suitable to participate in the formation of the hydrophobic core for a hydrophobic amino acid selected from the group consisting of Val, Ile, Leu, Met, Phe, Trp and Pro; thereby extending the hydrophobic core.

4. The method according to claim 1 or 2 or the protein of claim 3, wherein the at least one amino acid in the loop separating the C strand from the D strand is an amino acid at a position corresponding to position 48 in SEQ ID NO: 1 and wherein the at least one amino acid in the helix connecting the E strand with the F strand is an amino acid at a position corresponding to position 76 in SEQ ID NO: 1.

5. The method according to any one of claims 1, 2 or 4 or the protein according to claim 3 or 4, wherein the at least one non-hydrophobic amino acid in a position suitable to participate in the formation of the hydrophobic core is an amino acid in the D strand.

6. The method according to claim 5 or the protein according to claim 5, wherein the at least one non-hydrophobic amino acid in the D strand is an amino acid at a position corresponding to position 52 in SEQ ID NO: 1.

7. The method or the protein according to any one of the preceding claims, wherein the protein comprising at least one immunoglobulin constant domain-like region is an antibody.

8. The method or the protein according to claim 7, wherein the antibody is a human or humanised antibody.

9. The method or the protein according to any one of the preceding claims, wherein the protein comprising at least one immunoglobulin constant domain-like region is a eukaryotic protein.

10. A nucleic acid molecule encoding the protein obtained by the method of any one of claims 1, 2 or 4 to 9 or the protein of any one of claims 3 to 9.

11. A vector comprising the nucleic acid molecule of claim 10.

12. A host cell or a non-human host transformed with the vector of claim 11.

13. A method for the production of a modified protein according to claim 3, the method comprising the steps of:
(i) culturing the host cell of claim 12 under suitable conditions; and
(ii) isolating the modified protein according to claim 3 produced by said host cell.

14. A composition comprising at least one of
(i) the protein of any one of claims 3 to 9;
(ji) the nucleic acid molecule of claim 10;
(iii) the vector of claim 11; or
(iv) the host cell of claim 12.

15. A kit comprising at least one of
(i) the protein of any one of claims 3 to 9;
(ji) the nucleic acid molecule of claim 10;
(iii) the vector of claim 11; or
(iv) the host cell of claim 12.
